# EUROPEAN PATENT SPECIFICATION

(11) **EP 1 837 330 B1**
(45) Date of publication and mention of the grant of the patent: **24.10.2012**
(21) Application number: 07103182.7
(22) Date of filing: 10.05.2004
(51) Int. Cl.: C07D 213/56, C07D 231/12, C07D 233/54, C07D 237/14, C07D 249/04, C07D 261/08, C07D 277/30, C07D 403/12, C07D 405/06, A61K 31/395, A61P 11/06, A61P 37/00

(54) **Benzamide inhibitors of the P2X7 receptor**
Benzamidinhibitoren des P2X7-Rezeptors
Inhibiteurs benzamidiques du recepteur P2X7

(30) Priority: 12.05.2003 US 470001 P
(43) Date of publication of application: 26.09.2007
(62) Divisional of application: 04731975.1
(73) Proprietor: Pfizer Products Inc., Groton, CT 06340 (US)
(72) Inventor: Dombroski, Mark Anthony, Groton, CT 06340 (US); Duplantier, Allen Jacob, Groton, CT 06340 (US); Subramanyam, Chakrapani, Groton, CT 06340 (US)
(74) Representative: Pfizer

(56) References cited:
- EP-A- 0 881 219
- WO-A-02/094767
- WO-A-03/042191
- WO-A-2004/058270
- WO-A-2004/058731
- ARANAPAKAM V ET AL: "5-fluoro-2-methyl-N-[5-(5H-pyrrolo[2,1-c] [1,4]benzodiazepine-10(11H) YL carbonyl)-2-pyridinyl]benzamide (CL-385004) and analogs as orally active arginine vasopressin receptor antagonists" BIOORGANIC & MEDICINAL CHEMISTRY LETTERS, OXFORD, GB, vol. 9, no. 13, 5 July 1999 (1999-07-05), pages 1737-1740, XP004168830 ISSN: 0960-894X

## Description

The present invention relates to novel benzamide inhibitors of the P2X₇ receptor, processes for their preparation, intermediates useful in their preparation, pharmaceutical compositions containing them, and their use in therapy. The active compounds of the present invention are useful in the treatment of inflammatory diseases such as osteoarthritis and rheumatoid arthritis; allergies, asthma, COPD, cancer, reperfusion or ischemia in stroke or heart attack, autoimmune diseases and other disorders. The active compounds are also antagonists of the P2X₇ receptor.

The P2X₇ receptor (previously known as P2Z receptor), which is a ligand-gated ion channel, is present on a variety of cell types, largely those known to be involved in inflammatory/immune process, specifically, macrophages, mast cells and lymphocytes (T and B). Activation of the P2X₇ receptor by extracellular nucleotides, in particular adenosine triphosphate, leads to the release of interleukin-1β (IL-1β) and giant cell formation (macrophages/microglial cells), degranulation (mast cells) and proliferation (T cells), apoptosis, and L-selectin shedding (lymphocytes). P2X₇ receptors are also located on antigen-presenting cells (APC), keratinocytes, salivary acinar cells (parotid cells), hepatocytes and mesangial cells.

P2X₇ antagonists are known in the art, such as those described in International Patent Publications WO O1/46200, WO O1/42194, WO 01/44213, WO99/29660, WO 00/61569, WO 99/29661, WO 99/29686, WO 00/71529, WO 02/094767and WO 01/44170. Other inhibitors of P2X₇ are described in United States Non-provisional Application 10/292887, filed November 12, 2002 which claimed priority to United States Provisional Application 60/336781, filed November 12, 2001. Other adamantyl P2X₇ inhibitors are described in United States Non-provisional application 10/292886 filed November 12, 2002 which claimed priority to United States provisional application 60/336892 filed November 12, 2001. Yet other P2X₇ inhibitors are described in United States Provisional Applications 60/437228 and 60/437505 both filed December 31, 2002. Each of the foregoing application is incorporated herein by reference in their entirety.

Benzamides, heteroarylamides and reverse amides for uses other than inhibition of the P2X₇ receptor are described in various publications, such as International Patent Publications WO 97/22600, EP 138,527, WO 00/71509, WO 98/28269, WO 99/17777 and WO 01/58883.

### SUMMARY OF THE INVENTION

In one aspect, the present invention relates to compounds of the formula 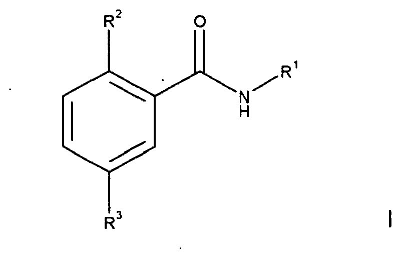 wherein R¹ is a (C₁-C₆)alkyl optionally substituted with one or two radicals independently selected from hydroxy, halo, -CN, (C₁-C₆)alkyl, HO(C₁-C₆)alkyl, NH₂(C=O)-, (C₁-C₆)alkyl-NH-(C=O)-, [(C₁-C₆)alkyl]₂N-(C=O)-, oxo and (C₁-C₆)alkoxy; wherein said R¹ (C₁-C₆)alkyl may also optionally be substituted with one or two groups independently selected from (C₁-C₆)alkyl, (C₃-C₁₀)cycloalkyl, (C₆-C₁₀)aryl and (C₁-C₁₀)heteroaryl; wherein said (C₃-C₁₀)cycloalkyl group is other than adamantyl; wherein said (C₁-C₆)alkyl and (C₃-C₁₀)cycloalkyl may be optionally substituted with oxo; wherein said (C₁-C₁₀)heteroaryl is selected from furanyl, thiophenyl, benzthiophenyl, benzfuranyl and chromanyl; wherein each of said (C₁-C₆)alkyl, (C₃-C₁₀)cycloalkyl, (C₆-C₁₀)aryl and (C₁-C₁₀)heteroaryl groups may also optionally be substituted by one to three radicals independently selected from hydroxy, halo, -CN, (C₁-C₆)alkyl, HO(C₁-C₆)alkyl, NH₂(C=O)-, (C₁-C₆)alkyl-NH(C=O)-, [(C₁-C₆)ₐlkylhN-(C_{=O})-, (C₁-C₆)alkoxy, (C₆-C₁₀)aryl and (C₃-C₁₀)cycloalkyl; wherein said (C₃-C₁₀)cycloalkyl and (C₆-C₁₀)aryl radicals are optionally substituted by one to three moieties independently selected from halo, (C₁-C₆)alkyl, -CN and (C₁-C₆)alkoxy;
R² is halo, -CN or (C₁-C₆)alkyl; wherein said (C₁-C₆)alkyl is optionally substituted by one to three radicals independently selected from the group consisting of: halo, hydroxy, amino, -CN, (C₁-C₆)alkyl, (C₁-C₆)alkoxy, -CF₃, CF₃O-, NH₂, (C₁-C₆)alkyl-NH-, [(C₁-C₆)alkyl]₂-N-, (C₁-C₆)alkyl-S-, (C₁-C₆)alkyl-(S=O)-, (C₁-C₆)alkyl-(SO₂)-, (C₁-C₆)alkyl-O-(C=O)-, formyl, (C₁-C₆)alkyl-(C=O)-, and (C₃-C₆)cycloalkyl;
R³ is an optionally substituted carbon linked (C₁-C₁₀)heteroaryl or (C₁-C₁₀)heterocyclyl; wherein said optional substituents can be on any carbon atom of said (C₁-C₁₀)heteroaryl or (C₁-C₁₀)heterocyclyl capable of substitution with one to three R⁴ per ring; wherein said optional substituents can be on any nitrogen atom of said (C₁-C₁₀)heteroaryl or (C₁-C₁₀)heterocyclyl capable of substitution with one to two R⁵ per ring;
wherein each R⁴ is independently selected from the group consisting of: halo, -CN, NH₂, hydroxy, H₂N(C=O)-, H₂N-SO₂-, and optionally substituted R⁶ substituents;
wherein said optionally substituted R⁶ substituents are selected from the group consisting of: (C₁-C₆)alkyl, (C₃-C₁₀)cycloalkyl, (C₁-C₁₀)heterocyclyl, (C₆-C₁₀)aryl, (C₁-C₁₀)heteroaryl, (C₁-C₆)alkoxy, (C₁-C₆)alkyl-(C=O)O-, (C₁-C₆)alkyl-NH-, [(C₁-C₆)alkyl]₂-N-, (C₆-C₁₀)aryl-NH-, (C₁-C₆)alkyl-(C=O)NH-, (C₁-C₆)alkyl-SO₂-NH-, (C₁-C₆)alkyl-(C=O)-, (C₁-C₆)alkyl-NH-(C=O)-, [(C₁-C₆)alkyl]₂-N-(C=O)-, (C₁-C₆)alkyl-O(C=O)-, (C₃-C₁₀)cycloalkyl-(C=O)-, (C₁-C₁₀)heterocyclyl-(C=O)-, (C₆-C₁₀)aryl-(C=O)-, (C₁-C₁₀)heteroaryl-(C=O)-, (C₁-C₆)alkyl-SO₂-, (C₆-C₁₀)aryl-SO₂-, (C₁-C₁₀)heteroaryl-SO₂-, (C₁-C₆)alkyl-NH-SO₂-, and [(C₁-C₆)alkyl]₂N-SO₂-,
wherein any two R⁴ radicals on a carbon atom of said (C₁-C₁₀)heterocycyl can be taken together to form an oxo group or a spiro (C₃-C₆)carbocyclic or (C₁-C₆)heterocyclic group;
wherein each R⁵ is independently selected from the group consisting of: H₂N(C=O)-, and the following optionally substituted R⁸ groups: (C₁-C₆)alkyl, (C₁-C₁₀)heterocyclyl, (C₁-C₁₀)heteroaryl, (C₁-C₆)alkyl-(C=O)-, (C₁-C₆)alkyl-NH-(C=O)-, [(C₁-C₆)alkyl]₂-N-(C=O)-, (C₁-C₆)alkyl-O(C=O)-, (C₃-C₁₀)cycloalkyl-(C=O)-, (C₁-C₁₀)heterocyclyl-(C=O)-, (C₆-C₁₀)aryl-(C=O)-, (C₁-C₁₀)heteroaryl-(C=O)-, and (C₁-C₆)alkyl-SO₂-;
wherein each of said optionally substituted R⁶ substituents may be substituted with one to three groups independently selected from the group consisting of: halo, NH₂, -CN, hydroxy, (C₁-C₆)alkyl-SO₂-, H₂N-SO₂-, (C₁-C₆)alkyl-NH-SO₂-, and [(C₁-C₆)alkyl]₂N-SO₂-, (C₁-C₆)alkoxy, (C₁-C₆)alkyl-SO₂-NH-, (C₁-C₆)alkyl-(C=O)-, (C₁-C₆)alkyl-(C=O)O-, H₂N(C=O)-, (C₁-C₆)alkyl-NH-(C=O)-, [(C₁-C₆)alkyl]-N-(C=O)-, (C₁-C₆)alkyl-O(C=O)-, and optionally substituted R⁷ groups;
wherein each of said optionally substituted R⁷ groups are independently selected from the group consisting of: (C₃-C₁₀)cycloalkyl, (C₁-C₁₀)heterocyclyl, (C₆-C₁₀)aryl, (C₁-C₁₀)heteroaryl, phenoxy, (C₁-C₆)alkyl-NH-, [(C₁-C₆)alkyl]₂-N-, (C₆-C₁₀)aryl-NH-, (C₃-C₁₀)cycloalkyl-(C=O)-, (C₁-C₁₀)heterocyclyl-(C=O)-, (C₆-C₁₀)aryl-(C=O)-, (C₁-C₁₀)heteroaryl-(C=O)-, (C₆-C₁₀)aryl-SO₂-, and (C₁-C₁₀)heteroaryl-SO₂-;
wherein each of said optionally substituted R⁷ groups may be optionally substituted with one to three substituents independently selected from the group consisting of: halo, CF₃, -CN, (C₁-C₆)alkyl, (C₁-C₁₀)heterocyclyl, (C₁-C₁₀)heteroaryl, hydroxy, (C₁-C₆)alkoxy, (C₁-C₆)alkyl-O(C=O)-, (C₁-C₆)alkyl-SO₂-NH-, (C₁-C₆)alkyl-SO₂-, (C₆-C₁₀)aryl-SO₂-, (C₁-C₁₀)heteroaryl-SO₂-, H₂N-SO₂-, (C₁-C₆)alkyl-NH-SO₂-, and [(C₁-C₆)alkylhN-SO₂-;
wherein each of said optionally substituted R⁸ groups may be substituted with one to three substituents may be independently selected from the group consisting of: halo, -CN, hydroxy, (C₁-C₆)alkoxy, (C₁-C₆)alkyl-(C=O)O-, H₂N-(C=O)O-, (C₁-C₆)alkyl-NH-(C=O)O-, ((C₁-C₆)alkyl)₂N-(C=O)O-, NH₂, (C₁-C₆)alkyl-NH-, [(C₁-C₆)alkylh-N-, (C₁-C₆)alkyl)-(C=O)NH- , (C₁-C₆)alkyl)-NH-(C=O)NH-, (C₁-C₆)alkyl-SO₂-NH- ,(C₁-C₆)alkyl-(C=O)NH-, (C₁-C₆)alkyl-(C=O)-, H₂N(C=O)-, (C₁-C₆)alkyl- NH -(C=O)-, [(C₁-C₆)alkyl]₂- N-(C=O)-, (C₁-C₆)alkyl-O(C=O)-, (C₁-C₆)alkyl-S_{O2}-, H₂N-SO₂-, (C₁-C₆)alkyl-NH-SO₂-, and [(C₁-C₆)alkyl]₂N-SO₂- and optionally substitued R⁹ groups;
wherein each of said optionally substituted R⁹ groups are independently selected from the group consisting of: (C₆-C₁₀)aryl, (C₃-C₁₀)cycloalkyl-µNH(C=O)-, (C₁-C₁₀)heterocyclyl-NH-(C=O)-, (C₆-C₁₀)aryl-NH-(C=O)-, (C₁-C₁₀)heteroaryl-NH-(C=O)-, (C₃-C₁₀)cycloalkyl-(C=O)-, (C₁-C₁₀)heterocyclyl-(C=O)-, (C₆-C₁₀)aryl-(C=O)-, (C₁-C₁₀)heteroaryl-(C=O)-, (C₆-C₁₀)aryl-SO₂-, (C₁-C₁₀₎heteroaryl-SO₂-;
wherein each of said optionally substituted R⁹ groups may be optionally substituted with one to three substituents independently selected from the group consisting of: halo, (C₁-C₆)alkyl, -CN, hydroxy, (C₁-C₆)alkoxy, (C₁-C₆)alkyl-(C=O)O-, NH₂, (C₁-C₆)alkyl-NH-, [(C₁-C₆)alkyl]₂-N-, ((C₁-C₆)alkyl)-(C=O)NH-, (C₁-C₆)alkyl-(C=O)-, H₂N(C=O)-, (C₁-C₆)alkyl-NH-(C=O)- and [(C₁-C₆)alkyl]₂-N-(C=O)-;
wherein the molecular weight of said compound of formula I is less than 700 AMU;
selected from the group consisting of:
(3-{4-Chloro-3-[(1-hydroxy-cycloheptylmethyl)-carbamoyl]-phenyl}-5-methyl-pyrazol-1-yl)-acetic acid methyl ester;
2-Chloro-5-(1-cyanomethyl-1H-pyrazol-3-yl)-N-(1-hydroxycycloheptylmethyl)-benzamide;
2-Chloro-5-(1-cyanomethyl-5-methyl-1H-pyrazol-3-yl)-N-(1-hydroxycycloheptylmethyl)-benzamide;
2-Chloro-5-(5-cyclopropyl-1H-pyrazol-3-yl)-N-(1-hydroxycycloheptylmethyl)-benzamide;
2-Chloro-5-(6-methyl-pyridin-3-yl)-N-(1-p-tolyl-cyclohexylmethyl)-benzamide;
2-Chloro-5-[1-(2,3-dihydroxy-2-methyl-propyl)-5-methyl-1H-pyrazol-3-yl]-N-(1-hydroxy-cycloheptylmethyl)-benzamide;
2-Chloro-5-[1-(3-fluoro-2-hydroxy-propyl)-1H-imidazol-4-yl]-N-(1-hydroxy-cycloheptylmethyl)-benzamide;
2-Chloro-5-[2-(2,3-dihydroxy-propyl)-5-methyl-2H-pyrazol-3-yl]-N-(1-hydroxy-cycloheptylmethyl)-benzamide;
2-Chloro-N-(1-cyano-cycloheptylmethyl)-5-(5-methyl-2H-pyrazol-3-yl)-benzamide;
2-Chloro-N-(1-hydroxy-3,3-dimethyl-cyclohexylmethyl)-5-(3-methylisoxazol-5-yl)-benzamide;
2-Chloro-N-(1-hydroxy-3,3-dimethyl-cyclohexylmethyl)-5-(5-methyl-2H-pyrazol-3-yl)-benzamide;
2-Chloro-N-(1-hydroxy-3,3-dimethyl-cyclohexylmethyl)-5-(5-methylpyridin-2-yl)-benzamide;
2-Chloro-N-(1-hydroxy-3,3-dimethyl-cyclohexylmethyl)-5-(5-trifluoromethyl-1H-pyrazol-3-yl)-benzamide;
2-Chloro-N-(1-hydroxy-cycloheptylmethyl)-5-(1H-imidazol-4-yl)-benzamide;
2-Chloro-N-(1-hydroxy-cycloheptylmethyl)-5-(1H-pyrazol-3-yl)-benzamide;
2-Chloro-N-(1-hydroxy-cycloheptylmethyl)-5-(1-methyl-1H-pyrazol-3-yl)-benzamide;
2-Chloro-N-(1-hydroxy-cycloheptylmethyl)-5-(2H-[1,2,3]triazol-4-yl)-benzamide; .
2-Chloro-N-(1-hydroxy-cycloheptylmethyl)-5-(2-methyl-2H-pyrazol-3-yl)-benzamide;
2-Chloro-N-(1-hydroxy-cycloheptylmethyl)-5-(2-methyl-thiazol-4-yl)-benzamide;
2-Chloro-N-(1-hydroxy-cycloheptylmethyl)-5-(3-methyl-isoxazol-5-yl)-benzamide;
2-Chloro-N-(1-hydroxy-cycloheptylmethyl)-5-(5-methyl-1-oxiranylmethyl-1H-pyrazol-3-yl)-benzamide;
2-Chloro-N-(1-hydroxy-cycloheptylmethyl)-5-(5-methyl-pyridin-2-yl)-benzamide;
2-Chloro-N-(1-hydroxy-cycloheptylmethyl)-5-(5-trifluoromethyl-1H-pyrazol-3-yl)-benzamide;
2-Chloro-N-(1-hydroxy-cycloheptylmethyl)-5-(6-methoxy-pyridazin-3-yl)-benzamide;
2-Chloro-N-(1-hydroxy-cycloheptylmethyl)-5-(hydroxyimino-methyl)-benzamide;
2-Chloro-N-(1-hydroxy-cycloheptylmethyl)-5-[1-(2-hydroxy-2-methylpropyl)-1H-pyrazol-3-yl]-benzamide;
2-Chloro-N-(1-hydroxy-cycloheptylmethyl)-5-[1-(2-hydroxy-2-methylpropyl)-5-methyl-1H-pyrazol-3-yl]-benzamide;
2-Chloro-N-(1-hydroxy-cycloheptylmethyl)-5-[1-(2-hydroxy-ethyl)-1H-pyrazol-3-yl]-benzamide;
2-Chloro-N-(1-hydroxy-cycloheptylmethyl)-5-[1-(2-hydroxy-ethyl)-6-oxo-1,6-dihydro-pyridazin-3-yl]-benzamide;
2-Chloro-N-(1-hydroxy-cycloheptylmethyl)-5-[2-(2-hydroxy-ethyl)-2H-pyrazol-3-yl]-benzamide;
2-Chloro-N-(1-hydroxy-cycloheptylmethyl)-5-[2-(2-hydroxy-ethyl)-5-methyl-2H-pyrazol-3-yl]-benzamide;
2-Chloro-N-(1-hydroxy-cycloheptylmethyl)-5-[5-(2-hydroxy-ethyl)-isoxazol-3-yl]-benzamide;
2-Chloro-N-(1-hydroxy-cycloheptylmethyl)-5-[5-methyl-l-(2-oxo-2-pyimlidin-1-yl-ethyl)-1H-pyrazol-3-yl]-benzamide;
2-Chloro-N-(1-hydroxy-cycloheptylmethyl)-5-[5-methyl-1-(pyrimidin-2-ylcarbamoylmethyl)-1H-pyrazol-3-yl]-benzamide;
2-Chloro-N-(1-hydroxy-cycloheptylmethyl)-5-{1-[(1-hydroxymethylpropylcarbamoyl)-methyl]-5-methyl-1H-pyrazol-3-yl}-benzamide;
2-Chlore-N-(1-hydroxy-cycloheptylmethyl)-5-{1-[(2-hydroxypropylcarbamoyl)-methyl]-5-methyl-1H-pyrazol-3-yl}-benzamide;
2-Chloro-N-[2-(2-chloro-phenyl)-ethyl]-5-(1H-pyrazol-3-yl)-benzamide;
2-Chloro-N-[2-(2-chloro-phenyl)-ethyl]-5-(1H-pyrazol-4-yl)-benzamide;
2-Chloro-N-[2-(2-chloro-phenyl)-ethyl]-5-(2-methyl-pyrimidin-4-yl)-benzamide;
2-Chloro-N-[2-(2-chloro-phenyl)-ethyl]-5-(5-ethyl-2H-pyrazol-3-yl)-benzamide;
2-Chloro-N-[2-(2-chloro-pbenyl)-ethyl]-5-(6-methoxy-pyridazin 3-yl)-benzamide;
2-Chloro-N-[2-(2-chloro-phenyl)-ethyl]-5-[1-(4-methoxy-benzyl)-1H-pyrazol-4-yl]-benzamide,
5-(1-Carbamoylmethyl-1H-pyrazol-3-yl)-2-chloro-N-(1-hydroxy-cycloheptylmethyl)-benzamide;
5-(1-Carbamoylmethyl-5-methyl-1H-pyrazol-3-yl)-2-chloro-N-(1-hydroxy-cyclohoptylmethyl)-benzamide;
5-[1-(3-Amino-2-hydroxy-propyl)-5-methyl-1H-pyrazol-3-yl]-2-chloro-N-(1-hydroxy-cycloheptylmethyl)-benzamide Hydrochloride; and
5-[2-(3-Amino-2-hydroxy-propyl)-5-methyl-2H-pyrazol-3-yl]-2-chloro-N-(1-hydroxy-cycloheptylmethyl)-benzamide;
or a pharmaceutically acceptable salt thereof.

The present invention also relates to the pharmaceutically acceptable acid addition salts of compounds of the formula I. The acids which are used to prepare the pharmaceutically acceptable acid addition salts of the aforementioned base compounds of this invention are those which form non-toxic acid addition salts, i.e., salts containing pharmacologically acceptable anions, such as the chloride, bromide, iodide, nitrate, sulfate, bisulfate, phosphate, acid phosphate, acetate, lactate, citrate, acid citrate, tartrate, bitartrate, succinate, maleate, fumarate, gluconate, saccharate, benzoate, methanesulfonate, ethanesulfonate, benzenesulfonate, p-toluenesulfonate and pamoate [i.e., 1,1'-methylene-bis-(2-hydroxy-3- naphthoate)]salts.

The invention also relates to base addition salts of formula I. The chemical bases that may be used as reagents to prepare pharmaceutically acceptable base salts of those compounds of formula I that are acidic in nature are those that form non-toxic base salts with such compounds. Such non-toxic base salts include, but are not limited to those derived from such pharmacologically acceptable cations such as alkali metal cations (e.g., potassium and sodium) and alkaline earth metal cations (e.g., calcium and magnesium), ammonium or water-soluble amine addition salts such as N-methylglucamine-(meglumine), and the lower alkanolammonium and other base salts of pharmaceutically acceptable organic amines.

This invention also encompasses pharmaceutical compositions containing prodrugs of compounds of the formula I. Compounds of formula I having free amino, amido, hydroxy or carboxylic groups can be converted into prodrugs. Prodrugs include compounds wherein an amino acid residue, or a polypeptide chain of two or more (e.g., two, three or four) amino acid residues which are covalently joined through peptide bonds to free amino, hydroxy or carboxylic acid groups of compounds of formula I. The amino acid residues include the 20 naturally occurring amino acids commonly designated by three letter symbols and also include, 4-hydroxyproline, hydroxylysine, demosine, isodemosine, 3-methylhistidine, norvalin, beta-alanine, gamma-aminobutyric acid, citrulline, homocysteine, homoserine, ornithine and methionine sulfone. Prodrugs also include compounds wherein carbonates, carbamates, amides and alkyl esters which are covalently bonded to the above substituents of formula I through the carbonyl carbon prodrug sidechain.

The compounds of this invention include all stereoisomers (e.g., cis and trans isomers) and all optical isomers of compounds of the formula I (e.g., R and S enantiomers), as well as racemic, diastereomeric and other mixtures of such isomers.

The compounds, and salts of the present invention can exist in several tautomeric forms, including the enol and imine form, and the keto and enamine form and geometric isomers and mixtures thereof. All such tautomeric forms are included within the scope of the present invention. Tautomers exist as mixtures of a tautomeric set in solution. In solid form, usually one tautomer predominates. Even though one tautomer may be described, the present invention includes all tautomers of the present compounds. One example of a tautomeric structure is when R³ is a group of the formula

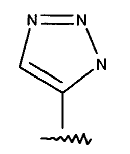

One skilled in the art will appreciate that this group can also be drawn as its tautomer

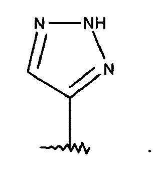

The present invention also includes atropisomers of the present invention. Atropisomers refer to compounds of formula I that can be separated into rotationally restricted isomers.

The compounds of this invention may contain olefin-like double bonds. When such bonds are present, the compounds of the invention exist as cis and trans configurations and as mixtures thereof.

As used herein, the term "spiro" refers to a connection between two groups, substituents etc., wherein the connection occurs at the same carbon atom such as can be depicted according to the following formula

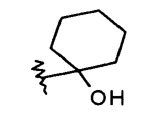

As used herein, the term "alkyl," as well as the alkyl moieties of other groups referred to herein (e.g., alkoxy), may be linear or branched (such as methyl, ethyl, *n-*propyl, isopropyl, *n*-butyl, *iso*-butyl, *secondary*-butyl*, tertiary-*butyl)*;* optionally substituted by 1 to 3 suitable substituents as defined above such as fluoro, chloro, trifluoromethyl, (C₁-C₆)alkoxy, (C₆-C₁₀)aryloxy, trifluoromethoxy, difluoromethoxy or (C₁-C₆)alkyl. The phrase "each of said alkyl" as used herein refers to any of the preceding alkyl moieties within a group such alkoxy, alkenyl or alkylamino. Preferred alkyls include (C₁-C₆)alkyl, more preferred are (C₁-C₄)alkyl, and most preferred are methyl and ethyl.

As used herein, the term "cycloalkyl" refers to a mono, bicyclic or tricyclic carbocyclic ring (e.g., cyclopropyl, cyclobutyl, cyclopentyl, cyclohexyl, cycloheptyl, cyclooctyl, cyclononyl, cyclopentenyl, cyclohexenyl, bicyclo[2.2.1]heptanyl, bicyclo[3.2.1]octanyl and bicyclo[5.2.0]nonanyl, etc.); optionally containing 1 or 2 double bonds and optionally substituted by 1 to 3 suitable substituents as defined above such as fluoro, chloro, trifluoromethyl, (C₁-C₆)alkoxy, (C₆-C₁₀)aryloxy, trifluoromethoxy, difluoromethoxy or (C₁-C₆)alkyl.

As used herein, the term "halogen" includes fluoro, chloro, bromo or iodo or fluoride, chloride, bromide or iodide.

As used herein, the term "carbonyl" or "(C=O)" (as used in phrases such as alkylcarbonyl, alkyl-(C=O)- or alkoxycarbonyl) refers to the joinder of the >C=O moiety to a second moiety such as an alkyl or amino group (i.e. an amido group). Alkoxycarbonylamino (i.e. alkoxy(C=O)-NH-) refers to an alkyl carbamate group. The carbonyl group is also equivalently defined herein as (C=O). Alkylcarbonylamino refers to groups such as acetamide.

As used herein, the term "oxo" is used herein to mean a double bonded oxygen (=O) radical wherein the bond partner is a carbon atom. Such a radical can also be thought as a carbonyl group.

As used herein, the term "aryl" means aromatic radicals such as phenyl, naphthyl, tetrahydronaphthyl, indanyl and the like; optionally substituted by 1 to 3 suitable substituents as defined above such as fluoro, chloro, trifluoromethyl, (C₁-C₆)alkoxy, (C₆-C₁₀)aryloxy, trifluoromethoxy, difluoromethoxy or (C₁-C₆)alkyl.

As used herein, the term "heteroaryl" refers to an aromatic heterocyclic group usually with one heteroatom selected from O, S and N in the ring. In addition to said heteroatom, the aromatic group may optionally have up to four N atoms in the ring. For example, heteroaryl group includes pyridyl, pyrazinyl, pyrimidinyl, pyridazinyl, thienyl, furyl, imidazolyl, pyrrolyl, oxazolyl (e.g., 1,3-oxazolyl, 1,2-oxazolyl), thiazolyl (e.g., 1,2-thiazolyl, 1,3-thiazolyl), pyrazolyl, tetrazolyl, triazolyl (e.g., 1,2,3-triazolyl, 1,2,4-triazolyl), oxadiazolyl (e.g., 1,2,3-oxadiazolyl), thiadiazolyl (e.g., 1,3,4-thiadiazolyl), quinolyl, isoquinolyl, benzothienyl, benzofuryl, indolyl, and the like; optionally substituted by 1 to 3 suitable substituents as defined above such as fluoro, chloro, trifluoromethyl, (C₁-C₆)alkoxy, (C₆-C₁₀)aryloxy, trifluoromethoxy, difluoromethoxy or (C₁-C₆)alkyl. Particularly preferred R³ heteroaryl groups include oxazolyl, imidazolyl, pyridyl, thienyl, furyl, thiazolyl, pyridazinyl and pyrazolyl.

The term "heterocyclic" or "heterocyclyl" as used herein refers to a cyclic group containing 1-10 carbon atoms and 1 to 4 hetero atoms selected from N, O, S(O)ₙ or NR (wherein "R" is a suitable substituent as defined above). A heterocyclic group may include a mono bicyclic or tricyclic ring having 1-10 carbon atoms and 1-4 hetero atoms selected from N, O S(O)n or NR. Examples of such rings include azetidinyl, tetrahydrofuranyl, imidazolidinyl, pyrrolidinyl, piperidinyl, piperazinyl, oxazolidinyl, thiazolidinyl, pyrazolidinyl, thiomorpholinyl, tetrahydrothiazinyl, tetrahydro-thiadiazinyl, morpholinyl, oxetanyl, tetrahydrodiazinyl, oxazinyl, oxathiazinyl, indolinyl, isoindolinyl, quinuclidinyl, chromanyl, isochromanyl, benzoxazinyl, and the like. Examples of said monocyclic saturated or partially saturated ring systems are tetrahydrofuran-2-yl, tetrahydrofuran-3-yl, imidazolidin-1-yl, imidazolidin-2-yl, imidazolidin-4-yl, pyrrolidin-1-yl, pyrrolidin-2-yl, pyrrolidin-3-yl, piperidin-1-yl, piperidin-2-yl, piperidin-3-yl, piperazin-1-yl, piperazin-2-yl, piperazin-3-yl, 1,3-oxazolidin-3-yl, isothiazolidine, 1,3-thiazolidin-3-yl, 1,2-pyrazolidin-2-yl, 1,3-pyrazolidin-1-yl, thiomorpholin-yl, 1,2-tetrahydrothiazin-2-yl, 1,3-tetrahydrothiazin-3-yl, tetrahydrothiadiazin-yl, morpholin-yl, 1,2-tetrahydrodiazin-2-yl, 1,3-tetrahydrodiazin-1-yl, 1,4-oxazin-2-yl, 1,2,5-oxathiazin-4-yl and the like; optionally containing 1 or 2 double bonds and optionally substituted by 1 to 3 suitable substituents as defined above such as fluoro, chloro, trifluoromethyl, (C₁-C₆)alkoxy, (C₆-C₁₀)aryloxy, trifluoromethoxy, difluoromethoxy or (C₁-C₆)alkyl. Preferred R³ heterocyclics include tetrahydrofuranyl, pyrrolidinyl, piperidinyl, piperazinyl and morpholinyl.

Nitrogen heteroatoms as used herein refers to N=, >N and -NH; wherein -N= refers to a nitrogen double bond; >N refers to a nitrogen containing two bond connections and -N refers to a nitrogen containing one bond.

"Embodiment" as used herein refers to specific groupings of compounds or uses into discrete subgenera. Such subgenera may be cognizable according to one particular substituent such as a specific R¹ or R³ group. Other subgenera are cognizable according to combinations of various substituents, such as all compounds
wherein R² is chloro and R¹ is (C₁-C₄)alkyl, optionally substituted by (C₃-C₁₀)cycloalkyl. The phrase "in combination with each of the aforementioned embodiments" refers to combinations of the identified embodiment with each embodiment previously identified in the specification. Thus an embodiment of compounds wherein R¹ is (C₁-C₄)alkyl, optionally substituted by (C₃-C₁₀)cycloalkyl "in combination with each of the aforementioned embodiments" refers to additional embodiments comprising combinations with each embodiment previously identified in the specification.

One embodiment of the invention includes compounds of formula (I) in which R² is halogen and (C₁-C₆)alkyl, and preferably compounds in which R² is chloro, methyl or ethyl.

Another embodiment of the invention includes compounds of formula (I) wherein R¹ is (C₁-C₁₂)alkyl optionally substituted by one to six (preferably one to three) radicals independently selected from the group consisting of: hydroxy, halogen, -CN, (C₁-C₆)alkyl, HO(C₁-C₆)alkyl, (C₁-C₆ )alkyl-NH(C=O)-, NH₂(C=O)- and (C₁-C₆)alkoxy.

Another embodiment of the invention includes compounds of formula (I) wherein R¹ is optionally substituted (C₁-C₂)alkyl.

Another embodiment of the invention includes compounds of formula (I) wherein R¹ is (C₁-C₄)alkyl substituted with one or two (preferably one) (C₃-C₁₀)cycloalkyl; wherein said (C₁-C₄)alkyl (more preferably (C₁-C₂)alkyl) is also optionally substituted by one to three radicals independently selected from the group consisting of: hydroxy, halogen, -CN, (C₁-C₆)alkyl, HO(C₁-C₆)alkyl, (C₁-C₆)alkyl-NH(C=O)-, NH₂(C=O)- and (C₁-C₆)alkoxy; wherein said (C₃-C₁₀)cycloalkyl is optionally substituted by one to three radicals independently selected from hydroxy, halogen, -CN, (C₁-C₆)alkyl, HO(C₁-C₆)alkyl, (C₁-C₆)alkyl-NH(C=O)-, NH₂(C=O)-, (C₁-C₆)alkoxy and (C₃-C₁₀)cycloalkyl; wherein said (C₃-C₁₀)cycloalkyl radical is optionally substituted by one to three moieties independently selected from halogen and (C₁-C₆)alkyl.

Another embodiment of the invention includes those compounds of formula (I) wherein R¹ is (C₁-C₄)alkyl (more preferably (C₁-C₂)alkyl) substituted with one or two (preferably one) (C₃-C₁₀)cycloalkyl; wherein said (C₃-C₁₀)cycloalkyl is spiro substituted at the (C₁-C₄)alkyl linkage by a radical selected from the group consisting of: hydroxy, halogen, -CN, (C₁-C₆)alkyl, HO(C₁-C₆)alkyl, (C₁-C₆)alkyl-NH(C=O)-, NH₂(C=O)-, (C₁-C₆)alkoxy and (C₆-C₁₀)aryl.

Another embodiment of the invention includes those compounds of formula (I) wherein R¹ is (C₁-C₄)alkyl substituted with one or two (preferably one) (C₆-C₁₀)aryl; wherein said (C₁-C₄)alkyl (more preferably (C₁-C₂)alkyl) is also optionally substituted by one to three radicals independently selected from the group consisting of: hydroxy, halogen, -CN, (C₁-C₆)alkyl, HO(C₁-C₆)alkyl, (C₁-C₆)alkyl-NH(C=O)-, H₂N (C=O)- and (C₁-C₆)alkoxy; wherein said (C₆-C₁₀)aryl is optionally substituted by one to three radicals independently selected from hydroxy, halogen, -CN, (C₁-C₆)alkyl, HO(C₁-C₆)alkyl, (C₁-C₆)alkyl-NH(C=O)-, NH₂(C=O)-, (C₁-C₆)alkoxy and (C₃-C₁₀)cycloalkyl; wherein said (C₃-C₁₀)cycloalkyl radical is optionally substituted by one to three moieties independently selected from halogen and (C₁-C₆)alkyl.

Another embodiment of the invention includes those compounds of formula (I) wherein R¹ is (C₁-C₄)alkyl substituted with one or two (preferably one) (C₁-C₁₀)heteroaryl; wherein said (C₁-C₄)alkyl (more preferably (C₁-C₂)alkyl) is also optionally substituted by one to three radicals independently selected from the group consisting of: hydroxy, halogen, -CN, (C₁-C₆)alkyl, HO(C₁-C₆)alkyl, (C₁-C₆)alkyl-NH(C=O)-, NH₂(C=O)- and (C₁-C₆)alkoxy; wherein said (C₁-C₁₀)heteroaryl is optionally substituted by one to three radicals independently selected from hydroxy, halogen, -CN, (C₁-C₆)alkyl, HO(C₁-C₆)alkyl, (C₁-C₆)alkyl-NH(C=O)-, NH₂(C=O)-, (C₁-C₆)alkoxy and (C₃-C₁₀)cycloalkyl; wherein said (C₃-C₁₀)cycloalkyl radical is optionally substituted by one to three moieties independently selected from halogen and (C₁-C₆)alkyl.

Another embodiment of the invention are compounds of formula (I) in which R³ is a carbon linked (C₁-C₁₀)heteroaryl or (C₁-C₁₀)heterocyclyl optionally substituted on any carbon or nitrogen atom capable of substitution with one to three R⁴ independently selected from the group consisting of: hydrogen, hydroxy, halogen, and -CN; more preferably one or two R⁴ per ring; wherein one of said R⁴ is hydrogen, - CN or halo.

In certain embodiment of formula I are compounds wherein R² is halo or (C₁-C₆)alkyl. In certain embodiment of formula I are compounds wherein R¹ is (C₁-C₂)alkyl substituted with one or two (C₃-C₁₀)cycloalkyl; wherein said (C₁-C₂)alkyl is also optionally substituted by one to three radicals independently selected from the group consisting of: hydroxy, halo, -CN, (C₁-C₆)alkyl, HO(C₁-C₆)alkyl, (C₁-C₆)alkyl-NH(C=O)-, NH₂(C=O)- and (C₁-C₆)alkoxy; wherein said (C₃-C₁₀)cycloalkyl is optionally substituted by one to three radicals independently selected from: hydroxy, halo, -CN, (C₁-C₆)alkyl, HO(C₁-C₆)alkyl, (C₁-C₆)alkyl-NH(C=O)-, NH₂(C=O)-, (C₁-C₆)alkoxy and (C₃-C₁₀)cycloalkyl; and said (C₃-C₁₀)cycloalkyl may be optionally spiro substituted at the (C₁-C₂)alkyl linkage by a radical selected from the group consisting of: hydroxy, halo, -CN, (C₁-C₆)alkyl, HO(C₁-C₆)alkyl, (C₁-C₆)alkyl-NH(C=O)-, NH₂(C=O)-, (C₁-C₆)alkoxy and (C₆-C₁₀)aryl.

In certain embodiment of formula I are compounds wherein R³ is a carbon linked (C₁-C₁₀)heteroaryl or (C₁-C₁₀)heterocyclyl substituted on a carbon atom capable of substitution with one to three R⁴; wherein said R⁴ is (C₁-C₆)alkoxy or phenoxy; wherein R⁴ is optionally substituted with one to three substituents independently selected from the group consisting of: halo, hydroxy, -CN, -NH₂, and (C₁-C₆)alkoxy and optionally substituted R⁶ substituents selected from the group consisting of: (C₁-C₆)alkyl-(C=O)- and (C₁-C₆)alkyl-O(C=O)-; or wherein R⁴ is optionally substituted with a (C₁-C₁₀)heterocyclyl that may be optionally substituted with 1 substituent selected from the group consisting of: halo, CF₃, -CN, (C₁-C₆)alkyl, hydroxy, (C₁-C₆)alkoxy, (C₁-C₆)alkyl-O(C=O)-, (C₁-C₆)alkyl-SO₂-, H₂N-SO₂-, [(C₁-C₆)alkyl]NH-SO₂-, [(C₁-C₆)alkyl]₂N-SO₂-, (C₁-C₆)alkyl-NH-, [(C₁-C₆)alkyl]₂-N-, (C₆-C₁₀)aryl-NH-, NH₂(C=O)-, (C₁-C₆)alkyl-NH-(C=O)- and [(C₁-C₆)alkyl]₂-N-(C=O)-.

In certain embodiment of formula I are compounds wherein R³ is a carbon linked (C₁-C₁₀)heteroaryl or (C₁-C₁₀)heterocyclyl substituted on any carbon atom capable of substitution with one to three optionally substituted R⁶ substituents independently selected from the group consisting of: (C₃-C₁₀)cycloalkyl, (C₁-C₁₀)heterocyclyl, (C₆-C₁₀)aryl, and (C₁-C₁₀)heteroaryl.

In certain embodiment of formula I are compounds wherein R³ is a carbon linked (C₁-C₁₀)heteroaryl or (C₁-C₁₀)heterocyclyl substituted on any carbon atom capable of substitution with one R⁴ selected from the group consisting of: H₂NSO₂-, and an optionally substituted R⁶ substituent selected from the group consisting of: (C₁-C₆)alkyl-SO₂-, (C₁-C₆)alkyl-NH-SO₂- and ((C₁-C₆)alkyl)₂N-SO₂-.

In certain embodiment of formula I are compounds wherein R³ is a carbon linked (C₁-C₁₀)heteroaryl or (C₁-C₁₀)heterocyclyl substituted on any nitrogen atom capable of substitution with one R⁵ per ring; wherein R⁵ is a (C₁-C₆)alkyl- which may be optionally substituted with one to three substituents independently selected from the group consisting of: halo, -CN, hydroxy, (C₁-C₆)alkoxy, (C₁-C₆)alkyl-(C=O)O-, H₂N-(C=O)O-,(C₁-C₆)alkyl-NH-(C=O)O-, ((C₁-C₆)alkyl)₂N-(C=O)O-, NH₂, (C₁-C₆)alkyl-NH-, [(C₁-C₆)alkyl]₂-N-,(C₁-C₆)alkyl)-(C=O)NH-, (C₁-C₆)alkyl)-NH-(C=O)NH-, (C₁-C₆)alkyl-(C=O)-, H₂N(C=O)-, [(C₁-C₆)alkyl]₂-N-(C=O)-, (C₁-C₆)alkyl-O(C=O)-, and an optionally substituted R⁹ selected from the group consisting of: (C₆-C₁₀)aryl, (C₁-C₆)alkyl-NH-(C=O)-, (C₃-C₁₀)cycloalkyl-NH(C=O)-, (C₁-C₁₀)heterocyclyl-NH-(C=O)-, (C₆-C₁₀)aryl-NH-(C=O)-, (C₁-C₁₀)heteroaryl-NH-(C=O)-, (C₃-C₁₀)cycloalkyl-(C=O)-, (C₁-C₁₀)heterocyclyl-(C=O)-, (C₆-C₁₀)aryl-(C=O)- and (C₁-C₁₀)heteroaryl-(C=O)-;
wherein said optionally substituted R⁹ may be substituted with one to three substituents independently selected from the group consisting of: halo, (C₁-C₆)alkyl, hydroxy, (C₁-C₆)alkoxy, NH₂, (C₁-C₆)alkyl-NH-, [(C₁-C₆)alkyl]₂-N-, H₂N(C=O)-, (C₁-C₆)alkyl-NH-(C=O)- and [(C₁-C₆)alkyl]₂-N-(C=O)-.

In certain embodiment of formula I are compounds wherein R³ is a suitably substituted five-membered carbon linked (C₁-C₅)heterocyclyl of formula II(a)-II(j):

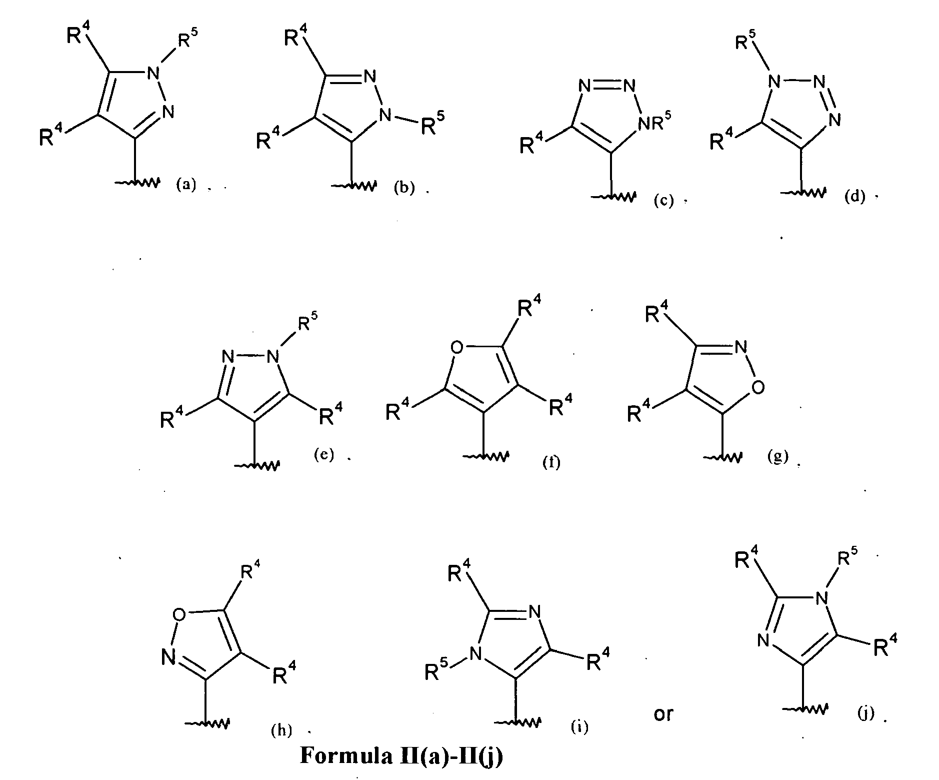

In certain embodiment of formula I are compounds wherein R³ is a six-membered carbon linked (C₁-C₆)heterocyclyl of formula III(a)-III(h), containing one to three R⁴ substitutents:

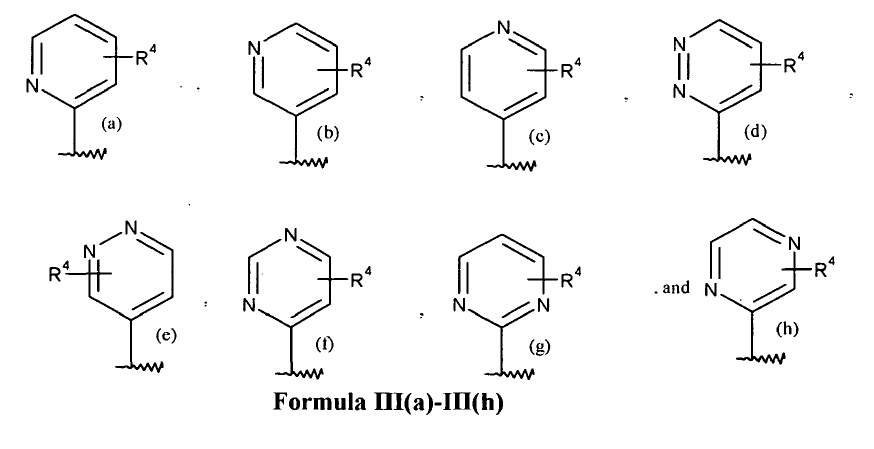

In certain embodiment of formula I are compounds wherein wherein R³ is a fused bicyclic carbon linked (C₁-C₁₀)heterocyclyl of formula IV(a)-IV(n), containing one to three R⁴:

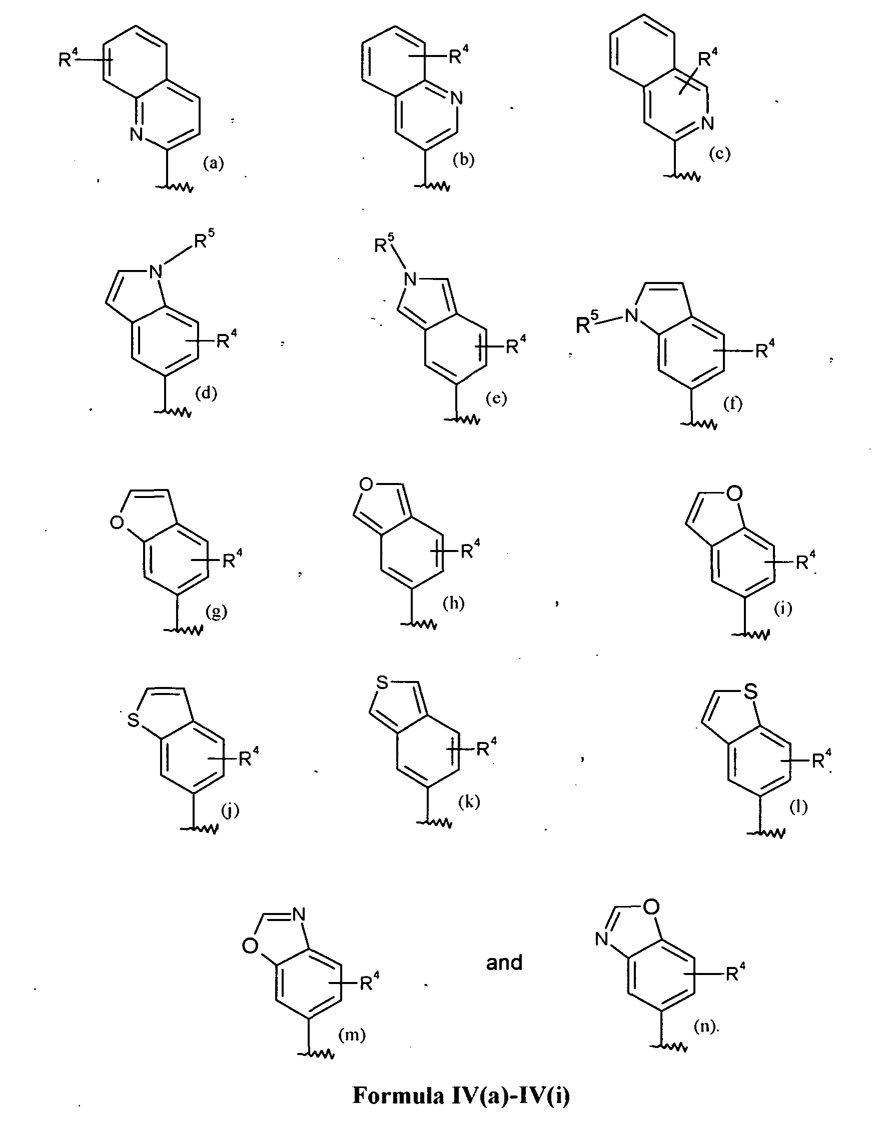

In certain embodiment of formula I are compounds wherein R³ is a suitably substituted oxo substituted, carbon linked (C₁-C₁₀)heterocyclyl of formula V(a)-V(d):

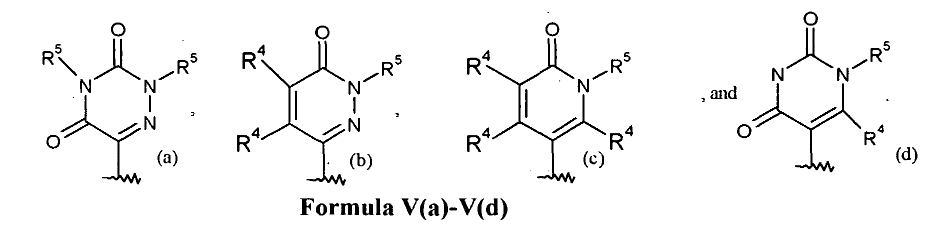

In certain embodiment of formula I are compounds of formula XXIX. 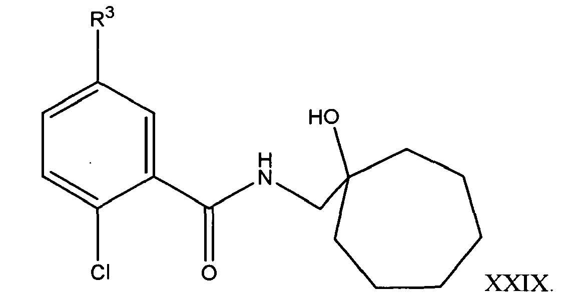 In certain embodiments of formula XXIX are compounds wherein R³ is a a suitably substituted five-membered carbon linked (C₁-C₅)heterocyclyl of formula II(a)-II(j).

Examples of compounds of formula I herein described are the following:
2-Chloro-5-[1-(2,3-dihydroxy-propyl)-5-methyl-1H-pyrazol-3-yl]-N-(1-hydroxy-cycloheptylmethyl)-benzamide;
2-Chloro-N-(1-hydroxy-cycloheptylmethyl)-5-[1-(2-hydroxy-3-methoxypropyl)-5-methyl-1H-pyrazol-3-yl]-benzamide;
5-[1-(2-Amino-ethyl)-1H-pyrazol-3-yl]-2-chloro-N-(1-hydroxycycloheptylmethyl)-benzamide;
5-[1-(2-Amino-ethyl)-5-methyl-1H-pyrazol-3-yl]-2-chloro-N-(1-hydroxycycloheptylmethyl)-benzamide;
2-Chloro-N-(1-hydroxy-cycloheptylmethyl)-5-[1-(2-hydroxy-ethyl)-5-methyl-1H-pyrazol-3-yl]-benzamide;
2-Chloro-5-(5-ethyl-2H-pyrazol-3-yl)-N-(1-hydroxy-cycloheptylmethyl)-benzamide;
2-Chloro-N-(1-hydroxy-cycloheptylmethyl)-5-(5-methyl-2H-pyrazol-3-yl)-benzamide;
2-Chloro-5-(5-methyl-pyridin-2-yl)-N-(1-p-tolyl-cyclohexylmethyl)-benzamide;
2-Chloro-5-(1H-pyrazol-3-yl)-N-(1-p-tolyl-cyclohexylmethyl)-benzamide;
2-Chloro-N-[2-(2-chloro-phenyl)-ethyl]-5-(6-oxo-1,6-dihydro-pyridazin-3-yl)-benzamide;
2-Chloro-N-(1-hydroxy-cycloheptylmethyl)-5-(6-oxo-1,6-dihydro-pyridazin-3-yl)-benzamide;
2-Chloro-N-[2-(2-chloro-phenyl)-ethyl]-5-(5-methyl-pyridin-2-yl)-benzamide;
2-Chloro-5- {1-[(2-dimethylamino-ethylcarbamoyl)-methyl]-5-methyl-1H-pyrazol-3-yl}-N-(1-hydroxy-cycloheptylmethyl)-benzamide;
2-Chloro- N-(1-hydroxy-cycloheptylmethyl)-5- {1-[(2-hydroxyethylcarbamoyl)-methyl]-5-methyl-1H-pyrazol-3-yl}-benzamide;
2-Chloro-N-(1-hydroxy-cycloheptylmethyl)-5- {1-[(2-hydroxy-1-methylethylcarbamoyl)-methyl]-5-methyl-1H-pyrazol-3-yl}-benzamide;
2-Chloro-N-(1-hydroxy-cycloheptylmethyl)-5- (5-methyl-1 - [(methylcarbamoylmethyl-carbamoyl)- methyl]-1H -pyrazol-3 -yl}-benzamide;
2-Chloro-N-(1-hydroxy-cycloheptylmethyl)-5- {1-[(2-hydroxy-1-hydroxymethyl-ethylcarbamoyl)-methyl]-5-methyl-1H-pyrazol-3-yl}-benzamide;
2-Chloro-5-{1-[2-(3,4-dihydroxy-pyrrolidin-1-yl)-2-oxo-ethyl]-5-methyl-1H-pyrazol-3-yl}-N-(1-hydroxy-cycloheptylmethyl)-benzamide; and
5-[1-(3-Amino-2-hydroxy-propyl)-5-methyl-1H-pyrazol-3-yl]-2-chloro- N-(1-hydroxy-cycloheptylmethyl)-benzamide.

Certain specific compounds of the invention are those compounds identified in Examples 1-58.

Other specific compounds described include:
2-Chloro-N-(1-hydroxy-cycloheptylmethyl)-5-[5-(2-hydroxy-ethyl)-2H-pyrazol-3-yl]-benzamide;
2-Chloro-N-(1-hydroxy-cyclooctylmethyl)-5-[5-(2-hydroxy-ethyl)-2H-pyrazol-3-yl]-benzamide;
2-Chloro-5-[5-(2-hydroxy-ethyl)-2H-pyrazol-3-yl]-N-(l-p-tolyl-cyclohexylmethyl)-benzamide;
2-Chloro-5-[5-(2-hydroxy-ethyl)-2H-pyrazol-3-yl]-N-(2-hydroxy-2-phenylethyl)-benzamide;
2-Chloro-N-[2-(2-chloro-phenyl)-ethyl]-5-[5-(2-hydroxy-ethyl)-2H-pyrazol-3-yl]-benzamide;
2-Chloro-N-(1-hydroxy-3,3-dimethyl-cyclohexylmethyl)-5-[5-(2-hydroxyethyl)-2H-pyrazol-3-yl]-benzamide;
N-(1-Hydroxy-cycloheptylmethyl)-5-[5-(2-hydroxy-ethyl)-2H-pyrazol-3-yl]-2-methyl-benzamide;
N-(1-Hydroxy-cyclooctylmethyl)-5-[5-(2-hydroxy-ethyl)-2H-pyrazol-3-yl]-2-methyl-benzamide;
5-[5-(2-Hydroxy-ethyl)-2H-pyrazol-3-yl]-2-methyl-N-(1-p-tolyl-cyclohexylmethyl)-benzamide;
5-[5-(2-Hydroxy-ethyl)-2H-pyrazol-3-yl]-N-(2-hydroxy-2-phenyl-ethyl)-2-methyl-benzamideN-[2-(2-Chloro-phenyl)-ethyl]-5-[5-(2-hydroxy-ethyl)-2H-pyrazol-3-yl]-2-methyl-benzamide;
N-(1-Hydroxy-3,3-dimethyl-cyclohexylmethyl)-5-[5-(2-hydroxy-ethyl)-2H-pyrazol-3-yl]-2-methyl-benzamide;
5-[1-(3-Amino-2-hydroxy-2-methyl-propyl)-5-cyclopropyl-1H-pyrazol-3-yl]-2-chloro-N-(1-hydroxy-cycloheptylmethyl)-benzamide;
5-[1-(3-Amino-2-hydroxy-2-methyl-propyl)-5-methyl-1H-pyrazol-3-yl]-2-chloro-N-(1-hydroxy-cyclooctylmethyl)-benzamide;
5-[1-(3-Amino-2-hydroxy-2-methyl-propyl)-5-methyl-1H-pyrazol-3-yl]-2-chloro-N-(1-hydroxy-3,3-dimethyl-cyclohexylmethyl)-benzamide;
5-[1-(3-Amino-2-hydroxy-2-methyl-propyl)-5-cyclopropyl-1H-pyrazol-3-yl]-2-chloro-N-(1-hydroxy-3,3-dimethyl-cyclohexylmethyl)-benzamide;
5-[1-(3-Amino-2-hydroxy-2-methyl-propyl)-5-methyl-1H-pyrazol-3-yl]-2-chloro-N-(1-hydroxy-cycloheptylmethyl)-benzamide;
5-[1-(3-Amino-2-hydroxy-2-methyl-propyl)-5-cyclopropyl-1H-pyrazol-3-yl]-2-chloro-N-(1-hydroxy-cyclooctylmethyl)-benzamide;
5-[5-(2-Amino-ethyl)-2H-pyrazol-3-yl]-2-chloro-N-(1-hydroxycycloheptylmethyl)-benzamide;
5-(1-Azetidin-3-yl-5-methyl-1H-pyrazol-3-yl)-2-chloro-N-(1-hydroxycyclooctylmethyl)-benzamide;
5-(1-Azetidin-3-yl-5-methyl-1H-pyrazol-3-yl)-2-chloro-N-(2-hydroxy-2-phenyl-ethyl)-benzamide;
5-(1-Azetidin-3-yl-5-cyclopropyl-1H-pyrazol-3-yl)-2-chloro-N-(2-hydroxy-2-phenyl-ethyl)-benzamide;
5-(1-Azetidin-3-yl-5-cyclopropyl-1H-pyrazol-3-yl)-2-chloro-N-(1-phenylcyclohexylmethyl)-benzamide;
2-Chloro-N-(1-hydroxy-cycloheptylmethyl)-5-[1-(4-hydroxy-pyrrolidin-3-yl)-5-methyl-1H-pyrazol-3-yl]-benzamide;
2-Chloro-N-(1-hydroxy-3,3-dimethyl-cyclohexylmethyl)-5-[1-(4-hydroxypyrrolidin-3-yl)-5-methyl-1H-pyrazol-3-yl]-benzamide;
2-Chloro-5-[5-cyclopropyl-1-(4-hydroxy-pyrrolidin-3-yl)-1H-pyrazol-3-yl]-N-(1-hydroxy-3,3-dimethyl-cyclohexylmethyl)-benzamide;
2-Chloro-N-(1-hydroxy-cycloheptylmethyl)-5-[5-methyl-1-(2-oxo-pyrrolidin-3-yl)-1H-pyrazol-3-yl]-benzamide;
2-Chloro-N-(1-hydroxy-cycloheptylmethyl)-5-[5-methyl-1-(5-oxo-pyrrolidin-3-yl)-1H-pyrazol-3-yl]-benzamide;
2-Chloro-N-(1-hydroxy-cycloheptylmethyl)-5-(5-methyl-1-pyrimidin-4-yl-1H-pyrazol-3-yl)-benzamide;
2-Chloro-5 -(5 -cyclopropyl-1-pyrimidin-4-yl-1H-pyrazol-3-yl)-N-(1-hydroxycycloheptylmethyl)-benzamide;
2-Chloro-5 -(5 -cyclopropyl-1-pyrimidin-4-yl-1H-pyrazol-3 -yl)-N-(1-hydroxy-3,3-dimethyl-cyclohexylmethyl)-benzamide;
2-Chloro-N-(1-hydroxy-cycloheptylmethyl)-5-[1-(2-hydroxy-ethyl)-5-oxo-4,5-dihydro-1H-[1,2,4]triazol-3-yl]-benzamide;
2-Chloro-5-[1-(2-hydroxy-ethyl)-5-oxo-4,5-dihydro-1H-[1,2,4]triazol-3-yl]-N-(1-phenyl-cyclohexylmethyl)-benzamide;
5-[1-(3-Amino-2-hydroxy-propyl)-5-oxo-4,5-dihydro-1H-[1,2,4]triazol-3-yl]-2-chloro-N-(1-hydroxy-cycloheptylmethyl)-benzamide2-Chloro-N-(1-hydroxycycloheptylmethyl)-5-[1,2,4]triazolo[4,3-a]pyridin-3-yl-benzamide;
5-[1-(3-Amino-2-hydroxy-propyl)-5-oxo-4,5-dihydro-1H-[1,2,4]triazol-3-yl]-2-chloro-N-(1-cyano-cycloheptylmethyl)-benzamide2-Chloro-N-[2-(2-chlorophenyl)-ethyl]-5-[1-(2,3-dihydroxy-propyl)-1H-indazol-3-yl]-benzamide;
2-Chloro-5-[1-(2,3-dihydroxy-propyl)-1H-indazol-3-yl]-N-(1-p-tolylcyclohexylmethyl)-benzamide2-Chloro-N-(1-hydroxy-cycloheptylmethyl)-5-(1H-indazol-3-yl)-benzamide;
2-Chloro-5-[1-(2,3 -dihydroxy-propyl)-1H-indazol-3-yl]-N-(1-hydroxycycloheptylmethyl)-benzamides-[5-(2-Amino-ethoxy)-1-methyl-1H-pyrazol-3-yl]-2-chloro-N-(1-hydroxy-cycloheptylmethyl)-benzamide;
5-[1-(3-Amino-2-hydroxy-propyl)-5-hydroxy-1H-pyrazol-3-yl]-2-chloro-N-(1-hydroxy-cycloheptylmethyl)-benzamide; and
2-Chloro-N-(1-hydroxy-cycloheptylmethyl)-5-(5-hydroxy-1H-pyrazol-3-yl)-benzamide.

The present invention also includes isotopically-labeled compounds, which are identical to those recited in Formula I, but for the fact that one or more atoms are replaced by an atom having an atomic mass or mass number different from the atomic mass or mass number usually found in nature. Examples of isotopes that can be incorporated into compounds of the invention include isotopes of hydrogen, carbon, nitrogen, oxygen, phosphorous, fluorine and chlorine, such as ²H, ³H, ¹³C, ¹⁴C, ¹⁵N, ¹⁸O, ¹⁷O, ³¹P, ³²P, ³⁵S, ¹⁸F and ³⁶Cl, respectively. Compounds of the present invention, prodrugs thereof, and pharmaceutically acceptable salts of said compounds or of said prodrugs which contain the aforementioned isotopes and/or other isotopes of other atoms are within the scope of this invention. Certain isotopically-labelled compounds of the present invention, for example those into which radioactive isotopes such as ³H and ¹⁴C are incorporated, are useful in drug and/or substrate tissue distribution assays. Tritiated, i.e., ³H, and carbon-14, i.e., ¹⁴C, isotopes are particularly preferred for their ease of preparation and detectability. Further, substitution with heavier isotopes such as deuterium, i.e., ²H, can afford certain therapeutic advantages resulting from greater metabolic stability, for example increased *in vivo* half-life or reduced dosage requirements and, hence, may be preferred in some circumstances. Isotopically-labelled compounds of Formula I of this invention and prodrugs thereof can generally be prepared by carrying out the procedures disclosed in the Schemes and/or in the Examples and Preparations below, by substituting a readily available isotopically-labelled reagent for a non-isotopically-labelled reagent.

The compounds of Formula I or a pharmaceutically acceptable salt thereof can be used in the manufacture of a medicament for the prophylactic or therapeutic treatment of any disease state in a human, or other mammal, which is exacerbated or caused by excessive or unregulated cytokine production by such mammal's cells, such as but not limited to monocytes and/or macrophages.

The present invention relates to a method for treating an IL-1 mediated disease in a mammal, preferably a human, in need thereof, which comprises administering to said mammal an effective amount of a compound of formula I.

The present invention also relates to a method for treating an IL-1 mediated condition. As defined herein, an "IL-1 mediated condition" includes but is not limited to a disease or disorder selected from the group consisting of: arthritis (including psoriatic arthritis, Reiter's syndrome, rheumatoid arthritis, gout, traumatic arthritis, rubella arthritis, rheumatoid spondylitis, osteoarthritis, gouty arthritis and acute synovitis), inflammatory bowel disease, Crohn's disease, emphysema, acute respiratory distress syndrome, adult respiratory distress syndrome, asthma, bronchitis chronic obstructive pulmonary disease, chronic pulmonary inflammatory disease, silicosis, pulmonary sarcoidosis, allergic reactions, allergic contact hypersensitivity, eczema, contact dermatitis, psoriasis, sunburn, cancer, tissue ulceration, restenosis, periodontal disease, epidermolysis bullosa, osteoporosis, bone resorption disease, loosening of artificial joint implants, atherosclerosis, aortic aneurysm, congestive heart failure, myocardial infarction, stroke, cerebral ischemia, head trauma, neurotrauma, spinal cord injury, neuro-degenerative disorders, Alzheimer's disease, Parkinson's disease, migraine, depression, peripheral neuropathy, pain, cerebral amyloid angiopathy, nootropic or cognition enhancement, amyotrophic lateral sclerosis, multiple sclerosis, ocular angiogenesis, corneal injury, macular degeneration, corneal scarring, scleritis, abnormal wound healing, burns, autoimmune disorders, Huntington's disease, diabetes, AIDS, cachexia, sepsis, septic shock, endotoxic shock, conjunctivitis shock, gram negative sepsis, toxic shock syndrome, cerebral malaria, cardiac and renal reperfusion injury, thrombosis, glomerularonephritis, graft vs. host reaction, allograft rejection, organ transplant toxicity, ulcerative colitis, or muscle degeneration, in a mammal, preferably a human, comprising administering to said mammal an amount of a compound to formula I, effective in treating such a condition.

The present invention relates to a pharmaceutical composition for the treatment of an IL-1 mediated disease in a mammal which comprises an effective amount of a compound according of formula I and a pharmaceutically acceptable carrier.

The present invention relates to a pharmaceutical composition for the treatment of an IL-1 mediated condition in a mammal, preferably a human, comprising an amount of a compound of formula I, effective in treating such a condition and a pharmaceutically acceptable carrier.

Preferably, the compounds of the invention are useful for the treatment of rheumatoid arthritis, osteoarthritis, psoriasis, allergic dermatitis, asthma, chronic obstructive pulmonary disease (COPD), hyperresponsiveness of the airway, septic shock, glomerulonephritis, irritable bowel disease, Crohn's disease, ulcerative colitis, atherosclerosis, growth and metastases of malignant cells, myoblastic leukemia, diabetes, Alzheimer's disease, meningitis, osteoporosis, burn injury, ischemic heart disease, stroke and varicose veins.

The present invention also describes a compound of formula (I), or a pharmaceutically acceptable salt or solvate thereof, as hereinbefore defined for use in therapy.

In another aspect, the invention describes the use of a compound of formula (I), or a pharmaceutically acceptable salt or solvate thereof, as hereinbefore defined in the manufacture of a medicament for use in therapy.

The invention further describes a method of treating osteoarthritis which comprises administering a therapeutically effective amount of a compound of formula (I), or a pharmaceutically acceptable salt or solvate thereof, as hereinbefore defined to a patient.

The invention further describes a method of effecting immunosuppression (e.g. in the treatment of rheumatoid arthritis, irritable bowel disease, atherosclerosis or psoriasis) which comprises administering a therapeutically effective amount of a compound of formula (I), or a pharmaceutically acceptable salt or solvate thereof, as hereinbefore defined to a patient.

The invention also describes a method of treating an obstructive airways disease (e.g. asthma or COPD) which comprises administering to a patient a therapeutically effective amount of a compound of formula (I), or a pharmaceutically acceptable salt or solvate thereof, as hereinbefore defined to a patient, preferably a human.

The present invention also relates to a compound of the formula: 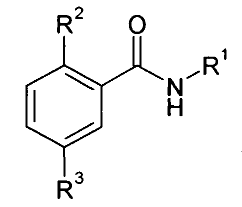 wherein R¹ is (C₁-C₆)alkyl optionally substituted with one or two radicals independently selected from hydroxy, halo, -CN, (C₁-C₆)alkyl, HO(C₁-C₆)alkyl, NH₂(C=O)-, (C₁-C₆)alkyl-NH-(C=O)-, [(C₁-C₆)alkyl]₂N-(C=O)-, oxo and (C₁-C₆)alkoxy;
wherein said R¹ (C₁-C₆)alkyl may also optionally be substituted with one or two groups independently selected from (C₁-C₆)alkyl, (C₃-C₁₀)cycloalkyl, (C₆-C₁₀)aryl and (C₁-C₁₀)heteroaryl; wherein said (C₃-C₁₀)cycloalkyl group is other than adamantyl; wherein said (C₁-C₆)alkyl and (C₃-C₁₀)cycloalkyl may be optionally substituted with oxo; wherein said (C₁-C₁₀)heteroaryl is selected from furanyl, thiophenyl, benzthiophenyl, benzfuranyl and chromanyl; wherein each of said (C₁-C₆)alkyl, (C₃-C₁₀)cycloalkyl, (C₆-C₁₀)aryl and (C₁-C₁₀)heteroaryl groups may also optionally be substituted by one to three radicals independently selected from hydroxy, halogen, -CN, (C₁-C₆)alkyl, HO(C₁-C₆)alkyl, NH₂(C=O)-, (C₁-C₆)alkyl-NH(C=O)-, [(C₁-C₆)alkyl]₂N-(C=O)-, (C₁-C₆)alkoxy, (C₆-C₁₀)aryl and (C₃-C₁₀)cycloalkyl; wherein said (C₃-C₁₀)cycloalkyl and (C₆-C₁₀)aryl radicals are optionally substituted by one to three moieties independently selected from halogen, - CN, (C₁-C₆)alkyl and (C₁-C₆)alkoxy;
R² is halogen, -CN or (C₁-C₆)alkyl; wherein said (C₁-C₆)alkyl is optionally substituted by one to three radicals independently selected from the group consisting of: halo, hydroxy, amino, -CN, (C₁-C₆)alkyl, (C₁-C₆)alkoxy, -CF₃, CF₃O-, NH₂, (C₁-C₆)alkyl-NH-, [(C₁-C₆)alkyl]₂-N-, (C₁-C₆)alkyl-S-, (C₁-C₆)alkyl-(S=O)-, (C₁-C₆)alkyl-(SO₂)-, (C₁-C₆)alkyl-O-(C=O)-, formyl, (C₁-C₆)alkyl-(C=O)-, and (C₃-C₆)cycloalkyl;
R³ is an optionally substituted carbon linked (C₁-C₁₀)heteroaryl or (C₁-C₁₀)heterocyclyl; wherein said optional substituents can be on any carbon atom of said (C₁-C₁₀)heteroaryl or (C₁-C₁₀)heterocyclyl capable of substitution with one to three R⁴ per ring; wherein said optional substituents can be on any nitrogen atom of said (C₁-C₁₀)heteroaryl or (C₁-C₁₀)heterocyclyl capable of substitution with one to two R⁵ per ring;
each R⁴ is independently selected from the group consisting of: halogen, -CN, (R⁶)n (C₁-C₆)alkyl, (R ⁶)n-(C₃-C₁₀)cycloalkyl, (R⁶)n-(C₁-C₁₀)heterocyclyl, (R⁶)n-(C₆-C₁₀)aryl, (R⁶)n-(C₁-C₁₀)heteroaryl, hydroxy, (R⁶)n (C₁-C₆)alkoxy, (R⁶)n-(C₁-C₆)alkyl-(C=O)O-; NH₂, (R⁶)n-(C₁-C₆)alkyl-NH-, [(R⁶)n-(C₁-C₆)alkylh-N-, (R⁶)ₙ-(C₆-C₁₀)aryl- NH -, (R⁶)ₙ-(C₁-C₆)alkyl-(C=O)NH-, (R⁶)ₙ-(C₁-C₆)alkyl-SO₂-NH-, (R⁶)ₙ-(C₁-C₆)alkyl-(C=O)-, H₂N(C=O)-, (R⁶)ₙ-(C₁-C₆)alkyl-NH-(C=O)-, [(R⁶)ₙ-(C₁-C₆)alkyl]₂-N-(C=O)-, (R⁶)ₙ-(C₁-C₆)alkyl-O(C=O)-, (R⁶)ₙ-(C₃-C₁₀)cycloalkyl-(C=O)-, (R⁶)ₙ-(C₁-C₁₀)heterocyclyl-(C=O)-, (R⁶)ₙ-(C₆-C₁₀)aryl-(C=O)-, (R⁶)ₙ-(C₁-C₁₀)heteroaryl-(C=O)-, (R⁶)ₙ-(C₁-C₆)alkyl-SO₂-, (R⁶)ₙ-(C₆-C₁₀)aryl-SO₂-, (R⁶)ₙ-(C₁-C₁₀)heteroaryl-SO₂-, H₂N-SO₂-, (R⁶)ₙ-(C₁-C₆)alkyl-NH-SO₂-, and [(R⁶)ₙ-(C₁-C₆)alkyl]₂N-SO₂-;
wherein any two R⁴ radicals on a carbon atom of said (C₁-C₁₀)heterocycyl can be taken together to form an oxo group or a spiro (C₃-C₆)carbocyclic or (C₁-C₆)heterocyclic group;
each R⁵ is independently selected from the group consisting of: (R⁸)ₘ-(C₁-C₆)alkyl, (R⁸)ₘ-(C₁-C₁₀)heterocyclyl, (R⁸)ₘ-(C₁-C₁₀)heteroaryl, (R⁸)ₘ-(C₁-C₆)alkyl-(C=O)-, H₂N(C=O)-, (R⁸)ₘ-(C₁-C₆)alkyl-NH-(C=O)-, [(R⁸)ₘ-(C₁-C₆)alkyl]₂-N-(C=O)-, (R⁸)ₘ-(C₁-C₆)alkyl-O(C=O)-, (R⁸)ₘ-(C₃-C₁₀)cycloalkyl-(C=O)-, (R⁸)ₘ-(C₁-C₁₀)heterocyclyl-(C=O)-, (R⁸)ₘ-(C₆-C₁₀)aryl-(C=O)- (R⁸)ₘ-(C₁-C₁₀)heteroaryl-(C=O)-, and (R⁸)ₘ-(C₁-C₆)alkyl-SO₂-;
wherein each of said R⁶ is independently selected from the group consisting of: hydrogen, halogen, -CN, (R⁷)p-(C₃-C₁₀)cycloalkyl, (R⁷)ₚ-(C₁-C₁₀)heterocyclyl, (R⁷)ₚ-(C₆-C₁₀)aryl, (R⁷)ₚ-(C₁-C₁₀)heteroaryl, hydroxy, (C₁-C₆)alkoxy, (R⁷)ₚ-phenoxy, (C₁-C₆)alkyl-(C=O)O-; NH₂, (R⁷)ₚ-(C₁-C₆)alkyl-NH-, [(R⁷)ₚ-(C₁-C₆)alkyl]₂-N-, (R⁷)ₚ-(C₆-C₁₀)aryl-NH-, (C₁-C₆)alkyl-SO₂-NH-, (C₁-C₆)alkyl-(C=O)-, H₂N(C=O)-, (C₁-C₆)alkyl-NH-(C=O)-, [(C₁-C₆)alkyl)₂-N-(C=O)-, (C₁-C₆)alkyl-O(C=O)-, (R⁷)ₚ-(C₃-C₁₀)cycloalkyl-(C=O)-, (R⁷)ₚ-(C₁-C₁₀)heterocyclyl-(C=O)-, (R⁷)ₚ-(C₆-C₁₀)aryl-(C=O)- (R⁷)ₚ-(C₁-C₁₀)heteroaryl-(C=O)-, (C₁-C₆)alkyl-SO₂-, (R⁷)ₚ-(C₆-C₁₀)aryl-SO₂-, (R⁷)ₚ-(C₁-C₁₀)heteroaryl-SO₂-, H₂N-SO₂-, (C₁-C₆)alkyl-NH-SO₂-, and [(C₁-C₆)alkyl]₂N-SO₂-;
wherein each of said R⁷ is independently selected from the group consisting of: hydrogen, halogen, CF₃, -CN, (C₁-C₆)alkyl, (C₁-C₁₀)heterocyclyl, (C₁-C₁₀)heteroaryl, hydroxy, (C₁-C₆)alkoxy, (C₁-C₆)alkyl-O(C=O)-, (C₁-C₆)alkyl-SO₂-NH-, (C₁-C₆)alkyl-SO₂-, (C₆-C₁₀)aryl-SO₂-, (C₁-C₁₀)heteroaryl-SO₂-, H₂N-SO₂-, (C₁-C₆)alkyl-NH-SO₂-, and [(C₁-C₆)alkyl]₂N-SO₂-;
wherein each of said R⁸ is independently selected from the group consisting of: hydrogen, halogen, -CN, (R⁹)ₜ-(C₆-C₁₀)aryl, hydroxy, (C₁-C₆)alkoxy, (C₁-C₆)alkyl-(C=O)O-; H₂N-(C=O)O-, (C₁-C₆)alkyl-NH-(C=O)O-, ((C₁-C₆)alkyl)₂N-(C=O)O-, NH₂, (C₁-C₆)alkyl-NH-, [(C₁-C₆)alkyl]₂-N-, (C₁-C₆)alkyl)-(C=O)NH-, (C₁-C₆)alkyl)-NH-(C=O)NH-, (C₁-C₆)alkyl-SO₂-NH-, (R⁹)ₜ-(C₃-C₁₀)cycloalkyl-NH(C=O)-, (R⁹)ₜ-(C₁-C₁₀)heterocyclyl-NH-(C=O)-, (R⁹)ₜ-(C₆-C₁₀)aryl-NH-(C=O)-, (R⁹)ₜ-(C₁-C₁₀)heteroaryl-NH-(C=O)-, (C₁-C₆)alkyl-(C=O)NH-, (C₁-C₆)alkyl-(C=O)-, H₂N(C=O)-, (C₁-C₆)alkyl-NH-(C=O)-, [(C₁-C₆)alkyl]-N-(C=O)-, (C₁-C₆)alkyl-O(C=O)-, (R⁹)ₜ(C₃-C₁₀)cycloalkyl-(C=O)-, (R⁹)ₜ-(C₁-C₁₀)heterocyclyl-(C=O)-, (R⁹)ₜ-(C₆-C₁₀)aryl-(C=O)-, (R⁹)ₜ-(C₁-C₁₀)heteroaryl-(C=O)-, (C₁-C₆)alkyl-SO₂-, (R⁹)ₜ-(C₆-C₁₀)aryl-SO₂-, (R⁹)ₜ-(C₁-C₁₀)heteroaryl-SO₂-, H₂N-SO₂-, (C₁-C₆)alkyl-NH-SO₂-, and [(C₁-C₆)alkylhN-SO₂-;
wherein each of said R⁹ is independently selected from the group consisting of: hydrogen, halogen, (C₁-C₆)alkyl, -CN, hydroxy, (C₁-C₆)alkoxy, (C₁-C₆)alkyl-(C=O)O-; NH₂, (C₁-C₆)alkyl-NH-, [(C₁-C₆)alkyl]₂-N-, ((C₁-C₆)alkyl)-(C=O)NH-, (C₁-C₆)alkyl-(C=O)-, H₂N(C=O)-, (C₁-C₆)alkyl-NH-(C=O)- and [(C₁-C₆)alkyl]₂-N-(C=O)-;
wherein each of m, n, p, and t are independently an integer from zero to three;
wherein the molecular weight of said compound of formula I is less than 700 AMU, preferably less than 550 AMU;
or the pharmaceutically acceptable salts or solvates thereof.

The term "treating", as used herein, refers to reversing, alleviating, inhibiting the progress of, or preventing the disorder or condition to which such term applies, or one or more symptoms of such disorder or condition. The term "treatment", as used herein, refers to the act of treating, as "treating" is defined immediately above.

The present invention also describes a pharmaceutical composition comprising a compound of formula (I), or a pharmaceutically acceptable salt or solvate thereof, as hereinbefore defined in association with a pharmaceutically acceptable adjuvant, diluent or carrier.

The invention further describes a process for the preparation of a pharmaceutical composition of the invention which comprises mixing a compound of formula (I), or a pharmaceutically acceptable salt or solvate thereof, as hereinbefore defined with a pharmaceutically acceptable adjuvant, diluent or carrier.

For the above-mentioned therapeutic uses the dosage administered will, of course, vary with the compound employed, the mode of administration, the treatment desired and the disorder indicated. The daily dosage of the compound of formula (I)/salt/solvate (active ingredient) may be in the range from 1 mg to 1 gram, preferably 1 mg to 250 mg, more preferably 10 mg to 100 mg.

The present invention also encompasses sustained release compositions.

The present invention also relates to processes of preparing the compounds of formula I and intermediates used in such processes.

One embodiment of the processes of the invention relates to the preparation of compounds of formula I, which may be carried out by one or more of the synthetic methods outlined in Schemes I-VIII, detailed below. The present invention also provides methods and intermediates useful in the synthesis of compounds of formula (I), and identified in Schemes I-VIII below.

One of ordinary skill in the art will appreciate that the compounds of the invention are useful in treating a diverse array of diseases. One of ordinary skill in the art will also appreciate that when using the compounds of the invention in the treatment of a specific disease that the compounds of the invention may be combined with various existing therapeutic agents used for that disease.

For the treatment of rheumatoid arthritis, the compounds of the invention may be combined with agents such as TNF-α inhibitors such as anti-TNF monoclonal antibodies (such as Remicade, CDP-870 and D₂E₇) and TNF receptor immunoglobulin molecules (such as Enbrel®), COX-2 inhibitors (such as meloxicam, celecoxib , rofecoxib, valdecoxib, paracoxib, and etoricoxib) low dose methotrexate, lefunomide; ciclesonide; hydroxychloroquine, d-penicillamine, auranofin or parenteral or oral gold.

The present invention still further relates to the combination of a compound of the invention together with a leukotriene biosynthesis inhibitor, 5-lipoxygenase (5-LO) inhibitor or 5-lipoxygenase activating protein (FLAP) antagonist selected from the group consisting of: zileuton; ABT-761; fenleuton; tepoxalin; Abbott-79175; Abbott-85761; *N*-(5-substituted)-thiophene-2-alkylsulfonamides; 2,6-di*-tert*butylphenol hydrazones; methoxytetrahydropyrans such as Zeneca ZD-2138; the compound SB-210661; pyridinyl-substituted 2-cyanonaphthalene compounds such as L-739,010; 2-cyanoquinoline compounds such as L-746,530; indole and quinoline compounds such as MK-591, MK-886, and BAY x 1005.

The present invention still further relates to the combination of a compound of the invention together with a receptor antagonists for leukotrienes LTB₄, LTC₄, LTD₄, and LTE₄ selected from the group consisting of: the phenothiazin-3-ones such as L-651,392; amidino compounds such as CGS-25019c; benzoxalamines such as ontazolast; benzenecarboximidamides such as BIIL 284/260; and compounds such as zafirlukast, ablukast, montelukast, pranlukast, verlukast (MK-679), RG-12525, Ro-245913, iralukast (CGP 45715A), and BAY x 7195.

The present invention still further relates to the combination of a compound of the invention together with a PDE4 inhibitor including inhibitors of the isoform PDE4D.

The present invention still further relates to the combination of a compound of the invention together with a antihistaminic H₁ receptor antagonists including cetirizine, loratadine, desloratadine, fexofenadine, astemizole, azelastine, and chlorpheniramine.

The present invention still further relates to the combination of a compound of the invention together with a gastroprotective H₂ receptor antagonist.

The present invention still further relates to the combination of a compound of the invention together with an α₁- and α₂-adrenoceptor agonist vasoconstrictor sympathomimetic agent, including propylhexedrine, phenylephrine, phenylpropanolamine, pseudoephedrine, naphazoline hydrochloride, oxymetazoline hydrochloride, tetrahydrozoline hydrochloride, xylometazoline hydrochloride, and ethylnorepinephrine hydrochloride.

The present invention still further relates to the combination of a compound of the invention together with anticholinergic agents including ipratropium bromide; tiotropium bromide; oxitropium bromide; pirenzepine; and telenzepine.

The present invention still further relates to the combination of a compound of the invention together with a β₁- to β₄-adrenoceptor agonists including metaproterenol, isoproterenol, isoprenaline, albuterol, salbutamol, formoterol, salmeterol, terbutaline, orciprenaline, bitolterol mesylate, and pirbuterol; or methylxanthanines including theophylline and aminophylline; sodium cromoglycate; or muscarinic receptor (M1, M2, and M3) antagonist.

The present invention still further relates to the combination of a compound of the invention together with an insulin-like growth factor type I (IGF-1) mimetic.

The present invention still further relates to the combination of a compound of the invention together with an inhaled glucocorticoid with reduced systemic side effects, including prednisone, prednisolone, flunisolide, triamcinolone acetonide, beclomethasone dipropionate, budesonide, fluticasone propionate, and mometasone furoate.

The present invention still further relates to the combination of a compound of the invention together with (a) tryptase inhibitors; (b) platelet activating factor (PAF) antagonists; (c) interleukin converting enzyme (ICE) inhibitors; (d) IMPDH inhibitors; (e) adhesion molecule inhibitors including VLA-4 antagonists; (f) cathepsins; (g) MAP kinase inhibitors; (h) glucose-6 phosphate dehydrogenase inhibitors; (i) kinin-B₁- and B₂-receptor antagonists; (j) anti-gout agents, e.g., colchicine; (k) xanthine oxidase inhibitors, e.g., allopurinol; (1) uricosuric agents, *e.g.,* probenecid, sulfinpyrazone, and benzbromarone; (m) growth hormone secretagogues; (n) transforming growth factor (TGFβ); (o) platelet-derived growth factor (PDGF); (p) fibroblast growth factor, *e.g.,* basic fibroblast growth factor (bFGF); (q) granulocyte macrophage colony stimulating factor (GM-CSF); (r) capsaicin cream; (s) Tachykinin NK₁ and NK₃ receptor antagonists selected from the group consisting of: NKP-608C; SB-233412 (talnetant); and D-4418; and (t) elastase inhibitors selected from the group consisting of: UT-77 and ZD-0892.

The present invention still further relates to the combination of a compound of the invention together with an inhibitor of matrix metalloproteases (MMPs), *i.e.,* the stromelysins, the collagenases, and the gelatinases, as well as aggrecanase; especially collagenase-1 (MMP-1), collagenase-2 (MMP-8), collagenase-3 (MMP-13), stromelysin-1 (MMP-3), stromelysin-2 (MMP-10), and stromelysin-3 (MMP-11).

The compounds of the invention can also be used in combination with existing therapeutic agents for the treatment of osteoarthritis. Suitable agents to be used in combination include standard non-steroidal anti-inflammatory agents (hereinafter NSAID's) such as piroxicam, diclofenac, propionic acids such as naproxen, flubiprofen, fenoprofen, ketoprofen and ibuprofen, fenamates such as mefenamic acid, indomethacin, sulindac, apazone, pyrazolones such as phenylbutazone, salicylates such as aspirin, COX-2 inhibitors such as celecoxib, valdecoxib, rofecoxib and etoricoxib, analgesics and intraarticular therapies such as corticosteroids and hyaluronic acids such as hyalgan and synvisc.

The compounds of the present invention may also be used in combination with anticancer agents such as endostatin and angiostatin or cytotoxic drugs such as adriamycin, daunomycin, cis-platinum, etoposide, taxol, taxotere and farnesyl transferase inhibitors, VEGF inhibitors, COX-2 inhibitors and antimetabolites such as methotrexate antineoplastic agents, especially antimitotic drugs including the vinca alkaloids such as vinblastine and vincristine.

The compounds of the invention may also be used in combination with antiviral agents such as Viracept, AZT, aciclovir and famciclovir, and antisepsis compounds such as Valant.

The compounds of the present invention may also be used in combination with cardiovascular agents such as calcium channel blockers, lipid lowering agents such as statins, fibrates, beta-blockers, Ace inhibitors, Angiotensin-2 receptor antagonists and platelet aggregation inhibitors.

The compounds of the present invention may also be used in combination with CNS agents such as antidepressants (such as sertraline), anti-Parkinsonian drugs (such as deprenyl, L-dopa, Requip, Mirapex, MAOB inhibitors such as selegine and rasagiline, comP inhibitors such as Tasmar, A-2 inhibitors, dopamine reuptake inhibitors, NMDA antagonists, Nicotine agonists, Dopamine agonists and inhibitors of neuronal nitric oxide synthase), and anti-Alzheimer's drugs such as donepezil, tacrine, COX-2 inhibitors, propentofylline or metryfonate.

The compounds of the present invention may also be used in combination with osteoporosis agents such as roloxifene, droloxifene, lasofoxifene or fosomax and immunosuppressant agents such as FK-506, rapamycin, cyclosporine, azathioprine, and methotrexate.

### DETAILED DESCRIPTION OF THE INVENTION

Compounds of the formula I may be prepared according to the following reaction schemes and discussion. Unless otherwise indicated R¹ through R⁹ in the reaction schemes and discussion that follows are as defined above.

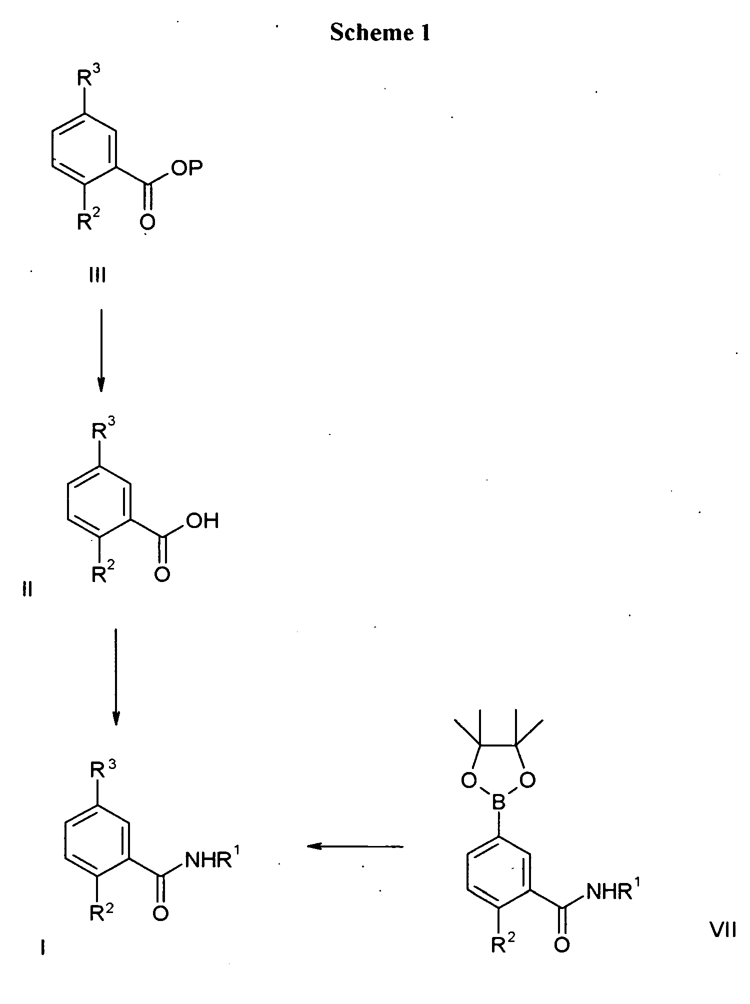

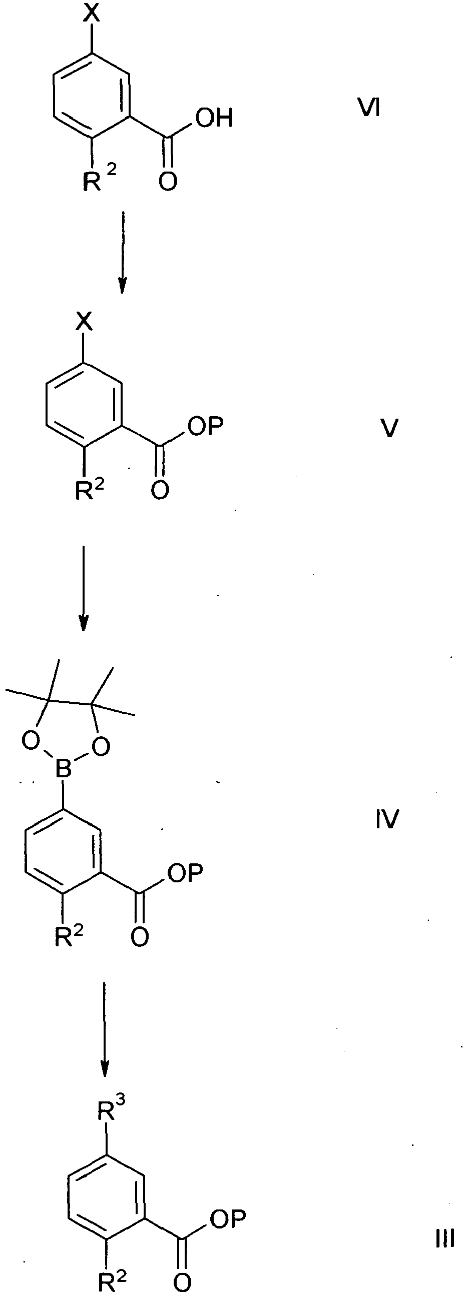

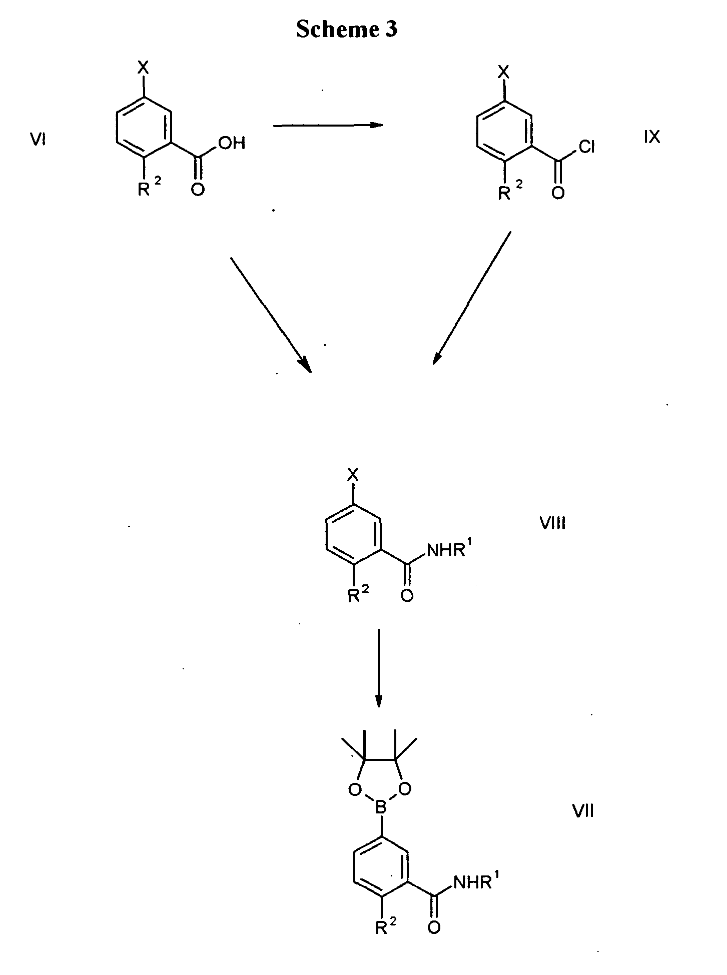

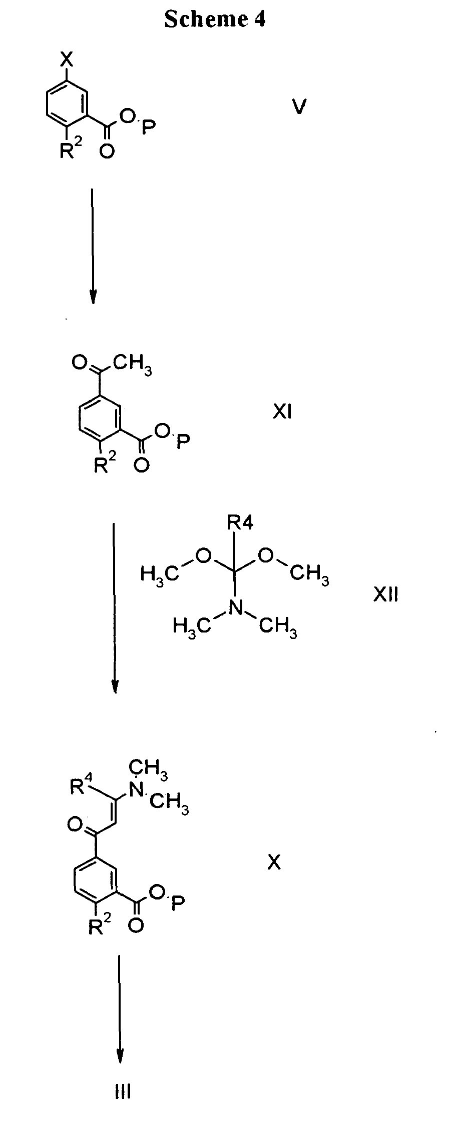

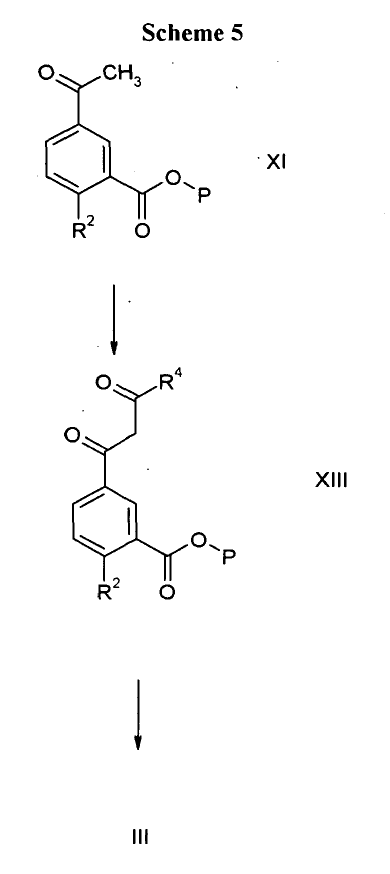

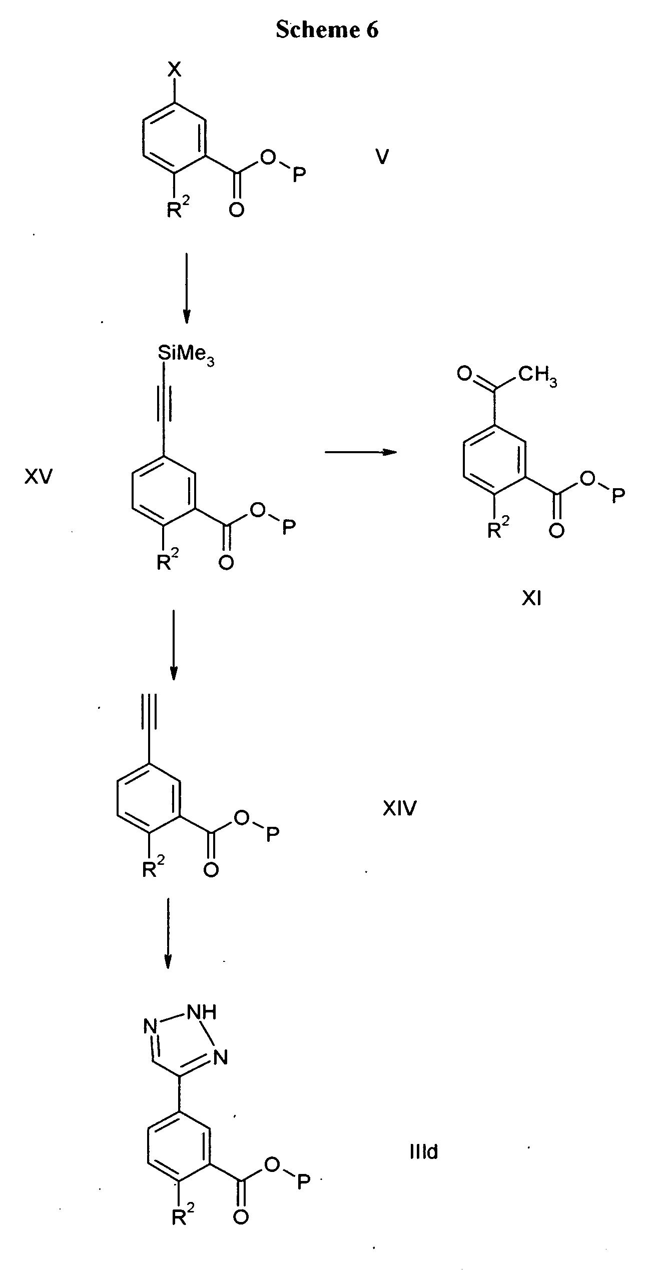

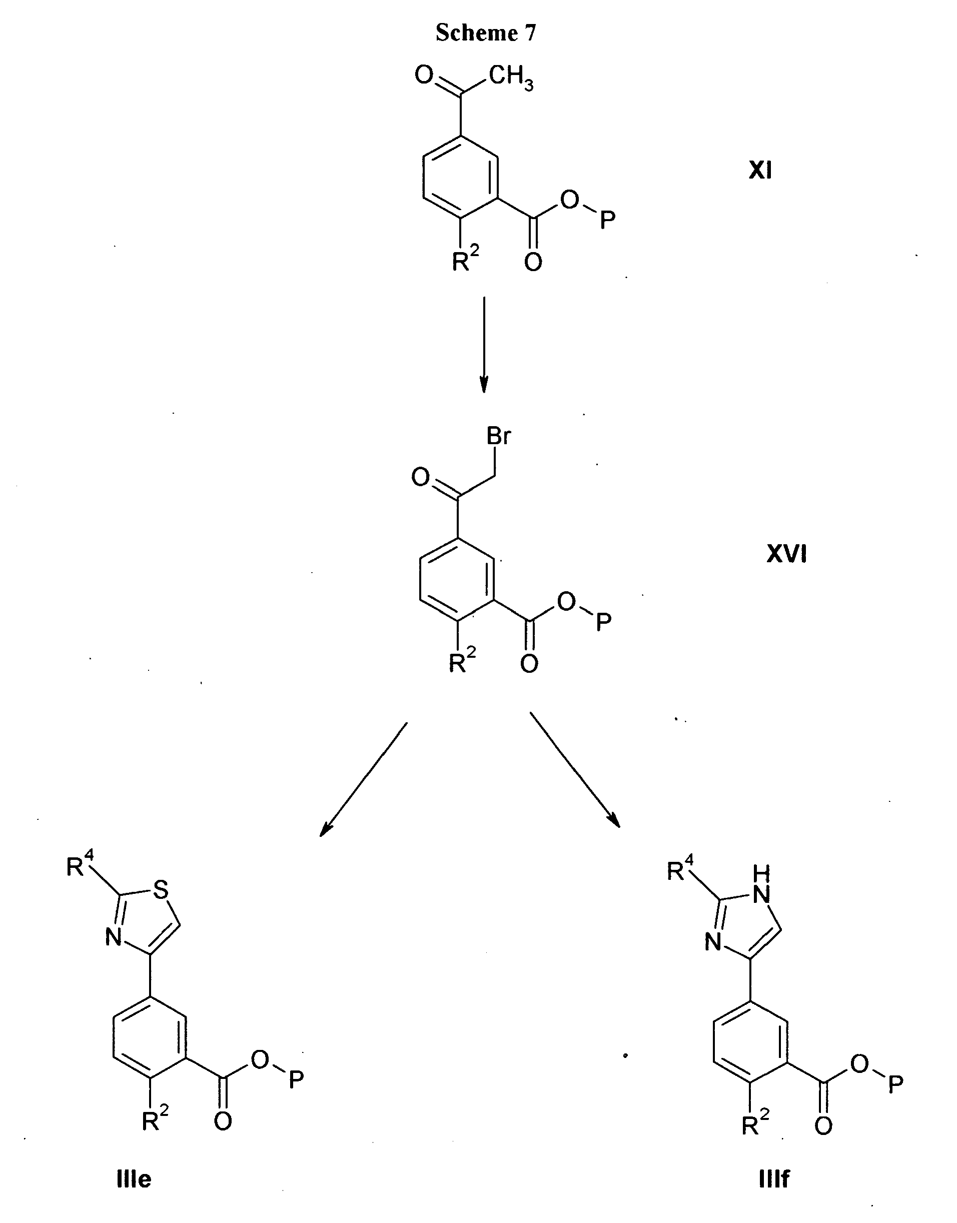

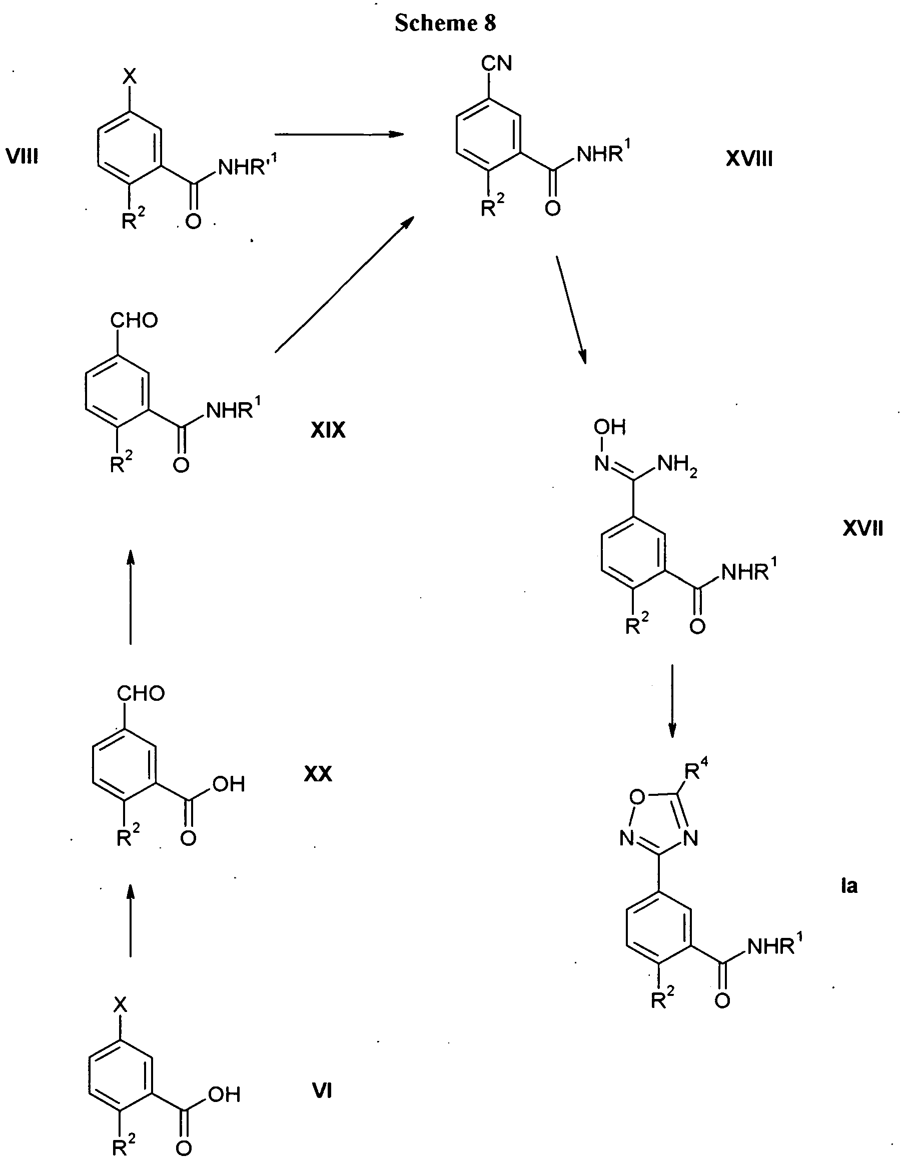

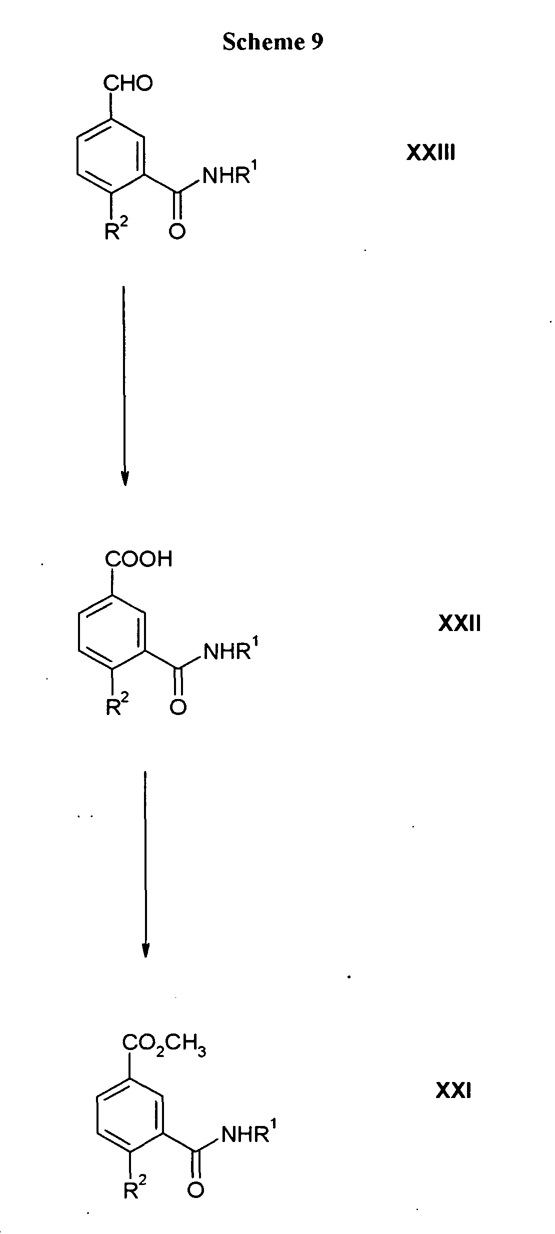

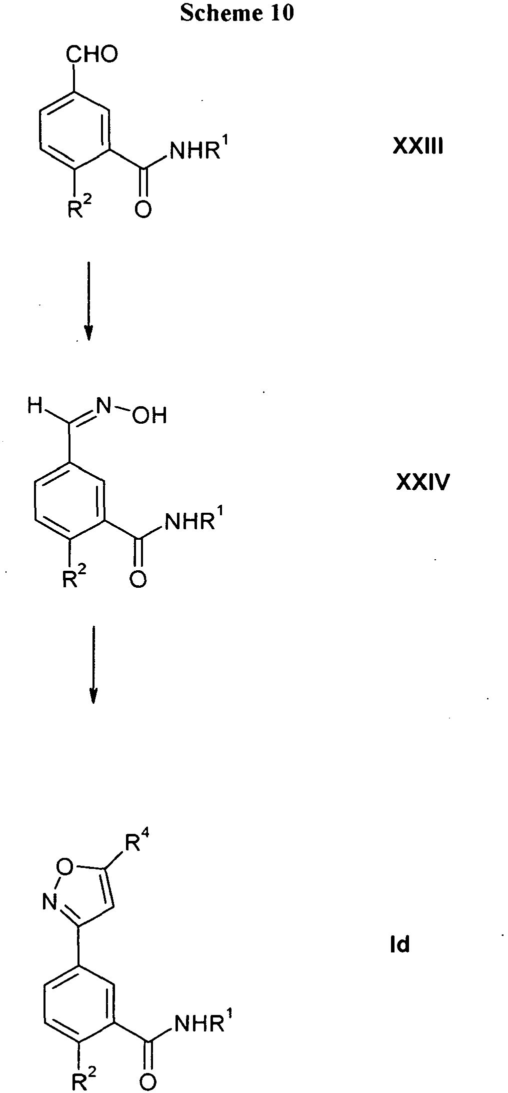

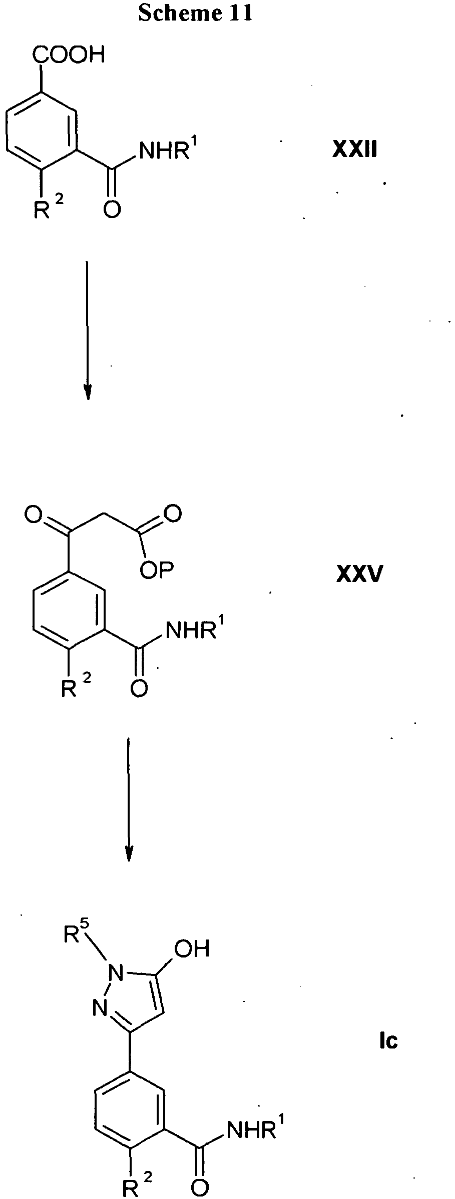

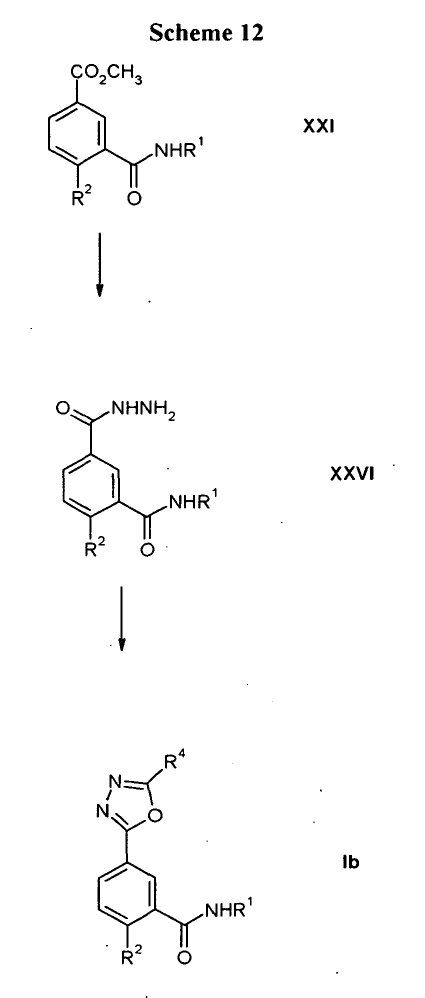

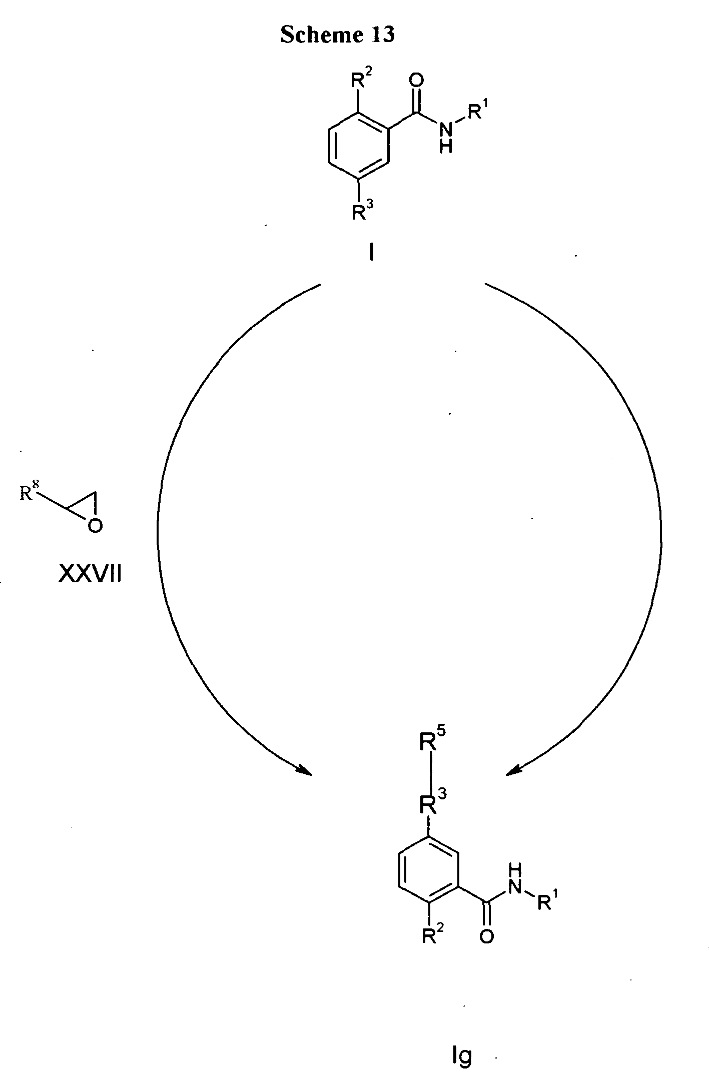

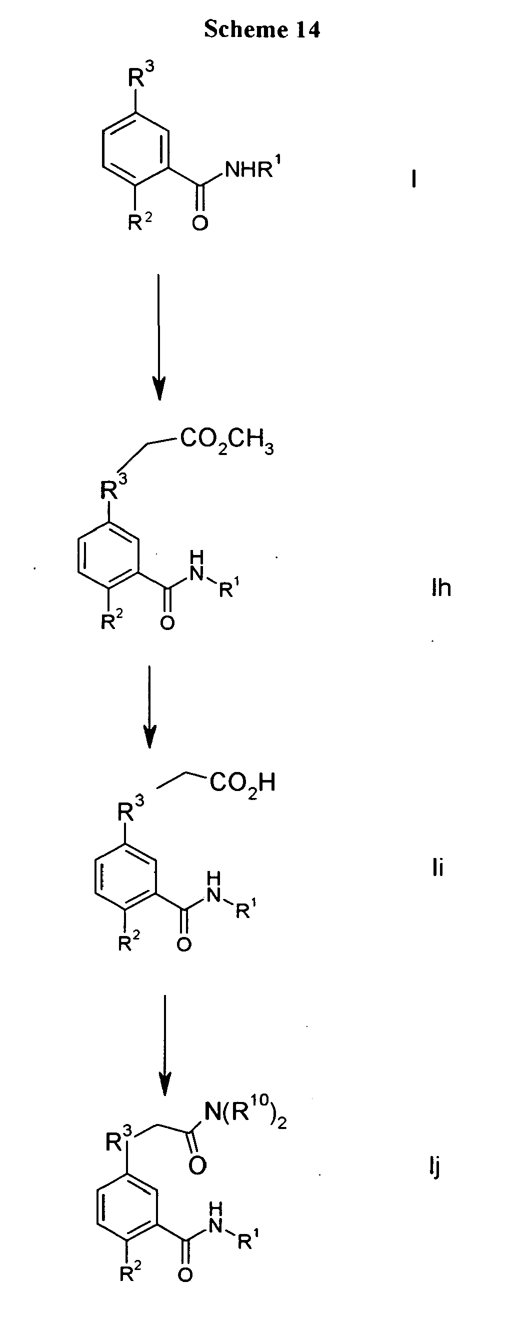

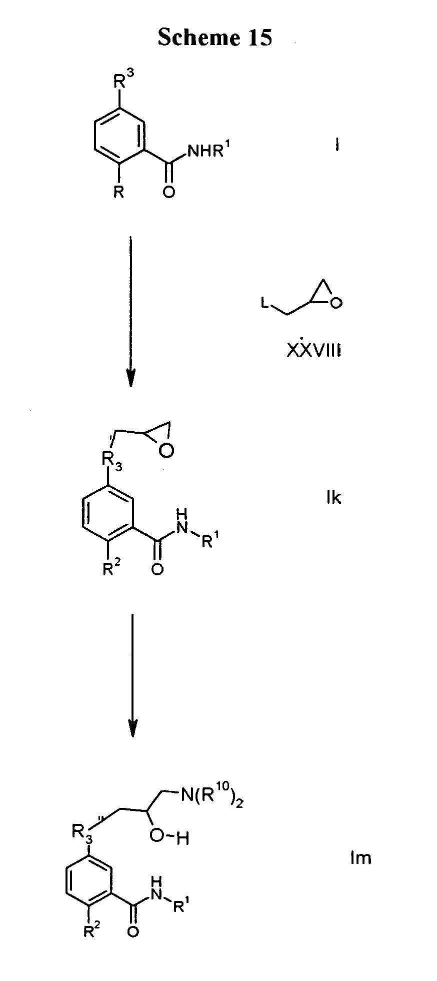

Scheme 1 refers to the preparation of compounds of the formula **I**. Compounds of the formula **I** may be prepared from compounds of formula **II** by reaction with an amine of the formula H₂N-R¹, in the presence of a coupling reagent such as 1-[3-(dimethylamino)propyl]-3-ethylcarbodiimide (EDCI), dicyclohexylcarbodiimide (DCC), 1,1'-carbonyldiimidazole (CDI) and a base such as dimethylaminopyridine (DMAP) or triethylamine in an aprotic solvent, such as methylene chloride, dimethylformamide, or dimethylsulfoxide, preferably 1-[3-(dimethylamino)propyl]-3-ethylcarbodiimide and dimethylaminopyridine in dimethyl formamide. The aforesaid reaction may be run at a temperature from about 22 °C to about 60 °C, for a period of about 1 hour to about 20 hours, preferably about 22 °C for about 18 hours.

Compounds of the formula **II** may be prepared by reacting a compound of the formula **III**, wherein P is a protecting group, with a base, such as sodium carbonate, potassium carbonate, cesium carbonate, sodium hydroxide, potassium hydroxide or lithium hydroxide, in a solvent such as methanol, ethanol, 2-propanol, tetrahydrofuran, dioxane with added water. Preferred conditions include potassium hydroxide and methanol. The aforesaid reaction may be run at a temperature from about 22 °C to about 60 °C, for a period of about 1 hour to about 20 hours, preferably about 22 °C for about 18 hours.

Alternatively, a compound of the formula **I** may also be prepared from a compound of the formula **VII** by reaction with a compound of formula R³-X, wherein X is a suitable leaving group, in the presence of a palladium catalyst, and a base in an aprotic solvent. Suitable leaving groups include chloro, bromo, iodo, triflate, tosylate or mesylate. Suitable palladium catalysts include 1,1'-bis(diphenylphosphino)ferrocenepalladium(II) dichloride dichloromethane, or tetrakis triphenylphosphine palladium (0). Suitable bases include potassium carbonate, cesium carbonate, triethylamine, aqueous sodium hydroxide or aqueous potassium hydroxide. Suitable solvents include methylene dimethylformamide. Preferred conditions are 1,1'-bis(diphenylphosphino)ferrocenepalladium(II) dichloride dichloromethane, aqueous sodium carbonate and dimethylfomamide. The aforesaid reaction may be run at a temperature from about 22 °C to about 100 °C, for a period of about 1 hour to about 24 hours, preferably at about 80°C for about 12 hours.

Compounds of formula III can be made according to the methods of Scheme 2 and 4-7. Compounds of formula VII can be made according to the methods of Scheme 3.

Scheme **2** refers to preparation of compounds of formula **III,** which are intermediates in Scheme 1 for the preparation of compounds of formula I. Referring to Scheme 2, compounds of formula **III** may be prepared by reacting a compound of the formula **IV** with a compound of formula R³-X, wherein X is a suitable leaving group, in the presence of a palladium catalyst and a base in an aprotic solvent. Suitable leaving groups include chloro, bromo or iodo, preferably iodo. Suitable palladium catalysts include 1,1'-bis(diphenylphosphino)ferrocenepalladium(II) dichloride dichloromethane, or tetrakis triphenylphosphine palladium (0). Suitable bases include potassium acetate, potassium carbonate, cesium carbonate, triethylamine, aqueous sodium hydroxide or aqueous potassium hydroxide. Suitable solvents include methylene chloride and dimethylformamide. Preferred conditions include 1,1'-bis(diphenylphosphino)ferrocenepalladium(II) dichloride dichloromethane, aqueous sodium carbonate and dimethylfomamide. The aforesaid reaction may be run at a temperature from about 22°C to about 60°C, for a period of about 1 hour to about 20 hours; preferably about 80°C for about 18 hours.

Compounds of formula **IV** can be prepared by reacting a compound of the formula V, wherein X is a suitable leaving group, with bis(pinacolato)diboron in the presence of a palladium catalyst and a base in an aprotic solvent. Suitable leaving groups include bromo, iodo, chloro and triflate. Suitable palladium catalysts include 1,1'-bis(diphenylphosphino)ferrocenepalladium(II) dichloride dichloromethane or tetrakis triphenylphosphine Palladium (0). Suitable bases include potassium acetate, potassium carbonate, cesium carbonate, triethylamine, aqueous sodium hydroxide or aqueous potassium hydroxide. Suitable solvents include methylene chloride and dimethylformamide. Preferred conditions include 1,1'-bis(diphenylphosphino)ferrocenepalladium(II) dichloride dichloromethane, potassium acetate and dimethylfomamide.

A compound of the formula **V** can be prepared from a compound of the formula **VI** by reaction with methanol in the presence of an acid such as sulfuric acid or gaseous hydrochloric acid at a temperature between 22°C and reflux for a period of 4 to 24 hours; preferably gaseous hydrochloric acid at 22°C for 24 hours.

Compounds of formula VI are commercially available or can be made according to the methods well known to those skilled in the art.

Scheme 3 refers to the preparation of compounds of the formula **VII,** which are intermediates for the preparation of compounds of formula I in Scheme 1. Referring to Scheme 3, a compound of formula **VII** may be prepared by reacting a compound of the formula **VIII,** wherein X is a leaving group, with bis(pinacolato)diboron in the presence of a palladium catalyst and a base in an aprotic solvent. Suitable leaving groups include chloro, bromo, iodo or triflate, preferably bromo or iodo. Suitable palladium catalysts include 1,1'-bis(diphenylphosphino)ferrocenepalladium(II) dichloride dichloromethane or tetrakis triphenylphosphine Palladium (0). Suitable bases include potassium acetate, potassium carbonate, cesium carbonate, triethylamine, aqueous sodium hydroxide or aqueous potassium hydroxide. Suitable solvents include methylene chloride and dimethylformamide. Preferred conditions include 1,1'-bis(diphenylphosphino)ferrocenepalladium(II) dichloride dichloromethane, potassium acetate and dimethylfomamide.

Alternatively, compounds of the formula VIII may be converted directly to compounds of formula I by reaction with a borate of formula R³-B(OH)₂ in the presence of a palladium catalyst and a base in an aprotic solvent Suitable catalysts include 1,1'-bis(diphenylphosphino)-ferrocenepalladium(II) dichloride dichloromethane, or tetrakistriphenylphosphine palladium (0); preferably tetrakistriphenylphosphine palladium (0). Suitable bases include potassium acetate, potassium carbonate, cesium carbonate, triethylamine, aqueous sodium hydroxide, aqueous sodium carbonate, aqueous potassium carbonate or aqueous potassium hydroxide. Suitable solvents include methylene chloride, ethyl acetate, toluene, dichloroethane, dimethylformamide, or dimethylsulfoxide. Preferred conditions include aqueous sodium carbonate in dimethyl formamide. The aforesaid reaction may be run at a temperature from 22°C to 100°C, for a period of 1 hour to 20 hours, preferably 80°C for 18 hours. Borates of the formula R³-B(OH)₂ can be prepared by reacting a compound of formula R³-X, wherein X is a leaving group as described above, with a base, and a trialkyl borate of formula B(OR)₃ in an aprotic solvent. Suitable bases include n-butyl lithium or isopropylmagnesium chloride; preferably isopropylmagnesium chloride. Suiable solvents include diethyl ether, tetrahydrofuran, dimethoxy ethane; preferably tetahydrofuran. The aforementioned reaction may be run at a temperature from -78°C to 22°C for a period of 1 hour to 24 hours; preferably 22°C for 18 hours. The resulting product is hydrolyzed using aqueous acid, such as hydrochloric acid, sulfuric acid, citric acid or perchloric acid; preferably hydrochloric acid. The hydrolysis reaction can be run at temperature from 22°C to 100°C for a period of 1 hour to 24 hours; preferably 22°C for 4 hours.

Compounds of the formula **VIII** may be prepared from compounds of formula **VI** by reaction with a compound of formula R¹NH₂, in the presence of a coupling reagent such as 1-[3-(dimethylamino)propyl]-3-ethylcarbodiimide (EDCI), dicyclohexylcarbodiimide (DCC), 1,1'-carbonyldiimidazole (CDI) and a base such as dimethylaminopyridine (DMAP) or triethylamine in an aprotic solvent, such as methylene chloride, dimethylformamide, or dimethylsulfoxide; preferably 1-[3-(dimethylamino)propyl]-3-ethylcarbodiimide and dimethylaminopyridine in dimethylformamide. The aforesaid reaction may be run at a temperature from 22 °C to 60 °C, for a period of 1 hour to 20 hours, preferably 22 °C for 18 hours.

Compounds of the formula **VIII** may also be prepared from compounds of the formula **IX** by reaction with a compound of formula R¹NH₂, in the presence of a base in an aprotic solvent. Suitable bases include dimethylaminopyridine (DMAP), triethylamine, diisopropylethylamine, aqueous sodium hydroxide or aqueous potassium hydroxide. Suitable solvents include methylene chloride, ethyl acetate, dichloroethane, dimethylformamide, or dimethylsulfoxide, preferably diisopropylethylamine and dichloroethane. The aforesaid reaction may be run at a temperature from 22 °C to 60 °C, for a period of 1 hour to 24 hours, preferably at 22°C for 3 hours.

Compounds of the formula **IX** may be prepared from compounds of the formula **VI** by reaction with a reagent capable of generating an acid chloride, such as thionyl chloride or oxalyl chloride, in the presence or absence of a polar aprotic solvent, such as ethyl acetate, methylene chloride, or dichloroethane, at a temperature of 22 °C to 80 °C, for a period of 1 hour to 24 hours. Preferred conditions include thionyl chloride at 80 °C for 4 hours.

Compounds of formula **VI** can be prepared by methods well known to those skilled in the art.

Scheme **4** refers to an alternative preparation of compounds of the formula **III,** which are intermediates useful for the preparation of compounds of formula I in Schemes 1 and 2. Referring to Scheme 4, a compound of formula **III** may be prepared by reacting a compound of formula **X** with a cyclization reagent in a protic solvent, such as methanol, ethanol or iso-propanol. The aforesaid reaction may be run at a temperature from about 22°C to about 100°C for a period of about 1 to about 24 hours; preferably in ethanol at about 22°C for about 16 hours.

When the cyclization reagent is a hydrazine, the compound of formula III has the formula

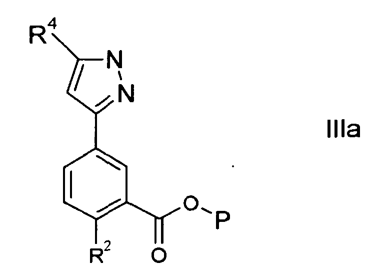

When the cyclization reagent is a hydroxylamine, the compound of formula III has the formula

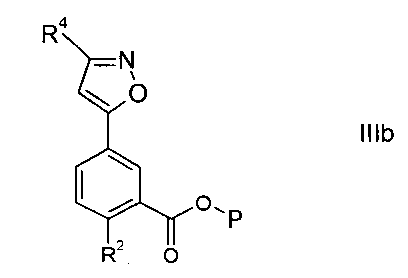

When the cyclization reagent is an amidine, the compound of formula III has the formula

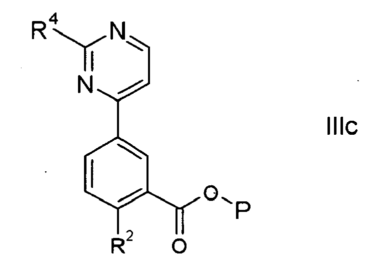

A compound of formula **X** may be prepared by reacting a compound of the formula **XI** with a reactant of the formula **XII,** either in the presence or absence of a solvent, such as ethanol, methanol or tetrahydrofuran, at a temperature from 22°C to 100°C; preferably in the absence of solvent at 90°C for about 4 hours.

Compounds of the formula **XI** may be prepared from compounds of the formula **V,** wherein X is a suitable leaving group, by reaction with acetic anhydride, a palladium catalyst, lithium chloride and a base in an aprotic solvent. Suitable leaving groups include bromo, iodo, chloro or triflate; preferably iodo. Suitable palladium caalysts include 1,1'-bis(diphenylphosphino)-ferrocenepalladium(II) dichloride dichloromethane, tetrakis triphenylphosphine palladium (0), or tris(dibenzylideneacetone)dipalladium(0); preferably, tris(dibenzylideneacetone)dipalladium(0). Suitable bases include dimethylaminopyridine (DMAP), triethylamine and diisopropylethylamine. Suitable solvents include methylene chloride, ethyl acetate, dichloroethane, dimethylformamide, or dimethylsulfoxide, preferably diisopropylethylamine and dimethylformamide. The aforesaid reaction may be run at a temperature from about 22 °C to about 100 °C, for a period of about 1 hour to about 24 hours, preferably at about 100°C for about 12 hours.

Compounds of formula V may be prepared according to the methods of Scheme 2. Compounds of the formula XII are commercially available or can be made by methods well known to those skilled in the art.

Scheme 5 refers to an alternate preparation of compounds of the formula **III (i.e. IIIa, IIIb, and IIIc),** which are intermediates useful for the preparation of compounds of formula I in Schemes 1 and 2. Referring to Scheme 5, a compound of formula **III (i.e. IIIa, IIIb, and IIIc)** may be prepared by reacting a compound of formula XIII with a cyclization reagent in a protic solvent, such as methanol, ethanol or iso-propanol. Suitable cyslization reagents are as described above in Scheme 4 for the conversion of compounds of formula X into compounds of the formula **IIIa, IIIb, and IIIc,** respectively. The aforesaid reaction may be run at a temperature from about 22°C to about 100°C for a period of 1 hour to about 24 hours; preferably in ethanol at about 22°C for about 16 hours.

Compounds of formula **XIII** may be prepared by reacting a compound of formula **XI** with a compound of formula R⁴-(C=O)-X, wherein X is a leaving group, with a base in an aprotic solvent Suitable leaving groups include chloro, bromo or alkoxy, preferably chloro. Suitable bases include potassium tertiary butoxide, dimethylaminopyridine (DMAP), triethylamine and diisopropylethylamine. Suitable solvents include ethylether, tetrahydrofuran, toluene or dimethylformamide; preferably toluene. The aforementioned reaction may be run at temperature 0°C to 80°C for about 1 to about 24 hours, preferably about 0°C to about 22°C for about 2 hours.

Compounds of formula XI can be prepared according to the methods of Scheme 4.

Scheme 6 refers to an alternative preparation of compounds of formula **IIId,** which are compounds of formula III, wherein R³ is triazolyl. Compounds of formula **IIId** are useful intermediates in the preparation of compounds of formula **I** in Schemes 1 and 2. Referring to Scheme 6, a compound of formula **IIId** can be prepared by reacting a compound of formula **XIV** with trimethylsilyl azide in a protic solvent such as methanol, ethanol or n-butyl alcohol; preferably n-butylalcohol. The aforesaid reaction may be run at a temperature from about 22°C to about 150°C, for a period of about 1 hour to about 144 hours, preferably about 125°C for about 120 hours.

A compound of formula **XIV** can be prepared by reacting a compound of the formula **XV** with 96%-98% formic acid. The aforementioned reaction may be run at temperature from about 22°C to about 120°C for a period of about 1 hour to about 120 hours; preferably with 98% formic acid at about 22°C for about 12 hours.

A compound of formula **XV** may be prepared by reacting a compound of formula **V,** wherein X is a suitable leaving group, with an acetylene of the formula (CH₃)₃Si-C≡C-H, and a base in the presence or absence of an aprotic solvent Suitable leaving groups include bromo, iodo, chloro or triflate, preferably iodo. Suitable bases include triethylamine and diisopropylethylamine. Suitable solvents include methylene chloride, tetrahydrofuran, dimthylformamide or dioxane, preferably in the absence of a solvent. The aforesaid reaction may be run at a temperature from about 22°C to about 100°C, for a period of 1 hour to 20 hours, preferably at 100 °C for 18 hours.

Compounds of the formula **XV** may also be used in an alternate preparation to form compounds of formula **XI,** which are intermediates for the preparation of compounds of formula III in Scheme 4. Compounds of formula **XV** may be reacted with 96%-98% formic acid to form a compound of formula **XI.** The aforementioned reaction may be run at temperature from about 22°C to about 120°C for a period of about 1 hour to about 120 hours, preferably with 98% formic acid at about 100°C for about 120 hours.

Compounds of the formula **V** may be prepared according to the methods of Scheme 2.

Scheme 7 refers to an alternate preparation of compounds of formulas **IIIe** and **IIIf.** Compounds of the formula **If** may be prepared from compounds of formula **XVI** by reaction with a compound of the formula R⁴-(C=NH)-NH₂ in an aprotic solvent such as dimethylsulfoxide, tetrahydrofuran, dimethlyformamide or toluene, at a temperature ranging from about 0°C to about 100°C for a period of about 15 minutes to about 18 hours. The preferred conditions are tetrahydrofuran at about 60°C for about 1 hour.

Compounds of the formula **IIIe** may be prepared from compounds of the formula **XVI** by reaction with a compound of the formula R⁴-(C=S)-NH₂ in a protic or aprotic solvent, such as dimethylsulfoxide, ethanol, tetrahydrofuran, dimethlyformamide or toluene, at a temperature ranging from about 0°C to about 100°C for a period of about 15 minutes to about 18 hours. The preferred conditions are ethanol at about 50°C for about 0.5 hour.

Compounds of the formula **XVI** may be prepared from compounds of the formula **XI** by reaction with bromine under various conditions known to those skilled in the art (H. O. House, "Modern Synthetic Reactions," W. A. Benjamin, Inc., Menlo Park, California (1972), pp 459-478).

Compounds of formula **XI** may be made by the methods of Schemes 4 and 6.

Scheme **8** refers to an alternate preparation of compounds of formula **Ia.** Compounds of the formula **Ia** may be prepared from compounds of formula **XVII** by reaction with a compound of the formula R⁴(C=O)Cl or (R⁴-(C=O))₂O in the presence or absence of an aprotic solvent such as dimethylsulfoxide, tetrahydrofuran, dimethlyformamide or methylene chloride, at a temperature ranging from about 20°C to about 150°C for a period of about 1 hour to about 18 hours. The preferred conditions are neat at about 120°C for about 2 hours.

Compounds of the formula **XVII** can be prepared from compounds of the formula **XVIII** by reaction with hydroxylamine in a protic solvent, such as methanol or ethanol, at a temperature ranging from about 20°C to about 100°C for a period of about 1 hour to about 24 hours. The preferred conditions are methanol at about 60°C for about 18 hours.

Compounds of formula **XVIII** can be prepared from compounds of the formula **VIII** by reaction with a cyanide reagent in the presence or absence of a palladium catalyst in an aprotic solvent at a temperature ranging from about 50°C to about 200°C for a period of about 1 hour to about 48 hours (Tetrahedron Letters 40 (1999) 8193-8195; 41 (2000) 3271-3273; 42 (2001) 6707-6710; 43 (2002) 387-389). Suitable cyanide reagents include zinc cyanide, copper cyanide, sodium cyanide and potassium cyanide. Suitable palladium catalysts include 1,1'-bis(diphenylphosphino)ferrocenepalladium(II) dichloride, dichloromethane, or tetrakis triphenylphosphine Palladium (0). Suitable solvents include dimethylformamide, dimethylacetamide, N-methylpyrrolidinone and toluene.

Compounds of the formula **XVIII** may also be prepared from compounds of the formula **XIX** by reaction with hydroxylamine hydrochloride and derivatives thereof under the conditions known to those skilled in the art (March, J. Advanced Organic Chemistry; John Wiley & Sons: New York, 1985; pp. 806-807).

Compounds of the formula **XIX** can be prepared from compounds of the formula **XX** by reaction with a reagent of the formula NH₂-R¹, in the presence of a coupling reagent and a base, such as dimethylaminopyridine (DMAP) or triethylamine, in an aprotic solvent Suitable coupling agents include 1-[3-(dimethylamino)propyl]-3-ethylcarbodiimide (EDCI), dicyclohexylcarbodiimide (DCC), 1,1'-carbonyldiimidazole (CDI), polymer bound carbodiimide and 1-hydroxy benzotriazole. Suitable solvents include methylene chloride, dimethylformamide, or dimethylsulfoxide. Preferred conditions include polymer bound carbodiimide and 1-hydroxy benzotriazole in dimethyl formamide. The aforesaid reaction may be run at a temperature from about 22 °C to about 60 °C, for a period of about 1 hour to about 20 hours, preferably about 22 °C for about 18 hours.

Compounds of the formula **XX** can be prepared from compounds of the formula **VI** by reaction with a base, such as methyl lithium, n-butyllithium or *tert-*butyllithium, in an aprotic solvent, such as ethyl ether, tetrahyrofuran or hexane, at a temperature ranging from about -80°C to about 25°C for a period of about 10 minutes to about 1 hour, followed by reaction with dimethylformamide in the same pot for a period of about 0.5 hours to about 3 hours. The preferred conditions are methyl lithium (1 equivalent) followed by *tert*-butyllithium (1 equivalent) in tetrahydrofuran at about -78°C for about 10 minutes, followed by reaction with dimethylformamide for about 30 minutes.

Compounds of the formula **VI** are commercially known or may be prepared according to the methods well known to those skilled in the art.

Scheme 9 refers to the preparation of compounds of the formulae **XXI** and **XXII,** which are intermediates in the preparation of compounds of formula I in Schemes 10-12. Referring to Scheme 9, a compound of the formula **XXI** can be prepared from a compound of the formula **XXII** by various methods known to those skilled in the art [R.C. Larock, "Comprehensive Organic Transformations. A Guide to Functional Group Preparations," VCH Publishers, Inc., New York, New York (1989), pp. 966-972].

Compounds of the formula **XXII** can be prepared from compounds of the formula **XXIII** also by various methods known to those skilled in the art (R.C. Larock, "Comprehensive Organic Transformations. A Guide to Functional Group Preparations," VCH Publishers, Inc., New York, New York (1989), pp. 838-841).

Compounds of formula **XXIII** are commercially available or can be made by methods well known to those skilled in the art.

Scheme 10 refers to an alternative preparation of compounds of the formula Id. Compounds of the formula **Id** can be prepared from compounds of the formula **XXIV** by reaction with a reagent of the formula HC≡C-R⁴, sodium hypochlorite and a base, such as triethylamine or diethylisopropyl amine, in a solvent, such as dichloromethane or dichloroethane, at a temperature ranging from 0°C to 60°C for a period of 3 hours to 72 hours. The preferred conditions are triethylamine, dichloromethane, 22°C for 20 hours.

Compounds of the formula **XXIV** may be prepared from compounds of the formula **XXIII** by reaction with hydroxylamine or hydroxylamine hydrochloride under the conditions known to those skilled in the art (March, J. Advanced Organic Chemistry; John Wiley & Sons: New York, 1985; pp. 805-806).

Scheme 11 refers to an alternative preparation of compounds of the formula **Ic.** Compounds of the formula **Ic** can be prepared from compounds of the formula **XXV** by reaction with a reagent of the formula NE₂NHR⁵ in the presence or absence of a solvent such as methanol, ethanol, tetrahydrofuran or dimethylformamide, at a temperature ranging from 22°C to 150°C for a period of 1 hour to 24 hours. The preferred conditions are ethanol, 80°C for 12 hours.

Compounds of the formula **XXV** can be prepared from compounds of the formula **XXII** by reaction with carbonyldiimidazole in a solvent including, but not limited to tetrahydrofuran, ether and dichloromethane at a temperature ranging from 0°C to 60°C for a period of 1 hour to 24 hours, followed by reaction with the magnesium salt of monomethyl malonate at a temperature ranging from 0°C to 60°C for a period of 1 hour to 24 hours. The preferred conditions are carbonyldiimidazole in tetrahydrofuran at 22°C for 6 hours, followed by reaction with the magnesium salt of monomethyl malonate at 22°C for 12 hours (D. W. Brooks, L. D. Lu and S. Masamune Angew. Chem. Int. Ed. Eng. 18 (1979) p. 72).

Scheme 12 refers to an alternate preparation of compounds of formula **Ib.** Compounds of the formula **Ib** may be prepared from compounds of the formula **XXVI** by reaction with a reagent of the formula R⁴C(-OR)₃ in the presence or absence of a solvent, such as dimethylacetamide, dimethylformamide or xylene, at a temperature ranging from 50°C to 200°C for a period of 1 hour to 12 hours. The preferred conditions are no solvent, 140°C for 2 hours.

Compounds of the formula **XXVI** may be prepared from compounds of the formula **XXI** by reaction with hydrazine or hydrazine hydrate in the presence or absence of a solvent, such as methanol or ethanol, at a temperature ranging from 50°C to 150°C for a period of 1 hour to 24 hours. The preferred conditions are no solvent, 120°C for 2 hours.

Scheme 13 refers to an alternate preparation of compounds of the formula **Ig.** Compounds of the formula **Ig** may be prepared from other compounds of formula **I** (wherein R⁵ is H) by reaction with a compound of the formula L-R⁵, in the presence of a base, wherein L is a suitable leaving group, such as chloro, bromo, iodo, tosylate or mesylate. Suitable bases include triethylamine, polymer supported 2-tert-butylimino-2-diethylamino-1,3-dimethylperhydro-1,3,2-diazaphosphorine (BEMP), cesium carbonate, potassium carbonate, and sodium hydride; cesium carbonate is preferred. The aforesaid reaction can be performed at temperatures ranging from 0 °C to 100 °C in the presence of a polar solvent including, but not limited to dimethylsulfoxide, dimethylformamide, equal amounts of dimethylsulfoxide and acetone, or equal amounts of dimethylformamide and acetone, generally for a period of 2 hours to 72 hours, where the preferred conditions are dimethylsulfoxide at 80°C for 18 hours.

Compounds of the formula **Ig** may also be prepared from other compounds of the formula **I** by reaction of an appropriately substituted epoxide of the formula **XXVII** either neat or in the presence of a polar solvent including but not limited to dimethylformamide, dimethylsulfoxide, and tetrahydrofuran. The aforesaid reaction can be performed at temperatures ranging from 0 °C to 100 °C for a period of 2 to 72 hours, wherein the preferred conditions are dimethylforamide at 60 °C for 16 hours.

Scheme 14 refers to alternative preparations of compounds of the formulae **Ih, Ii** and **Ij.** Compounds of the formula **Ij** may be prepared from compounds of formula **Ii** by reaction with a compound of formula HN(R¹⁰)₂, wherein R¹⁰ is as described above for amide substituents in R⁸, in the presence of a coupling reagent and a base, such as dimethylaminopyridine (DMAP) or triethylamine, in an aprotic solvent. Suitable coupling agents include 1-[3-(dimethylamino)propyl]-3-ethylcarbodiimide (EDCI), dicyclohexylcarbodiimide (DCC), 1,1'-carbonyldiimidazole (CDI), polymer bound carbodiimide and 1-hydroxy benzotriazole. Suitable solvents include methylene chloride, dimethylformamide, or dimethylsulfoxide. Preferred conditions include polymer bound carbodiimide and 1-hydroxy benzotriazole in dimethyl formamide. The aforesaid reaction may be run at a temperature from about 22 °C to about 60 °C, for a period of about 1 hour to about 20 hours, preferably about 22 °C for about 18 hours.

Compounds of the formula **Ii** may be prepared by reacting a compound of the formula **Ih** and a base, such as sodium carbonate, potassium carbonate, cesium carbonate, sodium hydroxide, potassium hydroxide or lithium hydroxide, in a solvent, such as methanol, ethanol, 2-propanol, tetrahydrofuran, dioxane with added water, preferably potassium hydroxide and methanol. The aforesaid reaction may be run at a temperature from 22 °C to 60 °C, for a period of 1 hour to 20 hours, preferably 22 °C for 18 hours.

Compounds of the formula **Ih** may be prepared from other compounds of formula **I** by reaction with a compound of the formula L-(CH₂)-CO₂P in the presence of base, wherein L is a suitable leaving group, such as chloro, bromo, iodo tosylate or mesylate; and P is a suitable protecting group such as alkyl. Suitable bases include triethylamine, polymer supported BEMP, cesium carbonate, potassium carbonate, and sodium hydride; polymer supported BEMP is preferred. The aforesaid reaction can be performed at temperatures ranging from 0 °C to 100 °C in the presence of a polar solvent; such as acetonitrile, varying combinations of acetonitrile and dimethylformamide, dimethylsulfoxide, dimethylformamide, equal amounts of dimethylsulfoxide and acetone, or equal amounts of dimethylformamide and acetone, generally for a period of 2 hours to 72 hours, where the preferred conditions are a 3:2 ratio of acetonitrile and dimethylformamide at 22°C for 4hours.

Scheme 15 refers to the preparation of compounds of the formulae **Ik** and **Im.** Compounds of the formula **Im** can be prepared from compounds of formula **Ik** by reacting with a compound of formula HN(R¹⁰)₂, wherein R¹⁰ is as described above for amine substituents in R⁸, either neat or in the presence of a polar solvent including, but not limited to methyl alcohol, ethyl alcohol, dimethylformamide, dimethylsulfoxide, and tetrahydrofuran. The aforesaid reaction can be performed at temperatures ranging from 0 °C to 100 °C for a period of 2 to 72 hours, where the preferred conditions are methanol at 65 °C for 16 hours.

Compounds of the formula **Ik** can be prepared from other compounds of formula **I** by reaction with a compound of the formula **XXVIII** in the presence of base, wherein L is a suitable leaving group, such as chloro, bromo, iodo tosylate, nosylate or mesylate. Suitable bases include, but are not limited to, triethylamine, polymer supported BEMP, cesium carbonate, potassium carbonate, and sodium hydride; polymer supported BEMP is preferred. The aforesaid reaction can be performed at temperatures ranging from 0 °C to 100 °C in the presence of a polar solvent, such as acetonitrile, varying combinations of acetonitrile and dimethylformamide, dimethylsulfoxide, dimethylformamide, equal amounts of dimethylsulfoxide and acetone, or equal amounts of dimethylformamide and acetone, generally for a period of 2 hours to 72 hours, wherein the preferred conditions are a 3:2 ratio of acetonitrile and dimethylformamide at 80°C for 8 hours.

The activity of the compounds of the invention for the various disorders described above can be determined according to one or more of the following assays. All of the compounds of the invention that were tested had an IC₅₀ of less than 10 µM in *the in vitro* assay described below.

Preferably, the compounds of the invention have an IC₅₀ in the in vitro assays described below of less than 100 nM, more preferably less than 50 nM, and most preferably less than 10 nM. Still further, the compounds of the invention preferably have an IC₅₀ in the range of 0.01 nM -100 nM, more preferably between 0.05 nM - 50 nM, and most preferably between 0.10 nM -10 nM.

### PHARMACOLOGICAL ANALYSIS

Certain compounds such as benzoylbenzoyl adenosine triphosphate (bbATP) are known to be agonists of the P2X₇ receptor, effecting the formation of pores in the plasma membrane (Drug Development Research (1996), 37(3), p. 126). Consequently, when the receptor is activated using bbATP in the presence of ethidium bromide (a fluorescent DNA probe), an increase in the fluorescence of intracellular DNA-bound ethidium bromide is observed. Alternatively, the propidium dye YOPRO-1 can be substituted for ethidium bromide so as to detect uptake of the dye. The increase in fluorescence can be used as a measure of P2X₇ receptor activation and therefore to quantify the effect of a compound on the P2X₇ receptor.

In this manner, the compounds of the invention can be tested for antagonist activity at the P2X₇ receptor. 96-Well flat bottomed microtitre plates are filled with 250 µl of test solution comprising 200 µl of a suspension of THP-1 cells (2.5 x 10⁶ cells/ml, more preferably prestimulated as described in the literature with a combination of LPS and TNF to promote receptor expression) containing 10⁻⁴M ethidium bromide, 25 µl of a high potassium, low sodium buffer solution (10mM Hepes, 150 mM KCI, 5 mM D-glucose and 1.0% FBS at pH 7.5) containing 10⁻⁵M bbATP, and 25 µl of the high potassium buffer solution containing 3 x 10⁻⁵M test compound (more preferably 5 x 10⁻⁴M, more preferably 1 x 10⁻⁴M.more preferably 1 x 10⁻³M). The plate is covered with a plastic sheet and incubated at 37°C for one hour. The plate is then read in a Perkin-Elmer fluorescent plate reader, excitation 520 nm, emission 595 nm, slit widths: Ex 15 nm, Em 20 nm. For the purposes of comparison, bbATP (a P2X₇ receptor agonist) and pyridoxal 5-phosphate (a P2X₇ receptor antagonist) can be used separately in the test as controls. From the readings obtained, a pIC₅₀ figure can be calculated for each test compound, this figure being the negative logarithm of the concentration of test compound necessary to reduce the bbATP agonist activity by 50%.

In like manner, the compounds of the invention can be tested for antagonist activity at the P2X₇ receptor using the cytokine IL-1β as the readout. Blood collected from normal volunteers in the presence of heparin is fractionated using lymphocyte separation medium obtained from Organon Technica (Westchester, PA). The region of the resulting gradient containing banded mononuclear cells is harvested, diluted with 10 ml of Maintenance Medium (RPMI 1640, 5% FBS, 25 mM Hepes, pH 7.2, 1% penicillin/streptomycin), and cells are collected by centrifugation. The resulting cell pellet was suspended in 10 ml of Maintenance Medium and a cell count was performed. In an average experiment, 2 x 10⁵ mononuclear cells are seeded into each well of 96-well plates in a total volume of 0.1 ml. Monocytes are allowed to adhere for 2 hours, after which the supernatants are discarded and the attached cells are rinsed twice and then incubated in Maintenance Medium overnight at 37°C in a 5% CO₂ environment.

The cultured monocytes can be activated with 10 ng/ml LPS (E. coli serotype 055:B5; Sigma Chemicals, St. Louis, MO). Following a 2-hour incubation, the activation medium is removed, the cells are rinsed twice with 0.1 ml of Chase Medium (RPMI 1640, 1% FBS, 20 mM Hepes, 5 mM NaHCO₃, pH 6.9), and then 0.1 ml of Chase Medium containing a test agent is added and the plate is incubated for 30 minutes; each test agent concentration can be evaluated in triplicate wells. ATP then is introduced (from a 100 mM stock solution, pH 7) to achieve a final concentration of 2 mM and the plate is incubated at 37°C for an additional 3 hours. Media were harvested and clarified by centrifugation, and their IL-1β content was determined by ELISA (R&D Systems; Minneapolis, MN).

The compositions of the present invention may be formulated in a conventional manner using one or more pharmaceutically acceptable carriers. Thus, the active compounds of the invention may be formulated for oral, buccal, intranasal, parenteral (e.g., intravenous, intramuscular or subcutaneous), topical or rectal administration or in a form suitable for administration by inhalation or insufflation.

For oral administration, the pharmaceutical compositions may take the form of, for example, tablets or capsules prepared by conventional means with pharmaceutically acceptable excipients such as binding agents (e.g., pregelatinized maize starch, polyvinylpyrrolidone or hydroxypropyl methylcellulose); fillers (e.g., lactose, microcrystalline cellulose or calcium phosphate); lubricants (e.g., magnesium stearate, talc or silica); disintegrants (e.g., potato starch or sodium starch glycolate); or wetting agents (e.g., sodium lauryl sulphate). The tablets may be coated by methods well known in the art. Liquid preparations for oral administration may take the form of, for example, solutions, syrups or suspensions, or they may be presented as a dry product for constitution with water or other suitable vehicle before use. Such liquid preparations may be prepared by conventional means with pharmaceutically acceptable additives such as suspending agents (e.g., sorbitol syrup, methyl cellulose or hydrogenated edible fats); emulsifying agents (e.g., lecithin or acacia); non-aqueous vehicles (e.g., almond oil, oily esters or ethyl alcohol); and preservatives (e.g., methyl or propyl p-hydroxybenzoates or sorbic acid).

For buccal administration, the composition may take the form of tablets or lozenges formulated in conventional manner.

The compounds of formula I can also be formulated for sustained delivery according to methods well known to those of ordinary skill in the art. Examples of such formulations can be found in United States Patents 3,538,214, 4,060,598, 4,173,626, 3,119,742, and 3,492,397 , which are herein incorporated by reference in their entirety.

The active compounds of the invention may be formulated for parenteral administration by injection, including using conventional catheterization techniques or infusion. Formulations for injection may be presented in unit dosage form, e.g., in ampules or in multi-dose containers, with an added preservative. The compositions may take such forms as suspensions, solutions or emulsions in oily or aqueous vehicles, and may contain formulating agents such as suspending, stabilizing and/or dispersing agents. Alternatively, the active ingredient may be in powder form for reconstitution with a suitable vehicle, e.g., sterile pyrogen-free water, before use.

The active compounds of the invention may also be formulated in rectal compositions such as suppositories or retention enemas, e.g., containing conventional suppository bases such as cocoa butter or other glycerides.

For intranasal administration or administration by inhalation, the active compounds of the invention are conveniently delivered in the form of a solution, dry powder formulation or suspension from a pump spray container that is squeezed or pumped by the patient or as an aerosol spray presentation from a pressurized container or a nebulizer, with the use of a suitable propellant, e.g., dichlorodifluoromethane, trichlorofluoromethane, dichlorotetrafluoroethane, heptafluoroalkanes, carbon dioxide or other suitable gas. In the case of a pressurized aerosol, the dosage unit may be determined by providing a valve to deliver a metered amount. The pressurized container or nebulizer may contain a solution or suspension of the active compound. Capsules and cartridges (made, for example, from gelatin) for use in an inhaler or insufflator may be formulated containing a powder mix of a compound of the invention and a suitable powder base such as lactose or starch.

A proposed dose of the active compounds of the invention for oral, parenteral or buccal administration to the average adult human for the treatment of the conditions referred to above (inflammation) is 0.1 to 200 mg of the active ingredient per unit dose which could be administered, for example, 1 to 4 times per day.

The compound of formula (I) and pharmaceutically acceptable salts and solvates thereof may be used on their own but will generally be administered in the form of a pharmaceutical composition in which the formula (I) compound/salt/solvate (active ingredient) is in association with a pharmaceutically acceptable adjuvant, diluent or carrier. Depending on the mode of administration, the pharmaceutical composition will preferably comprise from 0.05 to 99% w (percent by weight), more preferably from 0.10 to 70% w, of active ingredient, and, from 1 to 99.95% w, more preferably from 30 to 99.90% w, of a pharmaceutically acceptable adjuvant, diluent or carrier, all percentages by weight being based on total composition.

Aerosol formulations for treatment of the conditions referred to above in the average adult human are preferably arranged so that each metered dose or "puff' of aerosol contains 20µg to 1000µg of the compound of the invention. The overall daily dose with an aerosol will be within the range 100µg to 10 mg. Administration may be several times daily, for example 2, 3, 4 or 8 times, giving for example, 1, 2 or 3 doses each time.

Aerosol combination formulations for treatment of the conditions referred to above (e.g., adult respiratory distress syndrome) in the average adult human are preferably arranged so that each metered dose or "puff" of aerosol contains from about 1 µg to 1000 µg of the compound of the invention. The overall daily dose with an aerosol will be within the range 100 µg to 10 mg. Administration may be several times daily, for example 2, 3, 4 or 8 times, giving for example, 1, 2 or 3 doses each time.

Aerosol formulations for treatment of the conditions referred to above (e.g., adult respiratory distress syndrome) in the average adult human are preferably arranged so that each metered dose or "puff' of aerosol contains from about 20 µg to 1000 µg of the compound of the invention. The overall daily dose with an aerosol will be within the range 100 µg to 10 mg of the P2X₇ receptor inhibitor. Administration may be several times daily, for example 2, 3, 4 or 8 times, giving for example, 1, 2 or 3 doses each time.

This invention also encompasses pharmaceutical compositions containing and methods of treating or preventing comprising administering prodrugs of compounds of the formula I. Compounds of formula I having free amino, amido, hydroxy or carboxylic groups can be converted into prodrugs. Prodrugs include compounds wherein an amino acid residue, or a polypeptide chain of two or more (e.g., two, three or four) amino acid residues which are covalently joined through peptide bonds to free amino, hydroxy or carboxylic acid groups of compounds of formula I. The amino acid residues include the 20 naturally occurring amino acids commonly designated by three letter symbols and also include, 4-hydroxyproline, hydroxylysine, demosine, isodemosine, 3-methylhistidine, norvalin, beta-alanine, gamma-aminobutyric acid, citrulline homocysteine, homoserine, ornithine and methionine sulfone. Prodrugs also include compounds wherein carbonates, carbamates, amides and alkyl esters which are covalently bonded to the above substituents of formula I through the carbonyl carbon prodrug sidechain.

The following Examples illustrate the preparation of the compounds of the present invention. The present invention is not to be limited in scope by the specific embodiments described herein. Indeed, various modifications of the invention in addition to those described herein will become apparent to those skilled in the art from the foregoing description and the accompanying figures. Such modifications are intended to fall within the scope of the appended claims. All patents, applications, publications, test methods, literature, and other materials cited herein are hereby incorporated herein by reference in their entireties. Melting points are uncorrected. NMR data are reported in parts per million (d) and are referenced to the deuterium lock signal from the sample solvent (deuteriochloroform unless otherwise specified). Mass Spectral data were obtained using a Micromass ZMD APCI Mass Spectrometer equipped with a Gilson gradient high performance liquid chromatograph. The following solvents and gradients were used for the analysis. Solvent A; 98% water/2% acetonirile/0.01% formic acid and solvent B; acetonitrile containing 0.005% formic acid. Typically, a gradient was run over a period of about 4 minutes starting at 95% solvent A and ending with 100% solvent B. The mass spectrum of the major eluting component was then obtained in positive or negative ion mode scanning a molecular weight range from 165 AMU to 1100 AMU. Specific rotations were measured at room temperature using the sodium D line (589 nm). Commercial reagents were utilized without further purification. THF refers to tetrahydrofuran. DMF refers to N,N-dimethylformamide. Chromatography refers to column chromatography performed using 32-63 mm silica gel and executed under nitrogen pressure (flash chromatography) conditions. Room or ambient temperature refers to 20-25°C. All non-aqueous reactions were run under a nitrogen atmosphere for convenience and to maximize yields. Concentration at reduced pressure means that a rotary evaporator was used.

One of ordinary skill in the art will appreciate that in some cases protecting groups may be required during preparation. After the target molecule is made, the protecting group can be removed by methods well known to those of ordinary skill in the art, such as described in Greene and Wuts, "Protective Groups in Organic Synthesis" (3rd Ed, John Wiley & Sons 1999).

### EXAMPLE 1

### 2-Chloro-5-(6-methyl-pyridin-3-yl)-N-(1-p-tolyl-cyclohexylmethyl)-benzamide

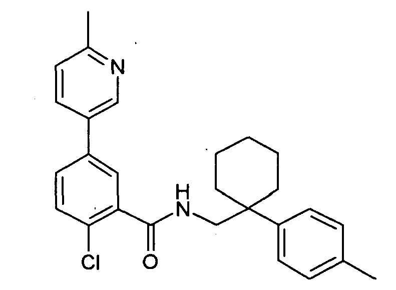

### 2-Chloro-5-iodo-benzoic acid methyl ester

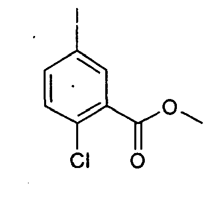

A solution of 2-chloro-5-iodo-benzoic acid (25.63 g, 90 mmol) in methanol (500 mL) saturated with HCL gas was stirred at room temperature for 48h. The reaction mixture was concentrated *in vacuo,* diluted with 1:1 ethyl acetate/Diethyl ether and washed with saturated aqueous sodium bicarbonate and brine. The organiclayer was dried over sodium sulfate, filtered and concentrated *in vacuo* to afford the title compound (25.0 g).

### 2-Chloro-5-(4,4,5,5-tetramethyl-[1,3,2] dioxaborolan-2-yl)-benzoic acid methyl ester

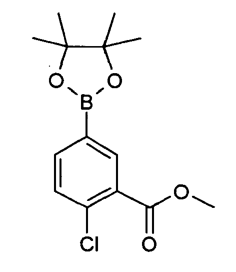

A mixture of 2-chloro-5-iodo-benzoic acid methyl ester (3.0 g, 10.17 mmol), bis(pinacolato)diborane (4.12 g, 16.27 mmol), [1,1'-bis(diphenylphosphino)ferrocene] dichloropalladium (II) dichloromethane (0.37 g, 0.46 mmol) and potassium acetate (4.48 g, 45.77 mmol) in *N,N* dimethylformamide (50 mL) was heated at 90 °C for 7h, then at room teperature for a further 16h. The mixture was diluted with 2:1 ethyl acetate - diethyl ether (250 mL) and filtered. The filtrate was washed with water and brine, dried over sodium sulfate and concentrated *in vacuo.* The residue was purified by flash column chromatography eluting with diethyl ether to afford the title compound as a dark oil (2.5 g)

### 2-Chloro-5-(6-methyl-pyridin-3-yl)-benzoic acid methyl ester

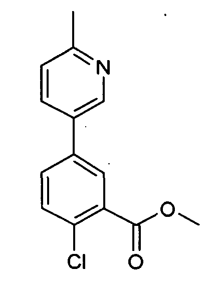

To a mixture of 2-chloro-5-(4,4,5,5-tetramethyl-[1,3,2]dioxaborolan-2-yl)-benzoic acid methyl ester (628 mg, 2.12 mmol), cesium carbonate (1.38 g, 4.25 mmol), tetrakis(triphenylphosphine) palladium (62 mg, 0.08 mmol) and molecular sieves (2 g, 4Å) in 1,4-dioxane (10 mL) was added 2-chloro-5-methylpyridine (226 mg, 1.77 mmol). The mixture was stirred for a few minutes at room temperature then warmed to 80 °C for 4h. A further portion of tetrakis(triphenylphosphine) palladium (120 mg, 0.10 mmol) was added and the resulting mixture was stirred at 80 °C for 16h. A second portion of 2-chloro-5-methylpyridine (117 mg, 0.66 mmol) was added. The resulting mixture was stirred at 80 °C for a further 5h. The reaction mixture was diluted with acetonitrile (30 mL) and filtered through a pad of celite. The filtrate was concentrated *in vacuo* and purified by flash column chromatography (gradient 5-100% ethyl acetate - hexane) to afford the title compound (146 mg).

### 2-Chloro-5-(6-methyl-pyridin-3-yl)-benzoic acid

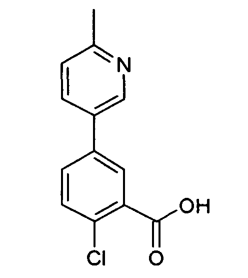

A solution of 2-chloro-5-(6-methyl-pyridin-3-yl)-benzoic acid methyl ester (146 mg, 0.56 mmol) in methanolic potassium hydroxide (2.24 mL, 1M) was stirred at 80 °C for 16h. The reaction mixture was acidified (pH 3) with 1N HCl and concentrated *in vacuo.* The residue was shaken thoroughly with methanol and filtered. The filtrate was concentrated *in vacuo* to afford the title compound (170 mg).

### 2-Chloro-5-(6-methyl-pyridin-3-yl)-N-(1-p-tolyl-cyclohexylmethyl)-benzamide

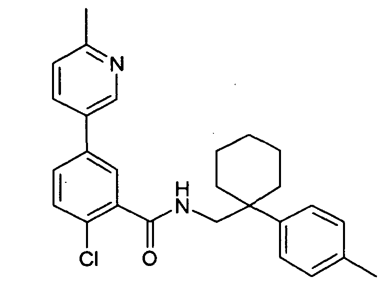

To a solution of 2-chloro-5-(6-methyl-pyridin-3-yl)-benzoic acid (73 mg, 0.29 mmol) in *N,N*-dimethylformamide (4 mL) was added sequentially, stirring at room temperature for 10 minutes after each addition, 1-hydroxybenzotriazole (48 mg, 0.35 mmol), 1-(3-dimethylaminopropyl)-3-ethylcarbodiimide hydrochloride (62 mg, 0.32 mmol), C-(1-p-tolyl-cyclohexyl)-methylamine (60 mg, 0.29 mmol) and triethylamine (31 mg, 0.31 mmol). The mixture was stirred at room temperature for 3h. The reaction mixture was diluted with water and extracted with ethyl acetate. The combined organic extracts were washed with brine, dried over sodium sulfate, filtered and concentrated *in vacuo.* The residue was purified by reverse phase chromatography to afford the title compound (35 mg). LCMS (m/z) 433.2 M+1.

The following compounds can be made according to the method of Example 1:

| EXAMPLES | STRUCTURE | NAME | LCMS (m/z) M+1. |
|---|---|---|---|
| 2 | 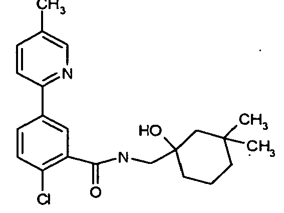 | 2-Chloro-N-(1-hydroxy-3,3-dimethyl-cyclohexylmethyl)-5-(5-methyl-pyridin-2-yl)-benzamide | 387.6 |
| 3 | 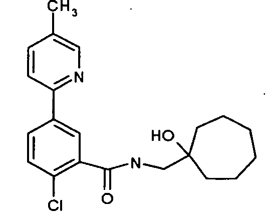 | 2-Chloro-N-(1-hydroxy-cycloheptylmethyl)-5-(5-methyl-pyridin-2-yl)-benzamide | 373.5 |
| 4 | 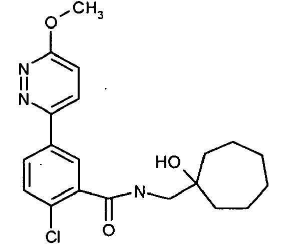 | 2-Chloro-N-(1-hydroxy-cycloheptylmethyl)-5-(6-methoxy-pyridazin-3-yl)-benzamide | 390.3 |
| 5 | 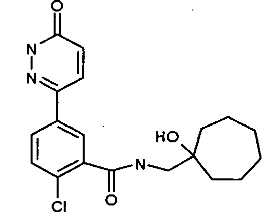 | 2-Chloro-N-(1-hydroxy-cycloheptylmethyl)-5-(6-oxo-1,6-dihydro-pyridazin-3-yl)-benzamide | 376.3 |
| 6 | 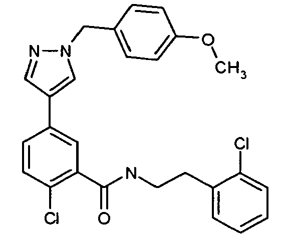 | 2-Chloro-N-[2-(2-chlorophenyl)-ethyl]-5-[1-(4-methoay-benzyl)-1H-pyrazol-4-yl]-benzamide | 480.5 |
| 7 | 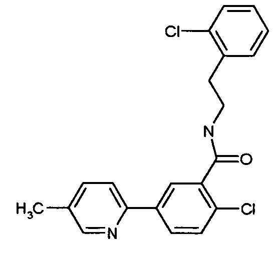 | 2-Chloro-N-[2-(2-chlorophenyl)-ethyl]-5-(5-methyl-pyridin-2-yl)-benzamide | 385.3 |
| 8 | 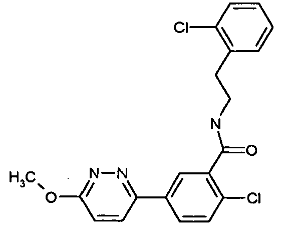 | 2-Chloro-N-[2-(2-chlorophenyl)-ethyl]-5-(6-methoxy-pyridazin-3-yl)-benzamide | 402.2 |
| 9 | 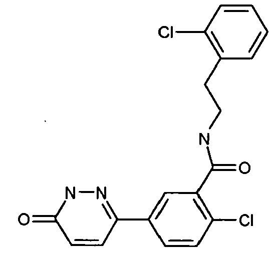 | 2-Chloro-N-[2-(2-chlorophenyl)-ethyl]-5-(6-oxo-1,6-dihydro-pyridazin-3-yl)-benzamide | 389.4 |

### EXAMPLE 10

### 2-Chloro-N-[2-(2-chloro-phenyl)-ethyl]-5-(1H-pyrazol-4-yl)-benzamide

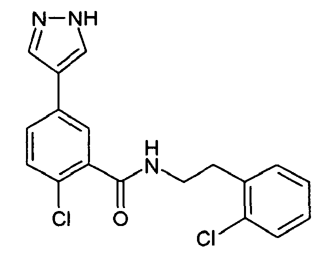

To as solution of 2-chloro-N-[2-(2-chloro-phenyl)-ethyl]-5-[1-(4-methoxybenzyl)-1H-pyrazol-4-yl]-benzamide (20 mg, 0.042 mmol) in dichloromethane (0.2 mL) was added trifluoroacetic acid (24 mg, 0.21 mmol) and anisole (45 mg, 0.42 mmol). The mixture was stirred at 110 °C for 16h. A second portion of trifluoroacetic acid (0.2 mL) and anisole (45 µL) was added and the mixture was stirred at 110 °C for a further 6h. The mixture was concentrated *in vacuo* to dryness and triturated with hexane to afford the title compound as an orange solid (17 mg). LCMS (m/z) 358.5 M+1.

### EXAMPLE 11

### 2-Chloro-(1-hydroxy-cycloheptylmethyl)-5-(5-methyl-1H-pyrazol-3-yl)-benzamide

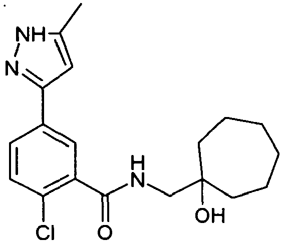

### 2-Chloro-5-trimethylsilanylethynyl-benzoic acid methyl ester

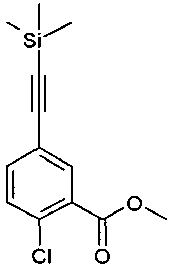

A mixture of 2-chloro-5-iodo-benzoic acid methyl ester (3.5 g, 12 mmol), dichlorobis(triphenylphosphino)palladium (II) (0.04 g, 0.06 mmol), triphenylphosphine (0.06 g, 0.24 mmol), copper iodide (0.05 g, 0.24 mmol), (trimethylsilyl)acetylene (1.9 g, 19.2 mmol) in triethylamine (40 mL) was heated at reflux for 12h. The mixture was concentrated *in vacuo,* diluted with ethyl acetate (150 mL) and washed sequentially with 10% aqueous citric acid, water, and brine. The organic solution was dried, filtered and concentrated *in vacuo* to afford the title compound (3.14 g).

### 5-Acetyl-2-chloro-benzoic acid methyl ester

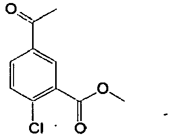

A mixture of 2-chloro-5-trimethylsilanylethynyl-benzoic acid methyl ester (3.2 g, 12 mmol) in formic acid (50 mL) was heated at reflux for 16h. The mixture was cooled to room temperature and concentrated *in vacuo.* The residue was diluted with ethyl acetate and washed with saturated aqueous sodium bicarbonate, water and brine. The organic layer was dried, filtered and concentrated *in vacuo* to afford the title compound (1.4 g).

### 2-Chloro-5-(5-methyl-1H-pyrazol-3-yl)-benzoic acid methyl ester

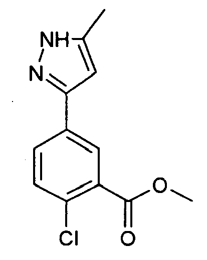

A solution of 5-acetyl-2-chloro-benzoic acid methyl ester (1.58 g, 7.5 mmol) and *N,N* dimethylacetamide dimethylacetal in *N,N* dimethylformamide and *N,N-*dimethylacetamide was stirred at 90 °C for 3h. The mixture was cooled to room temperature and concentrated *in vacuo.* The residue was diluted with ethanol (18 mL) and tetrahydrofuran (2 mL). Hydrazine hydrate (0.34 mL) was added and the mixture was stirred at room temperature for 16h. The mixture was concentrated *in vacuo.* The residue was purified by flash column chromatography (gradient 0-10% methanol -ethyl acetate) to afford the title compound (1.0 g).

### 2-Chloro-5-(5-methyl-1H-pyrazol-3-yl)-benzoic acid

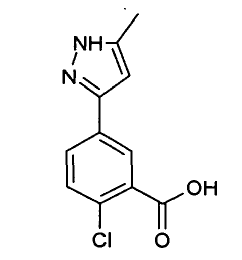

A solution of 2-chloro-5-(5-methyl-1H-pyrazol-3-yl)-benzoic acid methyl ester (4.5 g, 17.0 mmol) in methanol (20.0 mL) was treated with potassium hydroxide (4.52 g, 80.0 mmol). The mixture was stirred at room temperature for 16h. The mixture was acidified to pH 4.0 with conc. HCl Methanol was removed from the mixture under *vacuo.* The residue was stirred at rt for 2 h and the solids collected by filtration, washed with water, 2:1 Hexanes/ether and dried to give the title compound (2.2 g)

### 2-Chloro-(1-hydroxy-cycloheptylmethyl)-5-(5-methyl-1H-pyrazol-3-yl)-benzamide

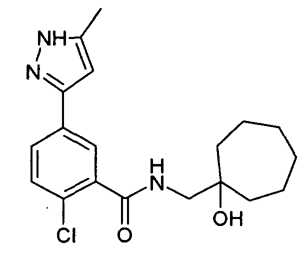

To a solution of 2-chloro-5-(5-methyl-1H-pyrazol-3-yl)-benzoic acid (100 mg, 0.422 mmol) in DMF (10 mL) was added 1-hydroxybenzotriazole (85 mg, 0.63 mmol), polystyrene supported 1-(3-dimethylaminopropyl)-3-ethylcarbodiimide (1.24 g, 1.27 mmol) and 1-aminomethyl-cycloheptanol hydrochloride (113.4 mg, 0.633 mmol). The mixture was stirred at room temperature for 15 minutes, then polystyrene supported *N,N*-dimethylaminopyridine (0.64 g, 0.93 mmol) was added and the mixture was stirred at room temperature for 16h. The mixture was filtered through a glass frit, and the residue was washed thoroughly with methanol. The filtrate was concentrated to dryness *in vacuo.* The residue was purified by reverse phase chormatography to afford the title compound as a white solid (40 mg).LC/MS (M/z): 362.5 (M+1)

The following examples can be made according to the method in Example III:

| EXAMPLES | STRUCTURE | NAME | DATA LCMS M/Z (M+1) |
|---|---|---|---|
| 12 | 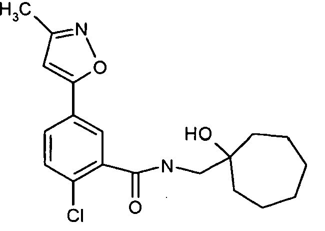 | 2-Chloro-N-(1-hydroxy-cycloheptylmethyl)-5-(3-methyl-isoxazol-5-yl)-benzamide | 363.4 |
| 13 | 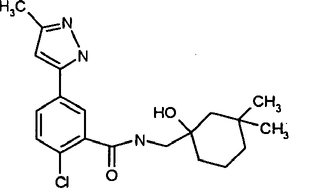 | 2-Chloro-N-(1-hydroxy-3,3-dimethyl-cyclohexylmethyl)-5-(5-methyl-2H-pyrazol-3-yl)-benzamide | 376.6 |
| 14 | 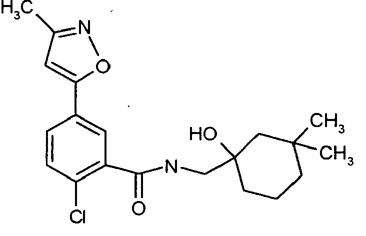 | 2-Chloro-N-(1-hydroxy-3,3-dimethyl-cyclohexylmethyl)-5-(3-methyl-isoxazol-5-yl)-benzamide | 377.5 |
| 15 | 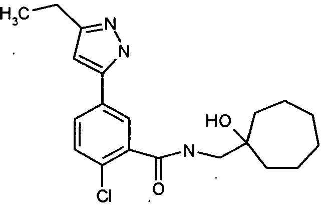 | 2-Chloro-5-(5-ethyl-2H-pyrazol-3-yl)-N-(1-hydroxy-cycloheptylmethyl)-benzamide | 377.5 |
| 16 | 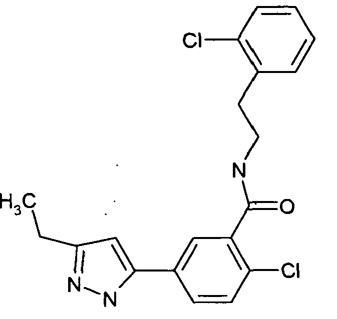 | 2-Chloro-N-[2-(2-chloro-phenyl)-ethyl]-5-(5-ethyl-2H-pyrazol-3-yl)-benzamide | 388.4 |
| 17 | 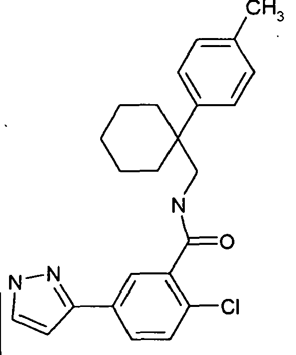 | 2-Chloro-5-(1H-pyrazol-3-yl)-N-(1-p-tolyl-cyclohexylmethyl)-benzamide | 408.6 |
| 18 | 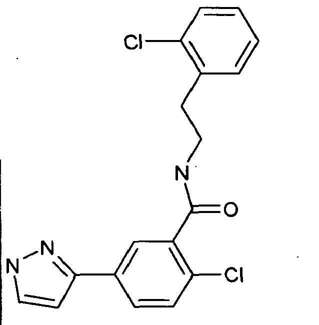 | 2-Chloro-N-[2-(2-chloro-phenyl)-ethyl]-5-(1H-pyrazol-3-yl)-benzamide | 360.5 |
| 19 | 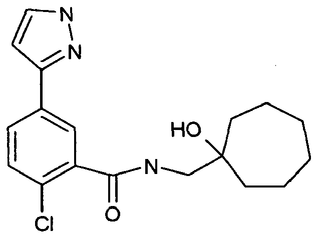 | 2-Chloro-N-(1-hydroxy-cycloheptylmethyl)-5-(1H-pyrazol-3-yl)-benzamide | 348.2 |
| 20 | 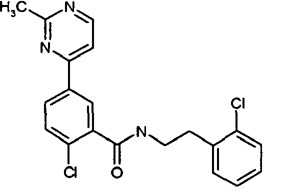 | 2-Chloro-N-[2-(2-chloro-phenyl)-ethyl]-5-(2-methyl-pyrimidin-4-yl)-benzamide | 389.3 |
| 21 | 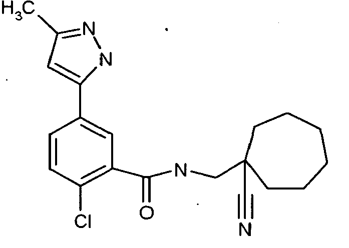 | 2-Chloro-N-(1-cyano-cycloheptylmethyl)-5-(5-methyl-2H-pyrazol-3-yl)-benzamide | 371.3 |

### EXAMPLE 22

### 2-Chloro-5-(5-cyclopropyl-1H-pyrazol-3-yl)-benzoic acid ethyl ester

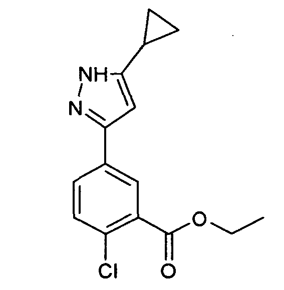

To a solution of 5-acetyl-2-chloro-benzoic acid ethyl ester (0.45 g, 2 mmol) in toluene (10 mL) at 0 °C was added potassium tert-butoxide (2.0 mL, 2 mmol, 1M THF). The mixture was stirred at room temperature for 10 miutes, then cyclopropanecarbonyl chloride (2.0 mL, 2 mmol, 1M toluene) was added. The mixture was stirred at room temperature for 2h. Polymer bound sulfonic acid (3 eq) was added and the mixture was stirred at room temperature for 10 minutes, filtered, and the filtrate concentrated *in vacuo.* The residue was dissolved in ethanol (10 mL) and stirred with hydrazine (0.15 mL) at room temperature for 12h. The mixture was concentrated *in vacuo,* stirred with silica bound sulfonic acid for 10 minutes, filtered, washed with methanol and eluted with methanolic ammonia (1.0 M). The crude residue was purified by flash column chromatography (hexane - ethyl acetate gradient) to afford the title compound (0.05 g).

### 2-Chloro-5-(5-cyclopropyl-1H-pyrazol-3-yl)-benzoic acid

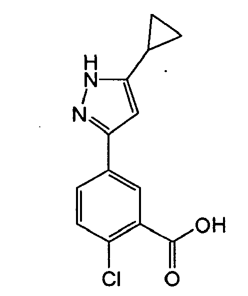

To a solution of 2-chloro-5-(5-cyclopropyl-1H-pyrazol-3-yl)-benzoic acid ethyl ester (0.05 g, 0.17 mmol) in methanol (4 mL) was added KOH (0.12 g). The mixture was shaken at room temperature for 16h. The mixture was acidified with 10% aqueous citric acid and extracted with ethyl acetate. The combined organic layers were washed with water and brine, and concentrated *in vacuo* to afford the title compound (0.06 g).

### 2-Chloro-5-(5-cyclopropyl-1H-pyrazol-3-yl)-N-(1-hydroxy-cycloheptylmethyl)-benzamide

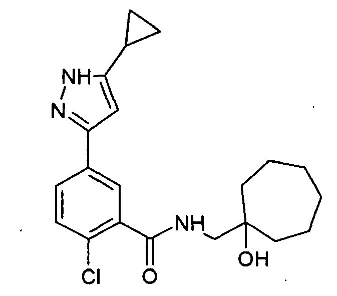

A mixture of 2-chloro-5-(5-cyclopropyl-1H-pyrazol-3-yl)-benzoic acid (0.06 g, 0.23 mmol), 1-aminomethyl-cycloheptanol ((0.082 g, 0.46 mmol), polystyrene supported carbonyldiimidazole (0.37 g, 0.46 mmol), polystyrene supported dimethylaminopyridine (0.32 g, 0.46 mmol) and 1-hydroxybenztriazole (0.06 g, 0.46 mmol) in *N,N*-dimethylformamide (3 mL) was shaken at room temperature for 16 h. MP-carbonate was added (0.5 g) and the resulting mixture was shaken at room temperature for 2h. The mixture was filtered and the filtrate was concentrated *in vacuo.* the residue was purified by reverse phase chromatography to afford the title compound (0.02 g). LCMS (m/z) 388.4 M+1.

The following examples can be made according to the method in Example 22:

| EXAMPLES | STRUCTURE | NAME | DATA LCMS M/Z (M+1) |
|---|---|---|---|
| 23 | 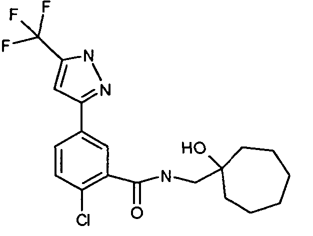 | 2-Chloro-N-(1-hydroxy-cycloheptylmethyl)-5-(5-trifluoromethyl-1H-pyrazol-3-yl)-benzamide | 416.5 |
| 24 | 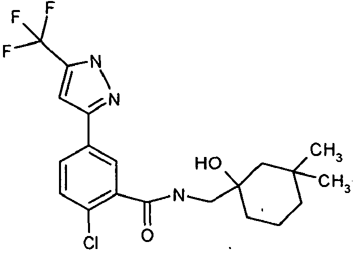 | 2-Chloro-N-(1-hydroxy-3,3-dimethyl-cyclohexylmethyl)-5-(5-trifluoromethyl-1H-pyrazol-3-yl)-benzamide | 430.6 |

### EXAMPLE 25

### 2-Chloro-N-(1-hydroxy-cycloheptylmethyl)-5-[1-(2-hydroxy-ethyl)-5-methyl-1H-pyrazol-3-yl]-benzamide

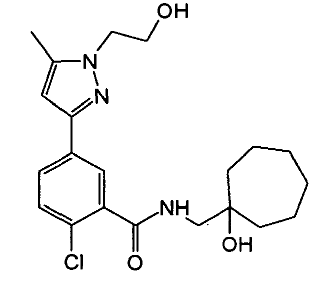

A mixture of 2-chloro-N-(1-hydroxy-cycloheptylmethyl)-5-(5-methyl-1H-pyrazol-3-yl)-benzamide (5.0 g, 13.8 mmol) and cesium carbonate (9.0 g, 27.7 mmol) in dimethylsulfoxide (50 mL) was stirred at room temperature for 10 minutes. Bromoethanol (1.9 g, 15.2 mmol) was added and the resulting mixture was stirred at 80 °C for 12h. The mixture was cooled to room temperature and filtered. The filtrate was diluted with ethyl acetate (300 mL), washed with water and brine, dried, filtered and concentrated *in vacuo.* The residue was triturated with dichloromethane - ethyl acetate - diethyl ether (1:1:1, 100 mL) and washed with ethyl acetate to afford the title compound (3.3 g). LCMS (m/z) 406.1 M+1.

The following examples can be made according to the method in Example 25:

| EXAMPLES | STRUCTURE | NAME | DATA LCMS M/Z (M+1) |
|---|---|---|---|
| 26 | 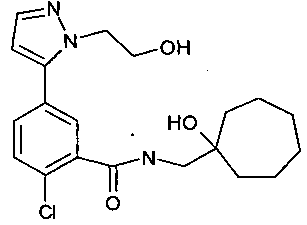 | 2-Chloro-N-(1-hydroxy-cycloheptylmethyl)-5-[2-(2-hydroxy-ethyl)-2H-pyrazol-3-yl]-benzamide | 392.5 |
| 27 | 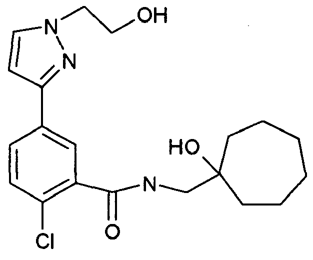 | 2-Chloro-N-(1-hydroxy-cycloheptylmethyl)-5-[1-(2-hydroxy-ethyl)-1H-pyrazol-3-yl]-benzamide | 392.5 |
| 28 | 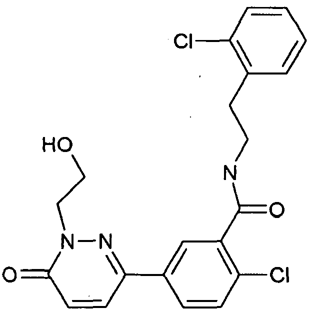 | 2-Chloro-N-[2-(2-chloro-phenyl)-ethyl]-5-[1-(2-hydroxy-ethyl)-6-oxo-1,6-dihydro-pyridazin-3-yl]-benzamide | 432.4 |
| 29 | 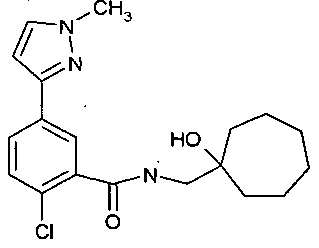 | 2-Chloro-N-(1-hydroxy-cycloheptylmethyl)-5-(1-methyl-1H-pyrazol-3-yl)-benzamide | 362.4 |
| 30 | 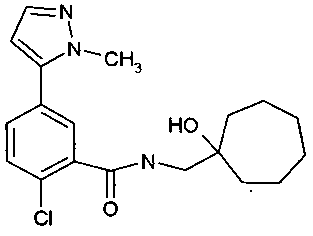 | 2-Chloro-N-(1-hydroxy-cycloheptylmethyl)-5-(2-methyl-2H-pyrazol-3-yl)-benzamide | 362.4 |
| 31 | 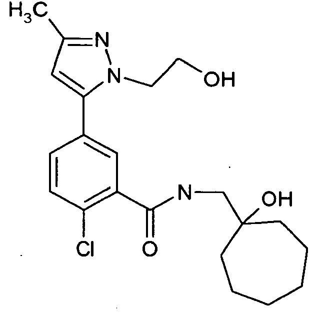 | 2-Chloro-N-(1-hydroxy-cycloheptylmethyl)-5-[2-(2-hydroxy-ethyl)-5-methyl-2H-pyrazol-3-yl]-benzamide | 407.3 |
| 32 | 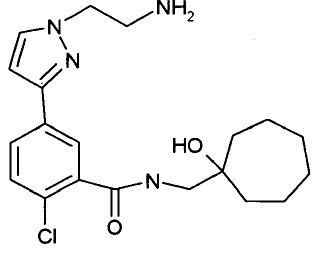 | 5-[1-(2-Amino-ethyl)-1H-pyrazol-3-yl]-2-chloro-N-(1-hydroxy-cycloheptylmethyl)-benzamide | 391.4 |
| 33 | 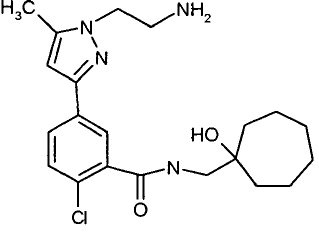 | 5-[1-(2-Amino-ethyl)-5-methyl-1H-pyrazol-3-yl]-2-chloro-N-(1-hydroxy-cycloheptylmethyl)-benzamide | 405.4 |
| 34 | 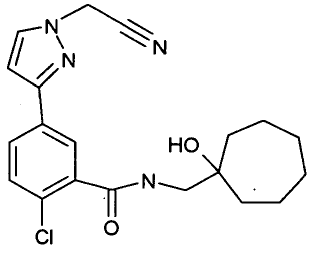 | 2-Chloro-5-(1-cyanomethyl-1H-pyrazol-3-yl)-N-(1-hydroxy-cycloheptylmethyl)-benzamide | 387.5 |
| 35 | 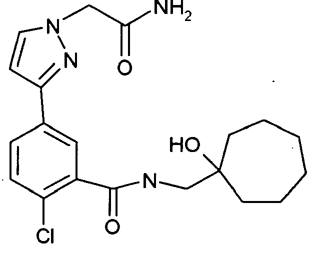 | 5-(1-Carbamoylmethyl-1H-pyrazol-3-yl)-2-chloro-N-(1-hydroxy-cycloheptylmethyl)-benzamide | 405.4 |
| 36 | 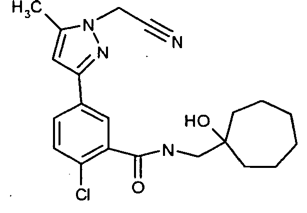 | 2-Chloro-5-(1-cyanomethyl-5-methyl-1H-pyrazol-3-yl)-N-(1-hydroxy-cycloheptylmethyl)-benzamide | 401.5 |
| 37 | 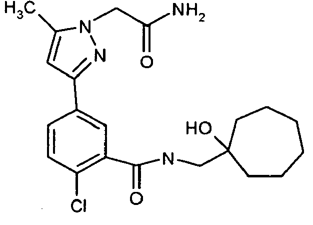 | 5-(1-Carbamoylmethyl-5-methyl-1H-pyrazol-3-yl)-2-chloro-N-(1-hydroxy-cycloheptylmethyl)-benzamide | 419.4 |
| 38 | 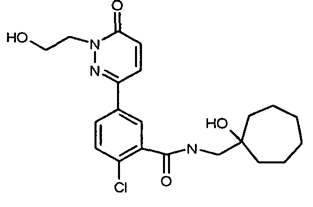 | 2-Chloro-N-(1-hydroxy-cycloheptylmethyl)-5-[1-(2-hydroxy-ethyl)-6-oxo-1,6-dihydro-pyridazin-3-yl]-benzamide | 420.5 |

### EXAMPLE 39

### 2-Chloro-N-(1-hydroxy-cycloheptylmethyl)-5-[1-(2-hydroxy-3-methoxy-propyl)-5-methyl-1H-pyrazol-3-yl]-benzamide

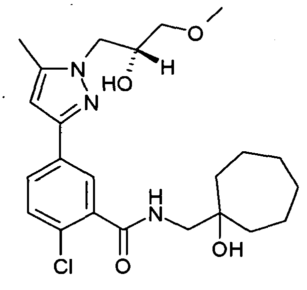

A mixture of 2-chloro-N-(1-hydroxy-cycloheptylmethyl)-5-(5-methyl-1H-pyrazol-3-yl)-benzamide (0.36 g, 1 mmol) and 2-methoxymethyl-oxirane (0.8 mL) was stirred in *N,N*-dimethylformamide (1.0 mL) at 90 °C for 12h. The mixture was concentrated *in vacuo.* The residue was purified by reverse phase chromatography, followed by trituration (1:1:1 diethyl ether - dichloromethane - ethyl acetate) to afford the title compound (0.08 g). LCMS (m/z) 450.1 M+1

The following examples can be made according to the method in Example 39:

| EXAMPLE | STRUCTURE | NAME | DATA LCMS M/Z (M+1) |
|---|---|---|---|
| 40 | 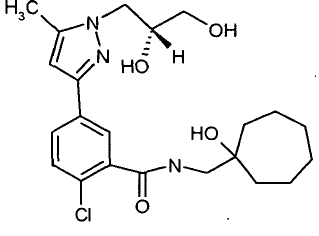 | 2-Chloro-5-[1-(2,3-dihydroxy-propyl)-5-methyl-1H-pyrazol-3-yl]-N-(1-hydroxy-cycloheptylmethyl)benzamide | 436.2 |
| 41 | 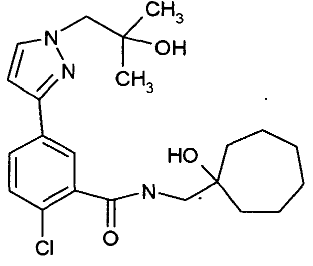 | 2-Chloro-N-(1-hydroxy-cycloheptylmethyl)-5-[1-(2-hydroxy-2-methyl-propyl)-1H-pyrazol-3-yl]-benzamide | 420.6 |
| 42 | 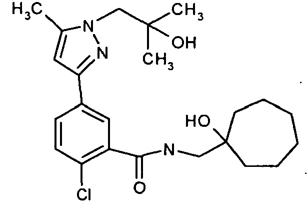 | 2-Chloro-N-(1-hydroxy-cycloheptylmethyl)-5-[1-(2-hydroxy-2-methyl-propyl)-5-methyl-1H-pyrazol-3-yl]-benzamide | 434.4 |
| 43 | 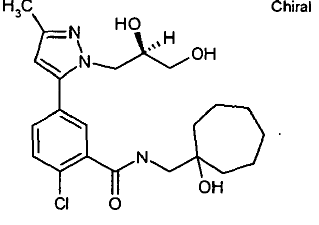 | 2-Chloro-5-[2-(2,3-dihydroxy-propyl)-5-methyl-2H-pyrazol-3-yl]-N-(1-hydroxy-cycloheptylmethyl)-benzamide | 436.6 |
| 44 | 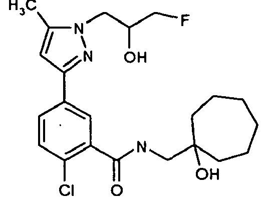 | 5-[1-(3-Amino-2-hydroxy-propyl)-5-methyl-1H-pyrazol-3-yl]-2-chloro-N-(1hydroxy-cycloheptylmethyl)-benzamide | 435.5 |
| 45 | 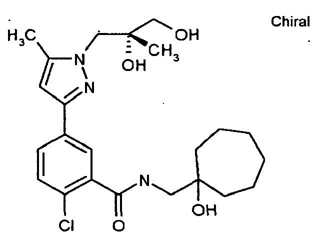 | 2-Chloro-5-[1-(2,3-dihydroxy-2-methyl-propyl)-5-methyl-1H-pyrazol-3-yl]-N-(1-hydroxy-cycloheptylmethyl)-benzamide | |

### EXAMPLE 46

### (3-{4-Chloro-3-[(1-hydroxy-cycloheptylmethyl)-carbamoyl]-phenyl}-5-methyl-pyrazol-1-yl)-acetic acid methyl ester

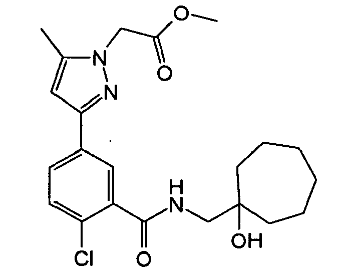

A mixture of 2-chloro-N-(1-hydroxy-cycloheptylmethyl)-5-(5-methyl-1H-pyrazol-3-yl)-benzamide (1.08 g, 3 mmol), 2-*tert*-butylimino-2-diethylamino-1,3-dimethyl-perhydro-1,2,3-diazaphosphorine on polystyrene (2.0 g, 4.6 mmol) and bromo-acetic acid methyl ester (0.69 g, 4.5 mmol) in *N,N*-dimethylformamide (10 mL) was stirred at room temperature for 2h. The mixture was filtered and washed with dichloromethane. The filtrate was concentrated *in vacuo.* The residue was triturated with ethyl acetate to afford the title compound (1.05 g). (3-{4-Chloro-3-[(1-hydroxy-cycloheptylmethyl)-carbamoyl]-phenyl}-5-methyl-pyrazol-1-yl)-acetic acid.

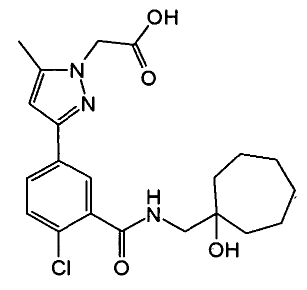

A mixture of (3-{4-chloro-3-[(1-hydroxy-cycloheptylmethyl)-carbamoyl]-phenyl}-5-methyl-pyrazol-1-yl)-acetic acid methyl ester (1.63 g, 3.76 mmol) and potassium hydroxide (1.12 g, 20 mmol) in methanol (25 mL) and water (5 mL) was stirred at room temperature for 6h. The mixture was acidified to pH6 with 10% aqueous citric acid and concentrated *in vacuo.* 1M HCl was added until the mixture reached pH4. The resulting solids were collected by filtration and washed sequentially with water, hexanes and diethyl ether to afford the title compound (1.1 g).

### 2-Chloro-N-(1-hydroxy-cycloheptylmethyl)-5-[5-methyl-1-(2-oxo-2-pyrrolidin-1-yl-ethyl)-1H-pyrazol-3-yl]-benzamide

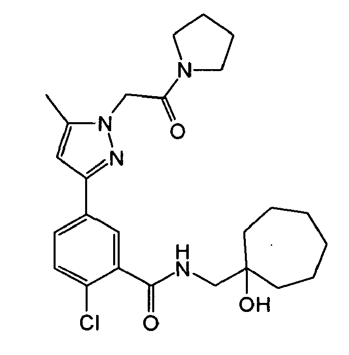

To a solution of (3-{4-chloro-3-[(1-hydroxy-cydoheptylmethyl)-carbamoyl]-phenyl}-5-methyl-pyrazol-l-yl)-acetic acid (41.9 mg, 0.1 mmol) in *N,N-*dimethylformamide (1 mL) was added a solution of 1-hydroxybenzotriazole (20.25 mg, 0.15 mmol) in *N,N*-dimethylformamide (0.5 mL) and carbonyldiimidazole on polystyrene (294 mg, 0.3 mmol). The mixture was shaken at room temperature for 10 minutes. Pyrrolidine (10.65 mg, 0.15 mmol) was added as a solution in *N,N-*dimethylformamide (1.5 mL). Polystyrene supported dimethylaminopyridine (152 mg, 0.22 mmol) was added and the mixture was shaken at room temperature for 16h. MP-carbonate resin (139 mg, 0.4 mmol) was added and the mixture was shaken at room temperature for 3h. The mixture was filtered, washed with methanol and concentrated *in vacuo.* The residue was purified by reverse phase chromatography to afford the title compound (22 mg). LCMS (m/z) 473.4 M+1.

**The following examples can be made according to the method in Example 47:**

| EXAMPLES | STRUCTURE | NAME | DATA LCMS M/Z (M+1) |
|---|---|---|---|
| 47 | 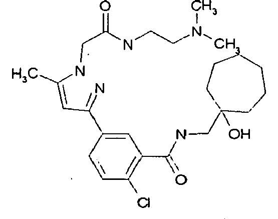 | 2-Chloro-5- {1-[(2-dimethylamino-ethylcarbamoyl)-methyl]-5-methyl-1H-pyrazol-3-yl}-N-(1-hydroxy-cycloheptylmethyl)-benzamide | 490.4 |
| 48 | 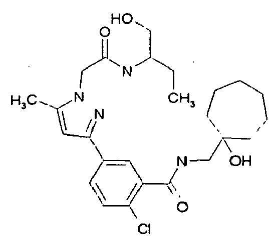 | 2-Chloro-N-(1-hydroxy-cycloheptylmethyl)-5-{1-[(1-hydroxymethyl-propylcarbamoyl)-methyl]-5-methyl-1H-pyrazol-3-yl)-benzamide | 491.4 |
| 49 | 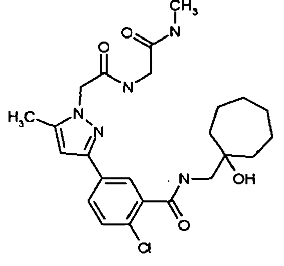 | 2-Chloro-N-(1-hydroxy-cycloheptylmethyl)-5-{5-methyl-1-[(methylcarbamoylmethyl-carbamoyl)-methyl]-1H-pyrazol-3-yl}-benzamide | 490.4 |
| 50 | 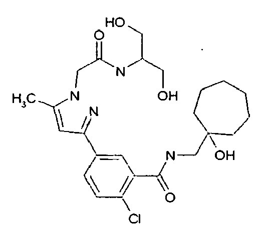 | 2-Chloro-N-(1-hydroxy-cycloheptylmethyl)-5- {1-[(2-hydroxy-1-hydroxymethyl-ethylcarbamoyl)-methyl]-5-methyl-1H-pyrazol-3-yl}-benzamide | 493.4 |
| 51 | 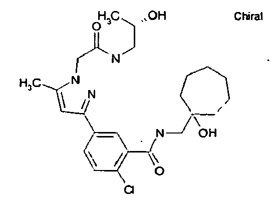 | 2-Chloro-N-(1-hydroxy-cycloheptylmethyl)-5- {1-[(2-hydroxy-propylcarbamoyl)-methyl]-5-methyl-1H-pyrazol-3-yl}-benzamide | 477.4 |
| 52 | 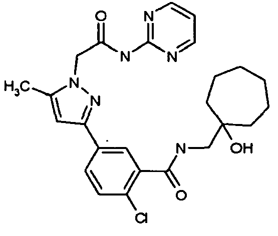 | 2-Chloro-N-(1-hydroxy-cycloheptylmethyl)-5-[5-methyl-1-(pyrimidin-2-ylcarbamoylmethyl)-1H-pyrazol-3-yl]-benzamide | 497.5 |
| 53 | 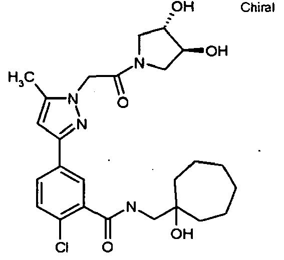 | 2-Chloro-5-{1-[2-(3,4-dihydroxy-pyrrolidin-1-yl)-2-oxo-ethyl]-5-methyl-1H-pyrazol-3-yl}-N-(1-hydroxy-cycloheptylmethyl)-benzamide | 505.3 |

### EXAMPLE 54

### 2-Chloro-N-(1-hydroxy-cycloheptylmethyl)-5-(5-methyl-l-oxiranylmethyl-1H-pyrazol-3-yl)-benzamide

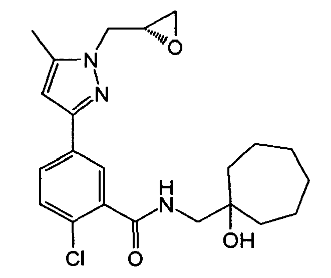

A mixture of 2-chloro-N-(1-hydroxy-cycloheptylmethyl)-5-(5-methyl-1H-pyrazol-3-yl)-benzamide (3.95 g, 11 mmol), 2-*tert*-butylimino-2-diethylamino-1,3-dimethyl-perhydro-1,2,3-diazaphosphorine on polystyrene (10.0 g, 23 mmol) in acetonitrile (60 mL) and *N,N*-dimethylformamide (20 mL) was stirred at room temperature for 10 minutes. 2R-(-)-glycidyl 3-nitrobenzenesulfonate (3.0 g, 12.5 mmol) was added and the resulting mixture was stirred at 80 °C for 8h. The mixture was filtered and concentrated *in vacuo.* the residue was purified by flash column chromatography (gradient dichloromethane - ethyl acetate - methanol) to afford the title compound (2.5 g).

### 5-[1-(3-Amino-2-hydroxy-propyl)-5-methyl-1H-pyrazol-3-yl]-2-chloro-N-(1-hydroxy-cycloheptylmethyl)-benzamide

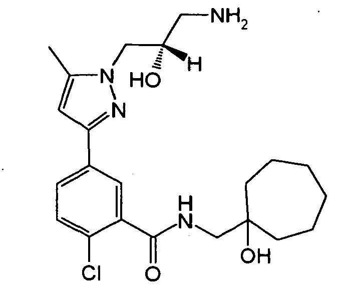

A mixture of 2-chloro-N-(1-hydroxy-cycloheptylmethyl)-5-(5-methyl-1-oxiranylmethyl-1H-pyrazol-3-yl)-benzamide (0.4 g, 0.96 mmol) in methanolic ammonia (20 mL, 7.0 N) was heated to 67 °C in a sealed tube for 16h. The mixture was concentrated *in vacuo,* and the residue was triturated with diethyl ether - ethyl acetate to afford the title compound (0.12 g). LCMS (m/z) 435.3 M+1.

### 5-[1-(3-Amino-2-hydroxy-propyl)-5-methyl-1H-pyrazol-3-yl]-2-chloro-N-(1-hydroxy-cycloheptylmethyl)-benzamide Hydrochloride.

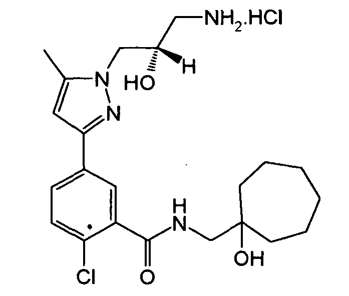

To a solution of 5-[1-(3-Amino-2-hydroxy-propyl)-5-methyl-1H-pyrazol-3-yl]-2-chloro-N-(1-hydroxy-cycloheptylmethyl)-benzamide (1.05 g, 2.41 mmol) in dichloromethane (10.0 mL) was added 2.0 N Hydrochloric acid in diethylether (4.0 mL, 8.0 mmol). The mixture was diluted with ether (50 mL) and stirred for 15 min. The precipitated solids were collected by filtration, washed with ether and dried to give title compound (1.0 g): LCMS (m/z) 435.3 M+1.

The following examples can be made according to the method in Example 55:

| EXAMPLE | STRUCTURE | NAME | DATA LCMS M/Z |
|---|---|---|---|
| 55 | 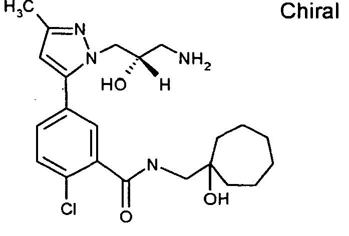 | 5-[2-(3-Amino-2-hydroxy-propyl)-5-methyl-2H-pyrazol-3-yl]-2-chloro-N-(1-hydroxy-cycloheptylmethyl)-benzamide | 435.5 |

### EXAMPLE 56

### 2-Chloro-5-(2H-[1,2,3]triazol-4-yl)-benzoic acid methyl ester

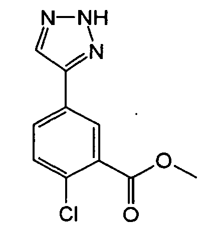

A mixture of 2-chloro-5-ethynyl-benzoic acid methyl ester (0.18 g, 0.92 mmol) and trimethylsilyl azide (0.16 g, 1.39 mmol) in *n*-butanol was heated to reflux for 16h. A second portion of trimethylsilyl azide (0.16 g, 1.39 mmol) was added and the mixture was refluxed for a further 16h. A third portion of trimethylsilyl azide (0.16 g, 1.39 mmol) was added and the mixture was refluxed for a further 16h. Silica gel was added and the mixture was concentrated *in vacuo.* The residue was purified by flash column chromatography (2:1 hexanes - ethyl acetate) to afford the title compound (0.095 g).

### 2-Chloro-5-(2H-[1,2,3]triazol-4-yl)-benzoic acid

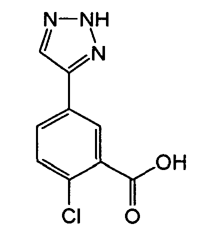

To a solution of 2-chloro-5-(2H-[1,2,3]triazol-4-yl)-benzoic acid methyl ester (0.095 g, 0.4 mmol) in methanol (2 mL) was added potassium hydroxide (0.25 g). The mixture was shaken at room temperature for12h. The mixture was diluted with methanol and acidified with HCl (2 mL, 6M). The mixture was concentrated *in vacuo,* diluted with water and sonicated. The solids were collected by filtration and washed with hexanes to afford the title compound (0.075 g).

### 2-Chloro-N-(1-hydroxy-cycloheptylmethyl)-5-(2H-[1,2,3]triazol-4-yl)-benzamide

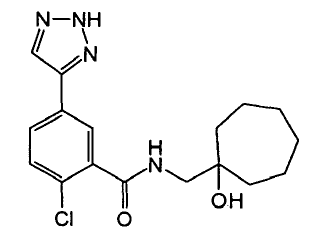

To a solution of 2-chloro-5-(2H-[1,2,3]triazol-4-yl)-benzoic acid (0.03g, 0.136 mmol) in DMF (1.0 mL) was added 1-hydroxybenzotriazole (28 mg, 0.204 mmol), polystyrene supported 1-(3-dimethylaminopropyl)-3-ethylcarbodiimide (0.4 g, 0.4 mmol) and 1-aminomethyl-cycloheptanol hydrochloride (0.036 g, 0.204 mmol). The mixture was stirred at room temperature for 10 minutes, then polystyrene supported N, N-dimethylaminopyri dine (0.2 g, 0.29 mmol) was added and the mixture was shaken at room temperature for 16h. MP-carbonate was added (200 mg) and the mixture was shaken at room temperature for 3h. The mixture was filtered and washed with methanol. the filtrate was concentrated *in vacuo* and purified by reverse phase chromatography to afford the title compound (22 mg). LCMS (m/z) 349.3 M+1.

### EXAMPLE 57

### 2-Chloro-N-(1-hydroxy-cycloheptylmethyl)-5-(1H-imidazol-4-yl)-benzamide

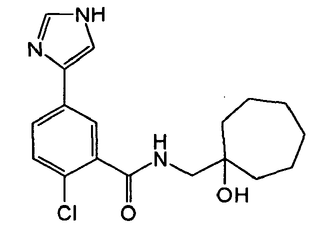

A solution of 2-chloro-5-formyl-N-(1-hydroxy-cycloheptylmethyl)-benzamide (50 mg, 0.16 mmol) and ammonium hydroxide (0.5 mL, 0.44 mmol) in THF (5 mL) was stirred at room temperature for 6h. *p*-Toluenesufonylmethylisocyanate (21 mg, 0.11 mmol) and piperazine (14 mg, 0.16 mmol) were added and the mixture was stirred at room temperature for 5d. The mixture was diluted with ethyl acetate and water. The organic layer was washed with brine, dried over sodium sulfate, filtered and concentrated *in vacuo.* The residue was dissolved in HCl (20 mL, 1M) and washed with ethyl acetate. The aqueous layer was saponified to pH12 with sodium hydroxide (6M) and extracted with ethyl acetate. The combined organic layers were washed with brine, dried over sodium sulfate, filtered and concentrated *in vacuo* to afford the title compound as a white solid (12 mg).

### 2-Chloro-5-[1-(3-fluoro-2-hydroxy-propyl)-1H-imidazol-4-yl]-N-(1-hydroxy-cycloheptylmethyl)-benzamide

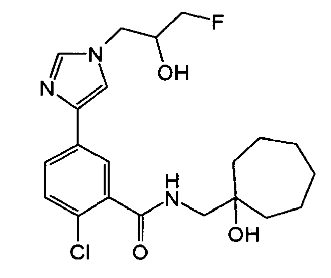

A solution of 2-chloro-N-(1-hydroxy-cycloheptylmethyl)-5-(1H-imidazol-4-yl)-benzamide(5 mg, 0.014 mmol) and 2-fluoromethyl-oxirane (0.06 mL, 0.086 mmol) in *N,N* dimethylformamide (0.2 mL) was heated at 65 °C in a sealed tube for 20h. The mixture was diluted with ethyl acetate and washed with water, and brine, dried over sodium sulfate, filtered and concentrated *in vacuo* to afford the title compound (5.2 mg).

### EXAMPLE 58

### 2-Chloro-N-(1-hydroxy-cycloheptylmethyl)-5-(hydroxyimino-methyl)-benzamide

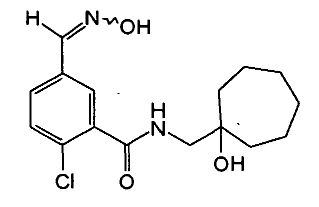

A solution of 2-chloro-5-formyl-N-(1-hydroxy-cycloheptylmethyl)-benzamide (290 mg, 0.94 mmol), hydroxylamine hydrochloride (78 mg, 1.13 mmol) and sodium acetate (223 mg, 2.72 mmol) in methanol (5 mL) and water (5 mL) was stirred at room temperature for 3h. The mixture was diluted with water and extracted with ethyl acetate. The combined organic layers were washed with water, and brine, dried over sodium sulfate, filtered and adsorbed onto silica gel. The residue was purified by chromatography (1:1 ethyl acetate - hexane) to afford the title compound (140 mg).

### 2-Chloro-N-(1-hydroxy-cycloheptylmethyl)-5-[5-(2-hydroxy-ethyl)-isoxazol-3-yl]-benzamide

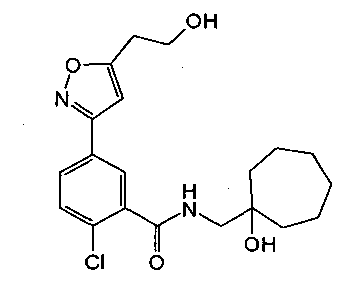

A mixture of 2-chloro-N-(1-hydroxy-cycloheptylmethyl)-5-(hydroxyiminomethyl)-benzamide (50 mg, 0.15 mmol), but-3-yn-1-ol (27 mg, 0.38 mmol), sodium hypochlorite (1 mL, 4%) and triethylamine (2 drops) in dichloromethane (2 mL) was stirred at room temperature for 5d. The mixture was diluted with water and extracted with ethyl acetate. The combined organic layers were washed with water, and brine, dried over sodium sulfate, filtered and adsorbed onto silica gel. The residue was purified by flash column chromatography (65% ethyl acetate - hexanes) to afford the title compound (13 mg).

### EXAMPLE 59

### 5-(2-Bromo-acetyl)-2-chloro-benzoic acid methyl ester

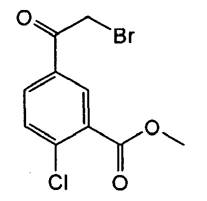

To a solution of 5-acetyl-2-chloro-benzoic acid methyl ester (100 mg, 0.47 mmol) in glacial acetic acid (5 mL) was added bromine (0.05 mL, 0.94 mmol). The mixture was stirred at room temperature for 2h. Hydrobromic acid - acetic acid (2 drops, 30%) was added and the mixture was stirred for 10 minutes. The mixture was concentrated *in vacuo* to afford the title compound (140 mg).

### 2-Chloro-5-(2-methyl-thiazol-4-yl)-benzoic acid methyl ester

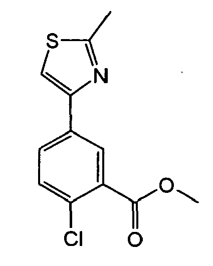

A mixture of 5-(2-bromo-acetyl)-2-chloro-benzoic acid methyl ester (25 mg, 0.09 mmol) and thioacetamide (6.5 mg, 0.08 mmol) in ethanol (2 mL) and stirred at 50 °C for 15 minutes. The mixture was cooled to room temperature and concentrated *in vacuo.* The residue was dissolved in ethyl acetate, washed with water, and brine, dried over sodium sulfate, filtered and concentrated *in vacuo* to afford the title compound (10 mg).

### 2-Chloro-5-(2-methyl-thiazol-4-yl)-benzoic acid

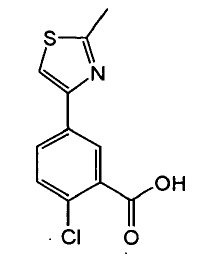

A mixture of 2-chloro-5-(2-methyl-thiazol-4-yl)-benzoic acid methyl ester (10 mg, 0.04 mmol) and sodium hydroxide (80 µL, 1M aqueous, 0.08 mmol) in *tert-*butanol (1 mL) was stirred at room temperature for 3d. The mixture was concentrated to dryness *in vacuo,* dissolved in water (5 mL), acidified to pH6 (1M HCl) and extracted with ethyl acetate. The combined organic layers were washed with water, and brine, dried over sodium sulfate, filtered and concentrated *in vacuo* to afford the title compound (7 mg).

### 2-Chloro-N-(1-hydroxy-cycloheptylmethyl)-5-(2-methyl-thiazol-4-yl)-benzamide

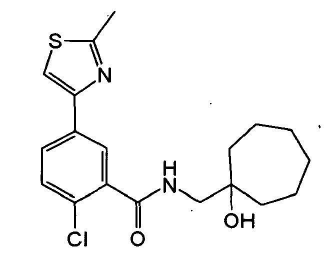

A mixture of 2-chloro-5-(2-methyl-thiazol-4-yl)-benzoic acid (7 mg, 0.027 mmol), 1-aminomethyl-cycloheptanol (6 mg, 0.03 mmol), 1-hydroxybenzotriazole (5 mg, 0.03 mmol), 1-(3-dimethylaminopropyl)-3-ethylcarbodiimide hydrochloride (6 mg, 0.03 mmol) and triethylamine (5 µL, 0.03 mmol) in *N,N*-dimethylformamide (0.5 mL) was stirred at room temperature for 16h. The mixture was diluted with ethyl acetate and washed with 5% citric acid, water, then brine. The organic layer was dried over sodium sulfate, filtered, and concentrated *in vacuo.* The residue was purified by column chromatography (1:1 ethyl acetate - hexanes) to afford the title compound (3.4 mg).

### EXAMPLE 60

Compounds of Examples 1-59 were tested for antagonist activity at the P2X₇ receptor using the cytokine IL-1β as the readout. Blood was collected from normal volunteers in the presence of heparin, and was fractionated using lymphocyte separation medium obtained from Organon Technica (Westchester, PA). The region of the resulting gradient containing banded mononuclear cells was harvested, diluted with 10 ml of Maintenance Medium (RPMI 1640, 5% FBS, 25 mM Hepes, pH 7.2, 1% penicillin/streptomycin), and the mononuclear cells were collected by centrifugation. The resulting cell pellet was suspended in 10 ml of Maintenance Medium and a cell count was performed. Approximately, 2 x 10⁵ mononuclear cells were seeded into each well of 96-well plates in a total volume of 0.1 ml. Monocytes were allowed to adhere for 2 hours, after which the supernatants were discarded and the attached cells were rinsed twice and then incubated in Maintenance Medium overnight at 37°C in a 5% CO₂ environment.

The cultured monocytes were activated with 10 ng/ml LPS (E. coli serotype 055:B5; Sigma Chemicals, St. Louis, MO). Following a 2-hour incubation, the activation medium was removed, the cells were rinsed twice with 0.1 ml of Chase Medium (RPMI 1640, 1% FBS, 20 mM Hepes, 5 mM NaHCO₃, pH 6.9), and then 0.1 ml of Chase Medium containing a compound of Example 1-59 was added and the plate was incubated for 30 minutes; each compound of Example 1-59 was evaluated in triplicate wells. ATP then was introduced (from a 100 mM stock solution, pH 7) to achieve a final concentration of 2 mM and the plate was incubated at 37°C for an additional 3 hours. Media were harvested and clarified by centrifugation, and their IL-1β content was determined by ELISA (R&D Systems; Minneapolis, MN). The ability of a compound of Example 1-59 to inhibit 50% of the stimulation of the release of IL-1β from monocytes by ATP (i.e., the "IC₅₀") is reported in Table 1:

**Table 1**

| Example | IC₅₀ (µM) | Example | IC₅₀ (µM) |
|---|---|---|---|
| 1 | 0.029 | 31 | 0.07 |
| 2 | 0.011 | 32 | 0.038 |
| 3 | 0.117 | 33 | 0.011 |
| 4 | 0.28 | 34 | 0.193 |
| 5 | 0.93 | 35 | 0.121 |
| 6 | 1.4 | 36 | 0.051 |
| 7 | 1.66 | 37 | 0.018 |
| 8 | 0.525 | 38 | 0.245 |
| 9 | 1 | 39 | 0.008 |
| 10 | 0.8 | 40 | 0.008 |
| 11 | 0.00867 | 41 | 0.030 |
| 12 | 0.24 | 42 | 0.008 |
| 13 | 0.008 | 43 | 0.13 |
| 14 | 0.081 | 44 | 0.0025 |
| 15 | 0.023 | 45 | 0.006 |
| 16 | 0.37 | 46 | 0.022 |
| 17 | 0.018 | 47 | 0.0035 |
| 18 | 0.545 | 48 | 0.0105 |
| 19 | 0.18 | 49 | 0.005 |
| 20 | 1 | 50 | 0.005 |
| 21 | 0.046 | 51 | 0.005 |
| 22 | 0.05 | 52 | 0.008 |
| 23 | 0.2 | 53 | 0.0155 |
| 24 | 1 | 54 | 0.005 |
| 25 | 0.004 | 55 | 0.365 |
| 26 | 0.373 | 56 | 0.48 |
| 27 | 0.031 | 57 | >1 |
| 28 | 1 | 58 | 0.43 |
| 29 | 0.242 | 59 | 0.28 |
| 30 | 1 | | |

## Claims

1. A compound of general formula I 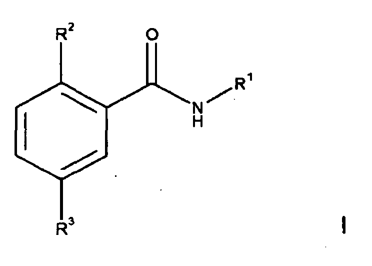 wherein R¹ is a (C₁-C₆)alkyl optionally substituted with one or two radicals independently selected from hydroxy, halo, -CN, (C₁-C₆)alkyl, HO(C₁-C₆)alkyl, NH₂(C=O)-, (C₁-C₆)alkyl-NH-(C=O)-, [(C₁-C₆)alkyl]₂N-(C=O)-, oxo and (C₁-C₆)alkoxy;
wherein said R¹ (C₁-C₆)alkyl may also optionally be substituted: with one or two groups independently selected from (C₁-C₆)alkyl, (C₃-C₁₀)cycloalkyl, (C₆-C₁₀)aryl and (C₁-C₁₀)heteroaryl; wherein said (C₃-C₁₀)cycloalkyl group is other than adamantyl; wherein said (C₁-C₆)alkyl and (C₃-C₁₀)cycloalkyl may be optionally substituted with oxo; wherein said (C₁-C₁₀)heteroaryl is selected from furanyl, thiophenyl, benzthiophenyl, benzfuranyl and chromanyl; wherein each of said (C₁-C₆)alkyl, (C₃-C₁₀)cycloalkyl, (C₆-C₁₀)aryl and (C₁-C₁₀)heteroaryl groups may also optionally be substituted by one to three radicals independently selected from hydroxy, halo, -CN, (C₁-C₆)alkyl, HO(C₁-C₆)alkyl, NH₂(C=O)-, (C₁-C₆)alkyl-NH(C=O)-, [(C₁-C₆)alkyl]₂N-(C=O)-, (C₁-C₆)alkoxy, (C₆-C₁₀)aryl and (C₃-C₁₀)cycloalkyl; wherein said (C₃-C₁₀)cycloalkyl and (C₆-C₁₀)aryl radicals are optionally substituted by one to three moieties independently selected from halo, (C₁-C₆)alkyl, -CN and (C₁-C₆)alkoxy;
R² is halo, -CN or (C₁-C₆)alkyl; wherein said (C₁-C₆)alkyl is optionally substituted by one to three radicals independently selected from the group consisting of: halo, hydroxy, amino, -CN, (C₁-C₆)alkyl, (C₁-C₆)alkoxy, -CF₃, CF₃O-, NH₂, (C₁-C₆)alkyl-NH-, [(C₁-C₆)alkyl]₂-N-, (C₁-C₆)alkyl-S-, (C₁-C₆)alkyl-(S=O)-, (C₁-C₆)alkyl-(SO₂)-, (C₁-C₆)alkyl-O-(C=O)-, formyl, (C₁-C₆)alkyl-(C=O)-, and (C₃-C₆)cycloalkyl;
R³ is an optionally substituted carbon linked (C₁-C₁₀)heteroaryl or (C₁-C₁₀)heterocyclyl; wherein said optional substituents can be on any carbon atom of said (C₁-C₁₀)heteroaryl or (C₁-C₁₀)heterocyclyle capable of substitution with one to three R⁴ per ring; wherein said optional substituents can be on any nitrogen atom of said (C₁-C₁₀)heteroaryl or (C₁-C₁₀)heterocyclyl capable of substitution with one to two R⁵ per ring;
wherein each R⁴ is independently selected from the group consisting of: halo, -CN, NH₂, hydroxy, H₂N(C=O)-, H₂N-SO₂-, and optionally substituted R⁶ substituents;
wherein said optionally substituted R⁶ substituents are selected from the group consisting of: (C₁-C₆)alkyl, (C₃-C₁₀)cycloalkyl; (C₁-C₁₀)heterocyclyl, (C₆-C₁₀)aryl, (C₁-C₁₀)heteroaryl, (C₁-C₆)alkoxy, (C₁-C₆)alkyl-(C=O)O-, (C₁-C₆)alkyl-NH-, [(C₁-C₆)alkyl]₂-N-, (C₆-C₁₀)aryl-NH-, (C₁-C₆)alkyl-(C=O)NH-, (C₁-C₆)alkyl-SO₂-NH-, (C₁-C₆)alkyl-(C=O)-, (C₁-C₆)alkyl-NH-(C=O)-, [(C₁-C₆)alkyl]₂-N-(C=O)-, (C₁-C₆)alkyl-O(C=O)-, (C₃-C₁₀)cycloalkyl-(C=O)-, (C₁-C₁₀)heterocyclyl-(C=O)-, (C₆-C₁₀)aryl-(C=O)-, (C₁-C₁₀)heteroaryl-(C=O)-, (C₁-C₆)alkyl-SO₂-, (C₆-C₁₀)alkyl-SO₂-, (C₁-C₁₀)heteroaryl-SO₂-, (C₁-C₆)alkyl-NH-SO₂-, and [(C₁-C₆)alkyl]₂N-SO₂-;
wherein any two R⁴ radicals on a carbon atom of said (C₁-C₁₀)heterocycyl can be taken together to form an oxo group or a spiro (C₃-C₆)carbocyclic or (C₁-C₆)heterocyclic group:
wherein each R⁵ is independently selected from the group consisting of: H₂N(C=O)-, and the following optionally substituted R⁸ groups: (C₁-C₆)alkyl, (C₁-C₁₀)heterocyclyl, (C₁-C₁₀)heteroaryl, (C₁-C₆)alkyl-(C=O)-, (C₁-C₆)alkyl-NH-(C=O)-, [(C₁-C₆)alkyl]₂-N-(C=O)-, (C₁-C₆)alkyl-O(C=O)-, (C₃-C₁₀)cycloalkyl-(C=O)-, (C₁-C₁₀)heterocyclyl-(C=O)-, (C₆-C₁₀)aryl-(C=O)-, (C₁-C₁₀)heteroaryl-(C=O)-, and (C₁-C₁₀)alkyl-SO₂-;
wherein each of said optionally substituted R⁶ substituents may be substituted with one to three groups independently selected from the group consisting of: halo, NH₂, -CN, hydroxy, (C₁-C₆)alkyl-SO₂-, H₂N-SO₂-, (C₁-C₆)alkyl-NH-SO₂-, and [(C₁-C₆)alkyl]₂N-SO₂-, (C₁-C₆)alkoxy, (C₁-C₆)alkyl]-SO₂-NH-, (C₁-C₆)alkyl-(C=O)-, (C₁-C₆)alkyl-(C=O)O-, H₂N(C=O)-, (C₁-C₆)alkyl-NH-(C=O)-, [(C₁-C₆)alkyl]₂-N-(C=O)-, (C₁-C₆)alkyl-O(C=O)-, and optionally substituted R⁷ groups;
wherein each of said optionally substituted R⁷ groups are independently selected from the group consisting of:
(C₃-C₁₀)cycloalkyl, (C₁-C₁₀)heterocyclyl, (C₆-C₁₀)aryl, (C₁-C₁₀)heteroaryl, phenoxy, (C₁-C₆)alkyl-NH-, [(C₁-C₆)alkyl]₂-N-, (C₆-C₁₀)aryl-NH-, (C₃-C₁₀)cycloalkyl-(C=O)-, (C₁-C₁₀)heterocyclyl-(C=O)-, (C₆-C₁₀)aryl-(C=O)-, (C₁-C₁₀)heteroaryl-(C=O)-, (C₆-C₁₀)aryl-SO₂-, and (C₁-C₁₀)heteroaryl-SO₂-;
wherein each of said optionally substituted R⁷ groups may be
optionally substituted with one to three substituents independently selected from the group consisting of: halo, CF₃, -CN, (C₁-C₆)alkyl, (C₁-C₁₀)heterocyclyl, (C₁-C₁₀)heteroaryl, hydroxy, (C₁-C₆)alkoxy, (C₁-C₆)alkyl-O(C=O)-, (C₁-C₆)alkyl-SO₂-NH-, (C₁-C₆)alkyl-SO₂-, (C₆-C₁₀)aryl-SO₂-, (C₁-C₁₀)heteroaryl-SO₂-, H₂N-SO₂-, (C₁-C₆)alkyl-NH-SO₂-, and [(C₁-C₆)alkyll₂N-SO₂-;
wherein each of said optionally substituted R⁸ groups may be substituted with one to three substituents may be independently selected from the group consisting of: halo, -CN, hydroxy, (C₁-C₆)alkoxy, (C₁-C₆)alkyl-(C=O)O-, H₂N-(C=O)O-, (C₁-C₆)alkyl-NH-(C=O)O-, ((C₁-C₆)alkyl)₂N-(C=O)O-, NH (C₁-C₆)alkyl-NH-, [(C₁-C₆)alkyl]₂-N-, (C₁-C₆)alkyl)-(C=O)NH-, (C₁-C₆)alkyl)-NH-(C=O)NH-, (C₁-C₆)alkyl-SO2-NH-,(C₁-C₆)alkyl-(C=O)NH-, (C₁-C₆)alkyl-(C=O)-, H₂N(C=O)-, (C₁-C₆)alkyl-NH-(C=O)-, [(C₁-C₆)alkyl]₂-N-(C=O)-, (C₁-C₆)alkyl-O(C=O)-, (C₁-C₆)alkyl-SO₂-, H₂N-SO₂-, (C₁-C₆)alkyl-NH-SO₂-, and [(C₁-C₆)alkyl]₂N-SO₂- and optionally substitued R⁹ groups;
wherein each of said optionally substituted R⁹ groups are independently selected from the group consisting of: (C₆-C₁₀)aryl, (C₃-C₁₀)cycloalkyl-NH(C=O)-, (C₁-C₁₀)heterocyclyl-NH-(C=O)-, (C₆-C₁₀)aryl-NH-(C=O)-, (C₁-C₁₀)heteroaryl-NH-(C=O)-, (C₃-C₁₀)cycloalkyl-(C=O)-, (C₁-C₁₀)heterocyclyl-(C=O)-, (C₆-C₁₀)aryl-(C=O)-, (C₁-C₁₀)heteroaryl-(C=O), (C₆-C₁₀)aryl-SO₂-, (C₁-C₁₀)heteroaryl-SO₂-;
wherein each of said optionally substituted R⁹ groups may be optionally substituted with one to three substituents independently selected from the group consisting of: halo, (C₁-C₆)alkyl, -CN, hydroxy, (C₁-C₆)alkoxy, (C₁-C₆)alkyl-(C=O)O-, NH₂, (C₁-C₆)alkyl-NH-, [(C₁-C₆)alkyl]₂-N-, ((C₁-C₆)alkyl)-(C=O)NH-, (C₁-C₆)alkyl-(C=O)-, H₂N(C=O)-, (C₁-C₆)alkyl-NH-(C=O)- and [(C₁-C₆)alkyl]₂-N-(C=O)-;
wherein the molecular weight of said compound of formula I is less than 700 AMU;
selected from the group consisting of:
(3-{4-Chloro-3-[(1-hydroxy-cycloheptylmethyl)-carbamoyl]-phenyl}-5-methyl-pyrazol-1-yl)-acetic acid methyl ester;
2-Chloro-5-(1-cyanomethyl-1H-pyrazol-3-yl)-N-(1-hydroxycycloheptylmethyl)-benzamide;
2-Chloro-5-(1-cyanomethyl-5-methyl-1H-pyrazol-3-yl)-N-(1-hydroxycycloheptylmethyl)-benzamide;
2-Chloro-5-(5-cyclopropyl-1H-pyrazol-3-yl)-N-(1-hydroxycycloheptylmethyl)-benzamide;
2-Chloro-5-(6-methyl-pyridin-3-yl)-N-(1-p-tolyl-cyclohexylmethyl)-benzamide;
2-Chloro-5-[1-(2,3-dihydroxy-2-methyl-propyl)-5-methyl-1H-pyrazol-3-yl]-N-(1-hydroxy-cycloheptylmethyl)-benzamide;
2-Chloro-5-[1-(3-fluoro-2-hydroxy-propyl)-1H-imidazol-4-yl]-N-(1-hydroxy-cycloheptylmethyl)-benzami de;
2-Chloro-5-[2-(2,3-dihydroxy-propyl)-5-methyl-2H-pyrazol-3-yl]-N-(1-hydroxy-cycloheptylmethyl)-benzamide;
2-Chloro-N-(1-cyano-cycloheptylmethyl)-5-(5-methyl-2H-pyrazol-3-yl)-benzamide;
2-Chloro-N-(1-hydroxy-3,3-dimethyl-cyclohexylmethyl)-5-(3-methylisoxazol-5-yl)-benzamide;
2-Chloro-N-(1-hydroxy-3,3-dimethyl-cyclohexylmethyl)-5-(5-methyl-2H-pyrazol-3-yl)-benzamide;
2-Chloro-N-(1-hydroxy-3, 3-dimethyl-cyclohexylmethyl)-5-(5-methylpyridin-2-yl)-benzamide;
2-Chloro-N-(1-hydroxy-3,3-dimethyl-cyclohexylmethyl)-5-(5-trifluoromethyl-1H-pyrazol-3-yl)-benzamide;
2-Chloro-N-(1-hydroxy-cycloheptylmethyl)-5-(1H-imidazol-4-yl)-benzamide;
2-Chloro-N-(1-hydroxy-cycloheptylmethyl)-5-(1H-pyrazol-3-yl)-benzamide;
2-Chloro-N-(1-hydroxy-cycloheptylmethyl)-5-(1-methyl-1H-pyrazol-3-yl)-benzamide;
2-Chloro-N-(1-hydroxy-cycloheptylmethyl)-5-(2H-[1,2,3]triazol-4-yl)-benzamide;
2-Chloro-N-(1-hydroxy-cycloheptylmethyl)-5-(2-methyl-2H-pyrazol-3-yl)-benzamide; .
2-Chloro-N-(1-hydroxy-cycloheptylmethyl)-5-(2-methyl-thiazol-4-yl)-benzamide;
2-Chloro-N-(1-hydroxy-cycloheptylmethyl)-5-(3-methyl-isoxazol-5-yl)-benzamide;
2-Chloro-N-(1-hydroxy-cycloheptylmethyl)-5-(5 -methyl-1-oxiranylmethyl-1H-pyrazol-3-yl)-benzamide;
2-Chloro-N-(1-hydroxy-cycloheptylmethyl)-5-(5-methyl-pyridin-2-yl)-benzamide;
2-Chloro-N-(1-hydroxy-cycloheptylmethyl)-5-(5-trifluoromethyl-1H-pyrazol-3-yl)-benzamide;
2-Chloro-N-(1-hydroxy-cycloheptylmethyl)-5-(6-methoxy-pyridazin-3-yl)-benzamide;
2-Chloro-N-(1-hydroxy-cycloheptylmethyl)-5-(hydroxyimino-methyl)-benzamide;
2-Chloro-N-(1-hydroxy-cycloheptylmethyl)-5-[1-(2-hydroxy-2-methylpropyl)-1H-pyrazol-3-yl]-benzamide;
2-Chloro-N-(1-hydroxy-cycloheptylmethyl)-5-[1-(2-hydroxy-2-methylpropyl)-5-methyl-1H-pyrazol-3-yl]-benzamide;
2-Chloro-N-(1-hydroxy-cycloheptylmethyl)-5-[1-(2-hydroxy-ethyl)-1H-pyrazol-3-yl]-benzamide;
2-Chloro-N-(1-hydroxy-cycloheptylmethyl)-5-[1-(2-hydroxy-ethyl)-6-oxo-1,6-dihydro-pyridazin-3-yl]-benzamide;
2-Chloro-N-(1-hydroxy-cycloheptylmethyl)-5-[2-(2-hydroxy-ethyl)-2H-pyrazol-3-yl]-benzamide;
2-Chloro-N-(1-hydroxy-cycloheptylmethyl)-5-[2-(2-hydroxy-ethyl)-5-methyl-2H-pyrazol-3-yl]-benzamide;
2-Chloro-N-(1-hydroxy-cycloheptylmethyl)-5-[5-(2-hydroxy-ethyl)-isoxazol-3-yl]-benzamide;
2-Chloro-N-(1-hydroxy-cycloheptylmethyl)-5-[5-methyl-1-(2-oxa-2-pyrrolidin-1-yl-ethyl)-1H-pyrazol-3-yl]-benzamide;
2-Chloro-N-(1-hydroxy-cycloheptylmethyl)-5-[5-methyl-1-(pyrimidin-2-ylcarbamoylmethyl)-1H-pyrazol-3-yl]-benzamide;
2-Chloro-N-(1-hydroxy-cycloheptylmethyl)-5-{1-[(1-hydroxymethylpropylcarbamoyl)-methyl]-5-methyl-1H-pyrazol-3-yl}-benzamide;
2-Chloro-N-(1-hydroxy-cycloheptylmethyl)-5-{1-[(2-hydroxypropylcarbamoyl)-methyl]-5-methyl-1H-pyrazol-3-yl}-benzamide;
2-Chloro-N-[2-(2-chloro-phenyl)-ethyl]-5-(1H-pyrazol-3-yl)-benzamide;
2-Chloro-N-[2-(2-chloro-phenyl)-ethyl]-5-(1H-pyrazol-4-yl)-benzamide;
2-Chloro-N-[2-(2-chloro-phenyl)-ethyl]-5-(2-methyl-pyrimidin-4-yl)-benzamide;
2-Chloro-N-[2-(2-chloro-phenyl)-ethyl]-5-(5-ethyl-2H-pyrazol-3-yl)-benzamide;
2-Chloro-N-[2-(2-chloro-phenyl)-ethyl]-5-(6-methoxy-pyridazine-3-yl)-benzamide;
2-Chloro-N-[2-(2-chloro-phenyl)-ethyl]-5-[1-(4-methoxy-benzyl)-1H-pyrazol-4-yl]-benzamide;
5-(1-Carbamoylmethyl-1H-pyrazol-3-yl)-2-chloro-N-(1-hydroxycycloheptylmethyl) benzamide;
5-(1-Carbamoylmethyl-5-methyl-1H-pyrazol-3-yl)-2-chlore-N-(1-hydroxy-cycloheptylmethyl)-benzamide;
5-[1-(3-Amino-2-hydroxy-propyl)-5-methyl-1H-pyrazol-3-yl]-2-chloro-N-(1-hydroxy-cycloheptylmethyl)-benzamide Hydrochloride; and
5-[2-(3-Amino-2-hydroxy-propyl)-5-methyl-2H-pyrazol-3-yl]-2-chloro-N-(1-hydroxy-cycloheptylmethyl)-benzamide;
or a pharmaceutically acceptable salt thereof

2. A pharmaceutical composition comprising a therapeutically effective amount of a compound according to claim 1, or a pharmaceutically acceptable salt thereof, and a pharmaceutically acceptable carrier or diluent.

3. A compound according to claim 1, or a pharmaceutically acceptable salt thereof, for use in the treatment of rheumatoid arthritis.

4. A compound or pharmaceutically acceptable salt according to claim 1 for use in combination with a further pharmaceutically active agent.

## Patentansprüche

1. Verbindung der allgemeinen Formel I 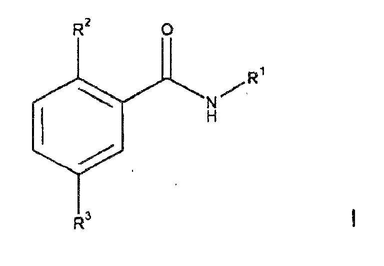 worin R¹ ein (C₁-C₆)Alkyl ist, das optional substituiert ist mit einem oder zwei Resten, die unabhängig ausgewählt sind aus Hydroxy, Halogen, -CN, (C₁-C₆)Alkyl, HO (C₁-C₆) Alkyl, NH₂ (C=O) -, (C₁-C₆) Alkyl-NH- (C=O)-, [(C₁-C₆)Alkyl]₂N-(C=O)-, Oxo und (C₁-C₆)Alkoxy;
wobei das R¹-(C₁-C₆)Alkyl auch optional substituiert sein kann mit einer oder zwei Gruppen, die unabhängig ausgewählt sind aus (C₁-C₆)Alkyl, (C₃-C₁₀) Cycloalkyl, (C₆-C₁₀)Aryl und (C₁-C₁₀)Heteroaryl; wobei die (C₃-C₁₀)Cycloalkyl-Gruppe von Adamantyl verschieden ist; wobei das (C₁-C₆)Alkyl und das (C₃-C₁₀)Cycloalkyl optional substituiert sein können mit Oxo; wobei das (C₁-C₁₀)Heteroaryl ausgewählt ist aus Furanyl, Thiophenyl, Benzothiophenyl, Benzfuranyl und Chromanyl; wobei jede der (C₁-C₆)Alkyl-, (C₃-C₁₀) Cycloalkyl-, (C₆-C₁₀)Aryl- und (C₁-C₁₀)Heteroaryl-Gruppen auch optional substituiert sein kann mit eins bis drei Resten, die unabhängig ausgewählt sind aus Hydroxy, Halogen, -CN, (C₁-C₆)Alkyl, HO(C₁-C₆)Alkyl, NH₂(C=O)-, (C₁-C₆) Alkyl-NH- (C=O) -, [(C₁-C₆)Alkyl]₂N- (C=O) -, (C₁-C₆)Alkoxy, (C₆-C₁₀)Aryl und (C₃-C₁₀)Cycloalkyl; wobei die (C₃-C₁₀)Cycloalkyl- und (C₆-C₁₀)Aryl-Reste optional substituiert sind mit eins bis drei Gruppierungen, die unabhängig ausgewählt sind aus Halo, (C₁-C₆)Alkyl, -CN und (C₁-C₆)Alkoxy;
R² Halogen, -CN oder (C₁-C₆)Alkyl ist; wobei das (C₁-C₆)Alkyl optional substituiert ist durch einen bis drei Rest(e), die unabhängig ausgewählt sind aus der Gruppe, bestehend aus: Halogen, Hydroxy, Amino, - CN, (C₁-C₆)Alkyl, (C₁-C₆)Alkoxy, -CF₃, CF₃O-, NH₂, (C₁-C₆)Alkyl-NH-, [(C₁-C₆)Alkyl]₂-N-, (C₁-C₆)Alkyl-S-, (C₁-C₆)Alkyl-(S=O)-, (C₁-C₆)Alkyl-(SO₂)-, (C₁-C₆)Alkyl-O-(C=O)-, Formyl, (C₁-C₆)Alkyl-(C=O)- und (C₃-C₆) Cycloalkyl;
R³ ein optional substituiertes, Kohlenstoff-gebundenes (C₁-C₁₀)Heteroaryl oder (C₁-C₁₀)Heterocyclyl ist; wobei sich die optionalen Substituenten an einem beliebigen Kohlenstoffatom des (C₁-C₁₀)Heteroaryls oder (C₁-C₁₀)Heterocyclyls befinden können, die zur Substitution befähigt sind mit eins bis drei R⁴ pro Ring; wobei die optionalen Substituenten sich befinden können an einem beliebigen Stickstoffatom des (C₁-C₁₀)Heteroaryls oder (C₁-C₁₀)Heterocyclyls, das zur Substitution befähigt ist mit eins bis zwei R⁵ pro Ring;
wobei jedes R⁴ unabhängig ausgewählt ist aus der Gruppe, bestehend aus: Halogen, -CN, NH₂, Hydroxy, H₂N(C=O)-, H₂N-SO₂- und optional substituierten R⁶-Substituenten;
wobei die optional substituierten R⁶-Substituenten ausgewählt sind aus der Gruppe, bestehend aus: (C₁-C₆)Alkyl, (C₃-C₁₀)Cycloalkyl, (C₁-C₁₀)Heterocyclyl, (C₆-C₁₀)Aryl, (C₁-C₁₀)Heteroaryl, (C₁-C₆)Alkoxy, (C₁-C₆)Alkyl-(C=O)O-, (C₁-C₆)Alkyl-NH-, [(C₁-C₆)Alkyl]₂-N-, (C₆-C₁₀)Aryl-NH-, (C₁-C₆)Alkyl-(C=O)NH-, (C₁-C₆)Alkyl-SO₂-NH-, (C₁-C₆)Alkyl-(C=O)-, (C₁-C₆)Alkyl-NH-(C=O)-, (C₁-C₆)Alkyl]₂-N-(C=O)-,(C₁-C₆)Alkyl-O(C=O)-, (C₃-C₁₀)Cycloalkyl-(C=O)-, (C₁-C₁₀)Heterocyclyl-(C=O)-, (C₆-C₁₀)Aryl-(C=O)-, (C₁-C₁₀)Heteroaryl-(C=O)-, (C₁-C₆)Alkyl-SO₂-, (C₆-C₁₀) Aryl-SO₂-, (C₁-C₁₀)Heteroaryl-SO₂-, (C₁-C₆)Alkyl-NH-SO₂- und [(C₁-C₆)Alkyl]₂N-SO₂-;
wobei zwei beliebige R⁴-Reste an einem Kohlenstoffatom des (C₁-C₁₀)Heterocyclyls zusammengenommen werden können, um eine Oxo-Gruppe oder eine Spiro- (C₃-C₆) carbocyclische oder (C₁-C₆)heterozyklische Gruppe zu bilden;
wobei jedes R⁵ unabhängig ausgewählt ist aus der Gruppe, bestehend aus: H₂N(C=O)- und den folgenden optional substituierten R⁸-Gruppen: (C₁-C₆)Alkyl, (C₁-C₁₀) Heterocyclyl, (C₁-C₁₀) Heteroaryl, (C₁-C₆)Alkyl-(C=O)-, (C₁-C₆)Alkyl-NH-(C=O)-, [(C₁-C₆)Alkyl]₂-N-(C=O)-, (C₁-C₆)Alkyl-O(C=O)-, (C₃-C₁₀)Cycloalkyl-(C=O)-, (C₁-C₁₀)Heterocyclyl-(C=O)-, (C₆-C₁₀)Aryl-(C=O)-, (C₁-C₁₀)Heteroaryl-(C=O)- und (C₁-C₆)Alkyl-SO₂-;
wobei jeder der optional substituierten R⁶-Substituenten substituiert sein kann mit eins bis drei Gruppen, die unabhängig ausgewählt sind aus der Gruppe, bestehend aus: Halogen, NH₂, -CN, Hydroxy, (C₁-C₆)Alkyl-SO₂-, H₂N-SO₂-, (C₁-C₆)Alkyl-NH-SO₂- und [(C₁-C₆)Alkyl]₂N-SO₂-, (C₁-C₆)Alkoxy, (C₁-C₆)Alkyl-SO₂-NH-, (C₁-C₆)Alkyl-(C=O) -, (C₁-C₆)Alkyl-(C=O)O-, H₂N(C=O)-, (C₁-C₆)Alkyl-NH-(C=O)-, [(C₁-C₆)Alkyl]₂-N-(C=O-, (C₁-C₆) Alkyl-O(C=O)- und optional substituierten R⁷-Gruppen;
wobei jede der optional substituierten R⁷-Gruppen unabhängig ausgewählt ist aus der Gruppe, bestehend aus: (C₃-C₁₀) Cycloalkyl, (C₁-C₁₀) Heterocyclyl, (C₆-C₁₀)Aryl, (C₁-C₁₀) Heteroaryl, Phenoxy, (C₁-C₆)Alkyl-NH-, [(C₁-C₆)Alkyl]₂-N-, (C₆-C₁₀)Aryl-NH-, (C₃-C₁₀)Cycloalkyl-(C=O)-, (C₁-C₁₀) Heterocyclyl-(C=O)-, (C₆-C₁₀)Aryl-(C=O)-, (C₁-C₁₀)Heteroaryl-(C=O)-, (C₆-C₁₀)Aryl-SO₂- und (C₁-C₁₀)Heteroaryl-SO₂-;
wobei jeder der optional substituierten R⁷-Gruppen optional substituiert sein kann mit eins bis drei Substituenten, die unabhängig ausgewählt sind aus der Gruppe, bestehend aus: Halogen, CF₃, -CN, (C₁-C₆)Alkyl, (C₁-C₁₀)Heterocyclyl, (C₁-C₁₀)Heteroaryl, Hydroxy, (C₁-C₆)Alkoxy, (C₁-C₆)Alkyl-O(C=O)-, (C₁-C₆)Alkyl-SO₂-NH-, (C₁-C₆)Alkyl-SO₂-, (C₆-C₁₀)Aryl-SO₂-, (C₁-C₁₀)Heteroaryl-SO₂-, H₂N-SO₂-, (C₁-C₆)Alkyl-NH-SO₂- und [(C₁-C₆)Alkyl]₂N-SO₂-;
wobei jede der optional substituierten R⁸-Gruppen substituiert sein kann mit eins bis drei Substituenten, die unabhängig ausgewählt sein können aus der Gruppe, bestehend aus: Halogen, -CN, Hydroxy, (C₁-C₆)Alkoxy, (C₁-C₆)Alkyl-(C=O)O-, H₂N-(C=O)O-, (C₁-C₆)Alkyl-NH-(C=O)O-, ((C₁-C₆)Alkyl)₂N-(C=O)O-, NH₂, (C₁-C₆)Alkyl-NH-, [(C₁-C₆)Alkyl]₂-N-, ((C₁-C₆)Alkyl)-(C=O)NH-, ((C₁-C₆)Alkyl)-NH-(C=O)NH-, (C₁-C₆)Alkyl-SO₂-NH-, (C₁-C₆)Alkyl-(C=O)NH-, (C₁-C₆)Alkyl-(C=O)-, H₂N(C=O)-, (C₁-C₆) Alkyl-NH-(C=O)-, [(C₁-C₆)Alkyl]₂-N-(C=O)-, (C₁-C₆)Alkyl-O(C=O)-, (C₁-C₆)Alkyl-SO₂-, H₂N-SO₂-, (C₁-C₆)Alkyl-NH-SO₂- und [(C₁-C₆)Alkyl]₂N-SO₂- und optional substituierten R⁹-Gruppen;
wobei jede der optional substituierten R⁹-Gruppen unabhängig ausgewählt ist aus der Gruppe, bestehend aus: (C₆-C₁₀)Aryl, (C₃-C₁₀)Cycloalkyl-NH(C=O)-, (C₁-C₁₀)Heterocyclyl-NH-(C=O) -, (C₆-C₁₀)Aryl-NH-(C=O)**-,** (C₁-C₁₀)Heteroaryl-NH-(C=O)-, (C₃-C₁₀)Cycloalkyl-(C=O)-, (C₁-C₁₀)Heterocyclyl-(C=O)-, (C₆-C₁₀)Aryl-(C=O) -, (C₁-C₁₀)Heteroaryl-(C=O)-, (C₆-C₁₀)Aryl-SO₂-, (C₁-C₁₀)Heteroaryl-SO₂-;
wobei jede der optional substituierten R⁹-Gruppen opoptional substituiert sein kann mit eins bis drei Substituenten, die unabhängig ausgewählt sind aus der Gruppe, bestehend aus: Halogen, (C₁-C₆)Alkyl, -CN, Hydroxy, (C₁-C₆)Alkoxy, (C₁-C₆)Alkyl-(C=O)O-, NH₂, (C₁-C₆)Alkyl-NH-, [(C₁-C₆)Alkyl]₂-N-, ((C₁-C₆)Alkyl)-(C=O)NH-, (C₁-C₆)Alkyl-(C=O)-, H₂N(C=O)-(C₁-C₆)Alkyl-NH-(C=O)- und [(C₁-C₆)Alkyl]₂-N-(C=0)-;
wobei das Molekulargewicht der Verbindung der Formel I kleiner als 700 AMU (Atomgewichtseinheiten) ist;
ausgewählt aus der Gruppe, bestehend aus:
(3-{4-Chlor-3-[(1-hydroxy-cycloheptylmethyl)-carbamoyl]-phenyl}-5-methyl-pyrazol-1-yl)-Essigsäuremethylester;
2-Chlor-5-(1-cyanomethyl-1H-pyrazol-3-yl)-N-(1-hydroxy-cycloheptylmethyl)-benzamid;
2-Chlor-5-(1-cyanomethyl-5-methyl-1H-pyrazol-3-yl)-N-(1-hydroxy-cycloheptylmethyl)-benzamid;
2-Chlor-5-(5-cyclopropyl-1H-pyrazol-3-yl)-N-(1-hydroxy-cycloheptylmethyl)-benzamid;
2-Chlor-5-(6-methyl-pyridin-3-yl)-N-(1-p-tolylcyclohexylmethyl)-benzamid;
2-Chlor-5-[1-(2,3-dihydroxy-2-methyl-propyl)-5-methyl-1H-pyrazol-3-yl]-N-(1-hydroxy-cycloheptylmethyl)-benzamid;
2-Chlor-5-[1-(3-fluor-2-hydroxy-propyl)-1H-imidazol-4-yl]-N-(1-hydroxy-cycloheptylmethyl)-benzamid;
2-Chlor-5-[2-(2,3-dihydroxy-propyl)-5-methyl-2H-pyrazol-3-yl]-N-(1-hydroxy-cycloheptylmethyl)-benzamid;
2-Chlor-N-(1-cyano-cycloheptylmethyl)-5-(5-methyl-2H-pyrazol-3-yl)-benzamid;
2-Chlor-N-(1-hydroxy-3,3-dimethyl-cyclohexylmethyl)-5-(3-methyl-isoxazol-5-yl)-benzamid;
2-Chlor-N-(1-hydroxy-3,3-dimethyl-cyclohexylmethyl)-5-(5-methyl-2H-pyrazol-3-yl)-benzamid;
2-Chlor-N-(1-hydroxy-3,3-dimethyl-cyclohexylmethyl)-5-(5-methyl-pyridin-2-yl)-benzamid;
2-Chlor-N-(1-hydroxy-3,3-dimethyl-cyclohexylmethyl)-5-(5-trifluormethyl-1H-pyrazol-3-yl)-benzamid;
2-Chlor-N-(1-hydroxy-cycloheptylmethyl)-5-(1H-imidazol-4-yl)-benzamid;
2-Chlor-N-(1-hydroxy-cycloheptylmethyl)-5-(1H-pyrazol-3-yl)-benzamid;
2-Chlor-N-(1-hydroxy-cycloheptylmethyl)-5-(1-methyl-1H-pyrazol-3-yl)-benzamid;
2-Chlor-N-(1-hydroxy-cycloheptylmethyl)-5-(2H-[1,2,3,]triazol-4-yl)-benzamid;
2-Chlor-N-(1-hydroxy-cycloheptylmethyl)-5-(2-methyl-2H-pyrazol-3-yl)-benzamid;
2-Chlor-N-(1-hydroxy-cycloheptylmethyl)-5-(2-methylthiazol-4-yl)-benzamid;
2-Chlor-N-(1-hydroxy-cycloheptylmethyl)-5-(3-methylisoxazol-5-yl)-benzamid;
2-Chlor-N-(1-hydroxy-cycloheptylmethyl)-5-(5-methyl-1-oxiranylmethyl-1H-pyrazol-3-yl)-benzamid;
2-Chlor-N-(1-hydroxy-cycloheptylmethyl)-5-(5-methylpyridin-2-yl)-benzamid;
2-Chlor-N-(1-hydroxy-cycloheptylmethyl)-5-(5-trifluormethyl-1H-pyrazol-3-yl)-benzamid;
2-Chlor-N-(1-hydroxy-cycloheptylmethyl)-5-(6-methoxypyridazin-3-yl)-benzamid;
2-Chlor-N-(1-hydroxy-cycloheptylmethyl)-5-(hydroxyimino-methyl)-benzamid;
2-Chlor-N-(1-hydroxy-cycloheptylmethyl)-5-[1-(2-hydroxy-2-methyl-propyl)-1H-pyrazol-3-yl]-benzamid;
2-Chlor-N-(1-hydroxy-cycloheptylmethyl)-5-[1-(2-hydroxy-2-methyl-propyl)-5-methyl-1H-pyrazol-3-yl]-benzamid;
2-Chlor-N-(1-hydroxy-cycloheptylmethyl)-5-[1-(2-hydroxy-ethyl)-1H-pyrazol-3-yl]-benzamid;
2-Chlor-N-(1-hydroxy-cycloheptylmethyl)-5-[1-(2-hydroxy-ethyl)-6-oxo-1,6-dihydro-pyridazin-3-yl]-benzamid;
2-Chlor-N-(1-hydroxy-cycloheptylmethyl)-5-[2-(2-hydroxy-ethyl)-2H-pyrazol-3-yl]-benzamid;
2-Chlor-N-(1-hydroxy-cycloheptylmethyl)-5-[2-(2-hydroxy-ethyl)-5-methyl-2H-pyrazol-3-yl]-benzamid;
2-Chlor-N-(1-hydroxy-cycloheptylmethyl)-5-[5-(2-hydroxy-ethyl)-isoxazol-3-yl]-benzamid;
2-Chlor-N-(1-hydroxy-cycloheptylmethyl)-5-[5-methyl-1-(2-oxo-2-pyrrolidin-1-yl-ethyl)-1H-pyrazol-3-yl]-benzamid;
2-Chlor-N-(1-hydroxy-cycloheptylmethyl)-5-[5-methyl-1-(pyrimidin-2-ylcarbamoylmethyl)-1H-pyrazol-3-yl]-benzamid;
2-Chlor-N-(1-hydroxy-cycloheptylmethyl)-5-{1-[(1-hydroxymethyl-propylcarbamoyl)-methyl]-5-methyl-1H-pyrazol-3-yl}-benzamid;
2-Chlor-N-(1-hydroxy-cycloheptylmethyl)-5-{1-[(2-hydroxy-propylcarbamoyl)-methyl]-5-methyl-1H-pyrazol-3-yl}-benzamid;
2-Chlor-N-[2-(2-chlor-phenyl)-ethyl]-5-(1H-pyrazol-3-yl)-benzamid;
2-Chlor-N-[2-(2-chlor-phenyl)-ethyl]-5-(1H-pyrazol-4-yl)-benzamid;
2-Chlor-N-[2-(2-chlor-phenyl)-ethyl]-5-(2-methylpyrimidin-4-yl)-benzamid;
2-Chlor-N-[2-(2-chlor-phenyl)-ethyl]-5-(5-ethyl-2H-pyrazol-3-yl)-benzamid;
2-Chlor-N-[2-(2-chlor-phenyl)-ethyl]-5-(6-methoxypyridazin-3-yl)-benzamid;
2-Chlor-N-[2-(2-chlor-phenyl)-ethyl]-5-[1-(4-methoxybenzyl)-1H-pyrazol-4-yl]-benzamid;
5-(1-Carbamoylmethyl-1H-pyrazol-3-yl)-2-chlor-N-(1-hydroxy-cycloheptylmethyl)-benzamid;
5-(1-Carbamoylmethyl-5-methyl-1H-pyrazol-3-yl)-2-chlor-N-(1-hydroxy-cycloheptylmethyl)-benzamid;
5-[1-(3-Amino-2-hydroxy-propyl)-5-methyl-1H-pyrazol-3-yl]-2-chlor-N-(1-hydroxy-cycloheptylmethyl)-benzamid-Hydrochlorid und
5-[2-(3-Amino-2-hydroxy-propyl)-5-methyl-2H-pyrazol-3-yl]-2-chlor-N-(1-hydroxy-cycloheptylmethyl)-benzamid;
oder ein pharmazeutisch annehmbares Salz davon.

2. Pharmazeutische Zusammensetzung, umfassend eine therapeutisch wirksame Menge einer Verbindung gemäß Anspruch 1, oder ein pharmazeutisch annehmbares Salz davon und einen pharmazeutisch annehmbaren Träger oder ein pharmazeutisch annehmbares Verdünnungsmittel.

3. Verbindung gemäß Anspruch 1, oder ein pharmazeutisch annehmbares Salz davon zur Verwendung bei der Behandlung von rheumatoider Arthritis.

4. Verbindung oder pharmazeutisch annehmbares Salz gemäß Anspruch 1 zur Verwendung in Kombination mit einem weiteren pharmazeutischen Wirkstoff.

## Revendications

1. Composé de formule générale I 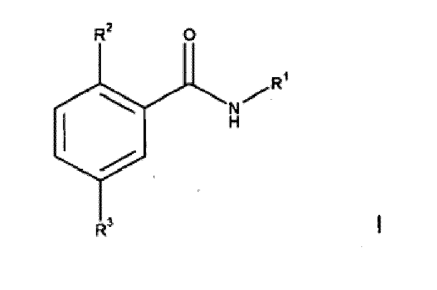 où R¹ est un groupe alkyle en C₁-C₆ éventuellement substitué par un ou deux radicaux choisis indépendamment parmi hydroxy, halo, -CN, alkyle en C₁-C₆, HO (alkyle en C₁-C₆), NH₂(C=O)-, alkyle en C₁-C₆-NH-(C=O)-, (alkyle en C₁-C₆)₂N-(C=O)-, oxo et alcoxy en C₁-C₆ ;
ledit groupe alkyle en C₁-C₆ R¹ pouvant également être éventuellement substitué par un ou deux groupes choisis indépendamment parmi alkyle en C₁-C₆, cycloalkyle en C₃-C₁₀, aryle en C₆-C₁₀ et hétéroaryle en C₁-C₁₀ ; ledit cycloalkyle en C₃-C₁₀ étant autre qu'adamantyle ; ledit alkyle en C₁-C₆ et ledit cycloalkyle en C₃-C₁₀ pouvant être éventuellement substitués par oxo ; ledit hétéroaryle en C₁-C₁₀ étant choisi parmi furanyle, thiophényle, benzthiophényl, benzfuranyle et chromanyle ; desdits groupes alkyle en C₁-C₆, cycloalkyle en C₃-C₁₀, aryle en C₆-C₁₀ et hétéroaryle en C₁-C₁₀ pouvant également être éventuellement substitué par un à trois radicaux choisis indépendamment parmi hydroxy, halo, -CN, alkyle en C₁-C₆, HO (alkyle en C₁-C₆), NH₂(C=O)-, alkyle en C₁-C₆-NH(C=O)-, (alkyle en C₁-C₆)₂N-(C=O)-, alcoxy en C₁-C₆, aryle en C₆-C₁₀ et cycloalkyle en C₃-C₁₀ ; lesdits radicaux cycloalkyle en C₃-C₁₀ et aryle en C₆-C₁₀ étant éventuellement substitués par un à trois fragments choisis indépendamment parmi halo, alkyle en C₁-C₆, -CN et alcoxy en C₁-C₆ ;
R² est un groupe halo, -CN or alkyle en C₁-C₆ ; ledit alkyle en C₁-C₆ étant éventuellement substitué par un à trois radicaux choisis indépendamment dans le groupe constitué par : halo, hydroxy, amino, -CN, alkyle en C₁-C₆, alcoxy en C₁-C₆, -CF₃, CF₃O-, NH₂, alkyle en C₁-C₆-NH-, (alkyle en C₁-C₆)₂-N-, alkyle en C₁-C₆-S-, alkyle en C₁-C₆-(S=O)-, alkyle en C₁-C₆-(SO₂)-, alkyle en C₁-C₆-O-(C=O)-, formyle, alkyle en C₁-C₆-(C=O)- et cycloalkyle en C₃-C₆ ;
R³ est hétéroaryle en C₁-C₁₀ ou un hétérocyclyle en C₁-C₁₀ lié au carbone éventuellement substitué ; lesdits substituants éventuels pouvant être sur n'importe quel atome de carbone dudit hétéroaryle en C₁-C₁₀ ou hétérocyclyle en C₁-C₁₀ capable d'être substitué par un à trois R⁴ par cycle ; lesdits substituants éventuels pouvant être sur n'importe quel atome d'azote dudit hétéroaryle en C₁-C₁₀ ou hétérocyclyle en C₁-C₁₀ capable d'être substitué avec un à deux R⁵ par cycle ;
où chaque R⁴ est choisi indépendamment dans le groupe constitué par : halo, -CN, NH₂, hydroxy, H₂N (C=O) -, H₂N-SO₂- et les substituants R⁶ éventuellement substitués ;
où lesdits substituants R⁶ éventuellement substitués sont choisis dans le groupe constitué par : alkyle en C₁-C₆, cycloalkyle en C₃-C₁₀, hétérocyclyle en C₁-C₁₀, aryle en C₆-C₁₀, hétéroaryle en C₁-C₁₀, alcoxy en C₁-C₆, alkyle en C₁-C₆-(C=O)O-, alkyle en C₁-C₆-NH-, (alkyle en C₁-C₆)₂-N-, aryle en C₆-C₁₀-NH-, alkyle en C₁-C₆-(C=O)NH-, alkyle en C₁-C₆-SO₂-NH-, alkyle en C₁-C₆-(C=O)-, alkyle en C₁-C₆-NH-(C=O)-, (alkyle en C₁-C₆)₂-N-(C=O)-, alkyle en C₁-C₆-O(C=O)-, cycloalkyle en C₃-C₁₀-(C=O)-, hétérocyclyle en C₁-C₁₀-(C=O)-, aryle en C₆-C₁₀-(C=O)-, hétéroaryle en C₁-C₁₀-(C=O)-, alkyle en C₁-C₆-SO₂-, aryle en C₆-C₁₀-SO₂-, hétéroaryle en C₁-C₁₀-SO₂-, alkyle en C₁-C₆-NH-SO₂- et (alkyle en C₁-C₆) ₂N-SO₂- ;
où deux quelconques radicaux R⁴ sur un atome de carbone dudit hétérocyclyle en C₁-C₁₀ peuvent être pris conjointement pour former un groupe oxo ou un groupe spiro en C₃-C₆ carbocyclique ou en C₁-C₆ hétérocyclique ;
où chaque R⁵ est indépendamment choisi dans le groupe constitué par : H₂N(C=O)-, et les groupes R⁸ éventuellement substitués suivants : alkyle en C₁-C₆, hétérocyclyle en C₁-C₁₀, hétéroaryle en C₁-C₁₀, alkyle en C₁-C₆-(C=O)-, alkyle en C₁-C₆-NH-(C=O)-, (alkyle en C₁-C₆)₂-N-(C=O)-, alkyle en C₁-C₆-O(C=O)-, cycloalkyle en C₃-C₁₀-(C=O)-, hétérocyclyle en C₁-C₁₀-(C=O)-, aryle en C₆-C₁₀-(C=O)-, hétéroaryle en C₁-C₁₀-(C=O)-, et alkyle en C₁-C₆-SO₂- ;
où chacun desdits R⁶ éventuellement substitués peut être substitué par un à trois groupes indépendamment choisis dans le groupe constitué par :halo, NH₂, -CN, hydroxy, alkyle en C₁-C₆-SO₂-, H₂N-SO₂-, alkyle en C₁-C₆-NH-SO₂- et (alkyle en C₁-C₆)₂N-SO₂-, alcoxy en C₁-C₆, alkyle en C₁-C₆-SO₂-NH-, alkyle en C₁-C₆-(C=O)-, alkyle en C₁-C₆-(C=O)O-, H₂N (C=O) -, alkyle en C₁-C₆-NH-(C=O)-, (alkyle en C₁-C₆)₂-N-(C=O)-, alkyle en C₁-C₆-O(C=O)-, et les groupes R⁷ éventuellement substitués ;
où chacun desdits groupes R⁷ éventuellement substitués est indépendamment choisi dans le groupe constitué par : cycloalkyle en C₃-C₁₀, hétérocyclyle en C₁-C₁₀, aryle en C₆-C₁₀, hétéroaryle en C₁-C₁₀, phénoxy, alkyle en C₁-C₆-NH-, (alkyle en C₁-C₆)₂-N-, aryle en C₆-C₁₀-NH-, cycloalkyle en C₃-C₁₀-(C=O)-, hétérocyclyle en C₁-C₁₀-(C=O)-, aryle en C₆-C₁₀-(C=O)-, hétéroaryle en C₁-C₁₀-(C=O)-, aryle en C₆-C₁₀-SO₂- et hétéroaryle en C₁-C₁₀-SO₂- ;
où chacun desdits groupes R⁷ peut être éventuellement substitué par un à trois substituants indépendamment choisis dans le groupe constitué par : halo, CF₃, -CN, alkyle en C₁-C₆, hétérocyclyle en C₁-C₁₀, hétéroaryle en C₁-C₁₀, hydroxy, alcoxy en C₁-C₆, alkyle en C₁-C₆-O(C=O)-, alkyle en C₁-C₆-SO₂-NH-, alkyle en C₁-C₆-SO₂-, aryle en C₆-C₁₀-SO₂-, hétéroaryle en C₁-C₁₀-SO₂-, H₂N-SO₂-, alkyle en C₁-C₆-NH-SO₂- et (alkyle en C₁-C₆)₂N-SO₂- ;
où chacun desdits groupes R⁸ éventuellement substitués peut être substitué par un à trois substituants choisis indépendamment dans le groupe constitué par : halo, -CN, hydroxy, alcoxy en C₁-C₆, alkyle en C₁-C₆-(C=O)O-, H₂N-(C=O)O-, alkyle en C₁-C₆-NH-(C=O)O-, (alkyle en C₁-C₆)₂N-(C=O)O-, NH₂, alkyle en C₁-C₆-NH-, (alkyle en C₁-C₆)₂-N-, alkyle en C₁-C₆)-(C=O)NH-, alkyle en C₁-C₆)-NH-(C=O)NH-, alkyle en C₁-C₆-SO₂-NH- , alkyle en C₁-C₆-(C=O)NH-, alkyle en C₁-C₆-(C=O)-, H₂N (C=O) -, alkyle en C₁-C₆-NH-(C=O)-, (alkyle en C₁-C₆)₂-N-(C=O)-, alkyle en C₁-C₆-O(C=O)-, alkyle en C₁-C₆-SO₂-, H₂N-SO₂-, alkyle en C₁-C₆-NH-SO₂- et (alkyle en C₁-C₆)₂N-SO₂-, et les groupes R⁹ éventuellement substitués ;
où chacun desdits groupes R⁹ éventuellement substitués est indépendamment choisi dans le groupe constitué par : aryle en C₆-C₁₀, cycloalkyle en C₃-C₁₀-NH(C=O)-, hétérocyclyle en C₁-C₁₀-NH-(C=O)-, aryle en C₆-C₁₀-NH-(C=O)-, hétéroaryle en C₁-C₁₀-NH-(C=O)-, cycloalkyle en C₃-C₁₀-(C=O)-, hétérocyclyle en C₁-C₁₀-(C=O)-, aryle en C₆-C₁₀-(C=O)-, hétéroaryle en C₁-C₁₀-(C=O)-, aryle en C₆-C₁₀-SO₂-, hétéroaryle en C₁-C₁₀-SO₂- ;
où chacun desdits groupes R⁹ éventuellement substitués peut être éventuellement substitué par un à trois substituants choisis indépendamment dans le groupe constitué par : halo, alkyle en C₁-C₆, -CN, hydroxy, alcoxy en C₁-C₆, alkyle en C₁-C₆-(C=O)O-, NH₂, alkyle en C₁-C₆-NH-, (alkyle en C₁-C₆)₂-N-, (alkyle en C₁-C₆)-(C=O)NH-, alkyle en C₁-C₆-(C=O)-, H₂N(C=O)-, alkyle en C₁-C₆-NH-(C=O)- et (alkyle en C₁-C₆)₂-N-(C=O)-;
où la masse moléculaire dudit composé de formule I est inférieure à 700 AMU ;
choisi dans le groupe constitué par :
ester méthylique d'acide (3-{4-chloro-3-[(1-hydroxy-cycloheptylméthyl)-carbamoyl]-phényl}-5-méthylpyrazol-1-yl)-acétique ;
2-chloro-5-(1-cyanométhyl-1H-pyrazol-3-yl)-N-(1-hydroxy-cycloheptylméthyl)-benzamide ;
2-chloro-5-(1-cyanométhyl-5-méthyl-1H-pyrazol-3-yl)-N-(1-hydroxy-cycloheptylméthyl)-benzamide ;
2-chloro-5-(5-cyclopropyl-1H-pyrazol-3-yl)-N-(1-hydroxy-cycloheptylméthyl)-benzamide ;
2-chloro-5-(6-méthyl-pyridin-3-yl)-N-(1-p-tolylcyclohexylméthyl)-benzamide ;
2-chloro-5-[1-(2,3-dihydroxy-2-méthylpropyl)-5-méthyl-1H-pyrazol-3-yl]-N-(1-hydroxycycloheptylméthyl)-benzamide ;
2-chloro-5-[1-(3-fluoro-2-hydroxy-propyl)-1H-imidazol-4-yl]-N-(1-hydroxy-cycloheptylméthyl)-benzamide ;
2-chloro-5-[2-(2,3-dihydroxy-propyl)-5-méthyl-2H-pyrazol-3-yl]-N-(1-hydroxy-cycloheptylméthyl)-benzamide ;
2-chloro-N-(1-cyano-cycloheptylméthyl)-5-(5-méthyl-2H-pyrazol-3-yl)-benzamide ;
2-chloro-N-(1-hydroxy-3,3-diméthylcyclohexylméthyl)-5-(3-méthyl-isoxazol-5-yl)-benzamide ;
2-chloro-N-(1-hydroxy-3,3-diméthylcyclohexylméthyl)-5-(5-méthyl-2H-pyrazol-3-yl)-benzamide ;
2-chloro-N-(1-hydroxy-3,3-diméthylcyclohexylméthyl)-5-(5-méthyl-pyridin-2-yl)-benzamide ;
2-chloro-N-(1-hydroxy-3,3-diméthylcyclohexylméthyl)-5-(5-trifluorométhyl-1H-pyrazol-3-yl)-benzamide ;
2-chloro-N-(1-hydroxy-3,3-diméthylcycloheptylméthyl)-5-(5-trifluorométhyl-1H-pyrazol-3-yl)-benzamide ;
2-chloro-N-(1-hydroxy-cycloheptylméthyl)-5-(1H-imidazol-4-yl)-benzamide ;
2-chloro-N-(1-hydroxy-cycloheptylméthyl)-5-(1H-pyrazol-3-yl)-benzamide ;
2-chloro-N-(1-hydroxy-cycloheptylméthyl)-5-(1-méthyl-1H-pyrazol-3-yl)-benzamide ;
2-chloro-N-(1-hydroxy-cycloheptylméthyl)-5-(2H-[1, 2,3]triazol-4-yl)-benzamide ;
2-chloro-N-(1-hydroxy-cycloheptylméthyl)-5-(2-méthyl-2H-pyrazol-3-yl)-benzamide ;
2-chloro-N-(1-hydroxy-cycloheptylméthyl)-5-(2-méthyl-thiazol-4-yl)-benzamide ;
2-chloro-N-(1-hydroxy-cycloheptylméthyl)-5-(3-méthyl-isoxazol-5-yl)-benzamide ;
2-chloro-N-(1-hydroxy-cycloheptylméthyl)-5-(5-méthyl-1-oxiranylméthyl-1H-pyrazol-3-yl)-benzamide ;
2-chloro-N-(1-hydroxy-cycloheptylméthyl)-5-(5-méthyl-pyridin-2-yl)-benzamide ;
2-chloro-N-(1-hydroxy-cycloheptylméthyl)-5-(5-trifluorométhyl-1H-pyrazol-3-yl)-benzamide ;
2-chloro-N-(1-hydroxy-cycloheptylméthyl)-5-(6-méthoxy-pyridazin-3-yl)-benzamide ;
2-chloro-N-(1-hydroxy-cycloheptylméthyl)-5-(hydroxyimino-méthyl)-benzamide ;
2-chloro-N-(1-hydroxy-cycloheptylméthyl)-5[1-(2-hydroxy-2- méthylpropyl)-1H-pyrazol-3-yl]-benzamide ;
2-chloro-N-(1-hydroxy-cycloheptylméthyl)-5-[1-(2-hydroxy-2-méthylpropyl)-5-méthyl-1H-pyrazol-3-yl]-benzamide ;
2-chloro-N-(1-hydroxy-cycloheptylméthyl)-5-[1-(2-hydroxy-éthyl)-1H-pyrazol-3-yl]-benzamide ;
2-chloro-N-(1-hydroxy-cycloheptylméthyl)-5-[1-(2-hydroxy-éthyl)-6-oxo-1,6-dihydro-pyridazin-3-yl]-benzamide ;
2-chloro-N-(1-hydroxy-cycloheptylméthyl)-5-[2-(2-hydroxy-éthyl)-2H-pyrazol-3-yl]-benzamide ;
2-chloro-N-(1-hydroxy-cycloheptylméthyl)-5-[2-(2-hydroxy-éthyl)-5-méthyl-2H-pyrazol-3-yl]-benzamide ;
2-chloro-N-(1-hydroxy-cycloheptylméthyl)-5-[5-(2-hydroxy-éthyl)-isoxazol-3-yl]-benzamide ;
2-chloro-N-(1-hydroxy-cycloheptylméthyl)-5-[5-méthyl-1-(2-oxo-2-pyrrolidin-1-yl-éthyl)-1H-pyrazol-3-yl]-benzamide ;
2-chloro-N-(1-hydroxy-cycloheptylméthyl)-5-[5-méthyl-1-(pyrimidin-2-ylcarbamoylméthyl)-1H-pyrazol-3-yl]-benzamide ;
2-chloro-N-(1-hydroxy-cycloheptylméthyl)-5-{1-[(1-hydroxyméthyl-propylcarbamoyl)-méthyl]-5-méthyl-1H-pyrazol-3-yl}-benzamide ;
2-chloro-N-(1-hydroxy-CyCloheptylméthyl)-5-{1-[(2-hydroxy-propylcarbamoyl)-méthyl]-5-méthyl-1H-pyrazol-3-yl}-benzamide ;
2-chloro-N-[2-(2-chloro-phényl)-éthyl]-5-(1H-pyrazol-3-yl)-benzamide ;
2-chloro-N-[2-(2-chloro-phényl)-éthyl]-5-(1H-pyrazol-4-yl)-benzamide ;
2-chloro-N-[2-(2-chloro-phényl)-éthyl]-5-(2-méthyl-pyrimidin-4-yl)-benzamide ;
2-chloro-N-[2-(2-chloro-phényl)-éthyl]-5-(5-éthyl-2H-pyrazol-3-yl)-benzamide ;
2-chloro-N-[2-(2-chloro-phényl)-éthyl]-5-(6-méthoxy-pyridazin-3-yl)-benzamide ;
2-chloro-N-[2-(2-chloro-phényl)-éthyl]-5-[1-(4-méthoxy-benzyl)-1H-pyrazol-4-yl]-benzamide ;
5-(1-Carbamoylméthyl-1H-pyrazol-3-yl)-2-chloro-N-(1-hydroxy-cycloheptylméthyl)-benzamide ;
5-(1-carbamoylméthyl-5-méthyl-1H-pyrazol-3-yl)-2-chloro-N-(1-hydroxy-cycloheptylméthyl)-benzamide ;
hydrochlorure de 5-[1-(3-amino-2-hydroxy-propyl)-5-méthyl-1H-pyrazol-3-yl]-2-chloro-N-(1-hydroxycycloheptylméthyl)-benzamide ; et
5-[2-(3-amino-2-hydroxy-propyl)-5-méthyl-2H-pyrazol-3-yl]-2-chloro-N-(1-hydroxy-cycloheptylméthyl)-benzamide ;
ou un sel pharmaceutiquement acceptable de ceux-ci.

2. Composition pharmaceutique comprenant une quantité thérapeutiquement efficace d'un composé selon la revendication 1, ou d'un sel pharmaceutiquement acceptable de celui-ci, et un support ou diluant pharmaceutiquement acceptable.

3. Composé selon la revendication 1 ou sel pharmaceutiquement acceptable de celui-ci, destiné à être utilisé dans le traitement de l'arthrite rhumatoïde.

4. Composé ou sel pharmaceutiquement acceptable de celui-ci selon la revendication 1, destiné à être utilisé en combinaison avec un autre agent pharmaceutiquement actif.
